# EUROPEAN PATENT SPECIFICATION

(11) **EP 4 347 887 B1**
(45) Date of publication and mention of the grant of the patent: **24.12.2025**
(21) Application number: 22729761.1
(22) Date of filing: 30.05.2022
(51) Int. Cl.: C12Q 1/6883

(54) **BIOMARKER PANEL FOR INCREASED LIKELIHOOD OF BLASTOCYST IMPLANTATION SUCCESS**
BIOMARKERTAFEL ZUR ERHÖHTEN WAHRSCHEINLICHKEIT DES IMPLANTATIONSERFOLGS VON BLASTOZYSTEN
PANEL DE BIOMARQUEURS POUR ACCROITRE LA PROBABILITÉ DE SUCCÈS DE L'IMPLANTATION DE BLASTOCYSTES

(30) Priority: 01.06.2021 GB 202107813
(43) Date of publication of application: 10.04.2024
(73) Proprietor: Ntostis, Panagiotis, Leeds LS2 7JQ (GB)
(72) Inventor: Ntostis, Panagiotis, Leeds LS2 7JQ (GB)
(74) Representative: Handley, Matthew Edward
(86) International application number: PCT/GB2022/051380
(87) International publication number: WO 2022/254198

(56) References cited:
- US-A1- 2011 124 511
- US-A1- 2015 119 282
- US-A1- 2016 017 422
- NTOSTIS PANAGIOTIS ET AL: "Can trophectoderm RNA analysis predict human blastocyst competency?", SYSTEMS BIOLOGY IN REPRODUCTIVE MEDICINE, vol. 65, no. 4, 27 June 2019 (2019-06-27), US, pages 312 - 325, XP055951777, ISSN: 1939-6368, Retrieved from the Internet <URL:https://tandfonline.com/doi/pdf/10.1080/19396368.2019.1625085> DOI: 10.1080/19396368.2019.1625085
- JASON C PARKS ET AL: "Blastocyst gene expression correlates with implantation potential", FERTILITY AND STERILITY, ELSEVIER, AMSTERDAM, NL, vol. 95, no. 4, 5 August 2010 (2010-08-05), pages 1367 - 1372, XP028169824, ISSN: 0015-0282, [retrieved on 20100813], DOI: 10.1016/J.FERTNSTERT.2010.08.009
- HAMATANI TOSHIO ET AL: "Global gene expression profiling of preimplantation embryos", HUMAN CELL, WILEY-BLACKWELL PUBLISHING LTD, UK; JP, vol. 19, no. 3, 1 August 2006 (2006-08-01), pages 98 - 117, XP035306834, ISSN: 0914-7470, [retrieved on 20061219], DOI: 10.1111/J.1749-0774.2006.00018.X
- KIRKEGAARD KIRSTINE ET AL: "Distinct differences in global gene expression profiles in non-implanted blastocysts and blastocysts resulting in live birth", GENE, ELSEVIER AMSTERDAM, NL, vol. 571, no. 2, 25 June 2015 (2015-06-25), pages 212 - 220, XP029264197, ISSN: 0378-1119, DOI: 10.1016/J.GENE.2015.06.057
- AFFYMETRIX: "GeneChip Human Genome U133 Plus 2.0 Array", 20030101, 1 January 2003 (2003-01-01), pages 1 - 2, XP007921349

## Description

The present invention relates to biomarkers, and to the field of blastocyst uterine transfer, and in particular, to biomarkers for the identification of blastocysts having a high (or conversely, low) likelihood of implantation success and/or developmental competency upon uterine transfer. The invention also extends to methods and kits for testing and identifying such blastocysts.

In recent years, maternal age has been rising world-wide, having an influence in reproductive success rates (Schmidt et al., 2012), and is one of the strongest predictors for assisted reproductive technology (ART) success (Nelson and Lawlor, 2011). Generally, women less than 35 years old report higher pregnancy, delivery and implantation rates (Medicine, 2006, Hull et al., 1996). In women over 35 years, euploid embryo transfer corresponds to decreased live birth rates (Scott Jr et al., 2012), while pregnancy/implantation rates are reduced in women over 40 years old (Harton et al., 2013). Uterine transfer of euploid blastocysts yields implantation rates approximately between 50% and 80%, with idiopathic (unexplained) infertility accounting for 14-26% of implantation failure cases (Saravelos and Li, 2012). NTOSTIS PANAGIOTIS ET AL. (2019-06-27) identifies a total of 47 transcripts that were found to be significantly differentially expressed between the trophectoderm cells from successfully implanted (competent) versus unsuccessful (incompetent) blastocysts.

There is, therefore, a need for a method of identifying blastocysts for embryo transfer, which have a high or increased (or low or decreased) likelihood of implantation success.

The inventors examined the effect of maternal age on trophectoderm transcriptome dynamics and identified patterns of gene expression that are characteristic of blastocysts having a young reproductive chronological (YMA) and/or biological maternal age (rba-YMA) versus an advanced reproductive chronological (AMA) and/or biological maternal age (rba-AMA). Blastocysts having a young reproductive chronological (YMA) and/or biological maternal age (rba-YMA) were found to have an increased likelihood of implantation success versus those having an advanced reproductive chronological (AMA) and/or biological maternal age (rba-AMA). The inventors have subsequently identified a set of molecular biomarkers that can be used to identify blastocysts having a young reproductive chronological and/or biological maternal age (YMA/rba-YMA), and that are predictive of implantation success (Tables A-E). Hierarchical clustering analysis on the differentially expressed trophectoderm genes was performed as a function of chronological age across the YMA and AMA datasets, guiding the reproductive biological age (rba-YMA and rba-AMA) classification.

Throughout this disclosure, the terms "young chronological age", "young chronological maternal age", "young reproductive chronological maternal age" and "YMA" are used interchangeably. Likewise, the terms "advanced chronological age", "advanced chronological maternal age", "advanced reproductive chronological maternal age" and "AMA" are also used interchangeably. Similarly, the terms "young biological age", "young biological maternal age", "young reproductive biological maternal age" and "rba-YMA" are used interchangeably. Likewise, the terms "advanced biological age", "advanced biological maternal age", "advanced reproductive biological maternal age" and "rba-AMA" are also used interchangeably.

Table A and Table C list the genes that have been found to have a significantly increased level of expression in trophoblasts identified as having a young chronological (YMA) and/or young biological reproductive maternal age (rba-YMA) relative to the level of expression in trophoblasts identified as having an advanced chronological (AMA) and/or advanced biological reproductive maternal age (rba-AMA). The genes listed in Table A and Table C, therefore, may be used as biomarkers of blastocysts having an increased likelihood of implantation success.

The inventors have also identified a set of molecular biomarkers that can be used to identify blastocysts having an advanced reproductive chronological and/or biological maternal age, and that are predictive of reduced likelihood of implantation success.

Table B and Table D list the genes that have been found to have a significantly increased level of expression in trophoblasts identified as having an advanced chronological (AMA) and/or advanced biological reproductive maternal age (rba-AMA) relative to the level of expression in trophoblasts identified as having a young chronological (YMA) and/or young biological reproductive maternal age (rba-YMA). The genes listed in Table B and Table D, therefore, may be used as biomarkers of blastocysts having a reduced likelihood of implantation success.

Therefore, all these biomarkers can be used in the context of predicting successful embryo implantation and/or developmental competency, considering that their significantly higher (Table A and Table C) or lower (Table B and Table D) gene expression levels, could be used as tools to identify the blastocysts with greater potential to implant into the uterus.

The biomarkers in Tables A-D are extremely specific and sensitive to detection.

Thus, according to a first aspect of the invention, there is provided the use of at least one gene or gene transcript or fragment thereof selected from those listed in Table A, Table B, Table C, and/or Table D, or an expression product or fragment thereof, as a biomarker for blastocyst implantation success.

In some embodiments, there is provided the use of at least two genes and/or gene transcripts selected from any of those listed in Table A, Table B, Table C, and/or Table D, or expression products or fragments thereof, as biomarkers for blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A, Table B, Table C, and/or Table D, or expression products or fragments thereof, as biomarkers, or in a biomarker panel, for blastocyst implantation success.

Throughout this disclosure, references to the use of at least one gene or gene transcript selected from one or more of the enclosed Tables preferably includes the selection of at least one of the genes in the Tables that have been found to be preferred for use in the disclosed invention, and these genes are highlighted in the enclosed Tables using an asterisk (*).

Thus, in some embodiments, there is provided the use of at least one gene or gene transcript or fragment thereof selected from those listed in Table A*, Table B*, Table C*, and/or Table D*, or an expression product or fragment thereof, as a biomarker for blastocyst implantation success. For example, in some embodiments, there is provided the use of at least 2, 3, 4, 5, 6, 7, 8, 9, 10, 12, 15, 18, 20, 25, 30, 35, 40, 45, or 50 genes and/or gene transcripts selected from any of those listed in Table A*, Table B*, Table C*, and/or Table D*, or expression products or fragments thereof, as biomarkers, or in a biomarker panel, for blastocyst implantation success.

In some embodiments, there is provided the use of at least 2, 3, 4, 5, 6, 7, 8, 9, 10, 12, 15, 18, 20, 25, 30, 35, 40, 45, or 50 genes and/or gene transcripts selected from any of those listed in Table A, Table B, Table C, and/or Table D, or expression products or fragments thereof, as biomarkers, or in a biomarker panel, for blastocyst implantation success, wherein at least one of the genes and/or gene transcripts or fragment thereof, such as at least 2, 3, 4, 5, 6, 7, 8, 9, 10, 12, 15, 18, 20, 25, 30, 35, 40, 45, or 50 genes and/or gene transcripts or fragments thereof is selected from Table A*, Table B*, Table C*, and/or Table D*.

More generally, there is provided the use of at least 2, 3, 4, 5, 6, 7, 8, 9, 10, 12, 15, 18, 20, 25, 30, 35, 40, 45, or 50 genes and/or gene transcripts from those listed in one or more selected Table, such as Table A, Table B, Table C, and/or Table D, or expression products or fragments thereof, as biomarkers, or in a biomarker panel, for blastocyst implantation success, wherein at least one of the genes and/or gene transcripts or fragment thereof, such as at least 2, 3, 4, 5, 6, 7, 8, 9, 10, 12, 15, 18, 20, 25, 30, 35, 40, 45, or 50 genes and/or gene transcripts or fragments thereof is selected from the corresponding asterisked Table or Tables, such as Table A*, Table B*, Table C*, and/or Table D*.

Throughout this disclosure, the terms "gene transcript" and "transcript" are used interchangeably. Unless otherwise stated, references to a plurality of "gene transcripts" selected from one or more of the enclosed Tables refers specifically to the selection of a plurality of transcripts each derived from a different gene, and does not encompass the selection of a plurality of transcripts derived from the same gene.

Also, throughout this disclosure, reference to "fragment thereof" refers to a fragment of a gene, a fragment of a gene transcript, or a fragment of an expression product. Preferably, the fragment thereof is a fragment that may be used to uniquely identify the gene, gene transcript, or expression product. Thus, in some embodiments, a fragment of a gene may be used as a biomarker. In some embodiments, a fragment of a gene transcript may be used as a biomarker. In some embodiments, or a fragment of an expression product may be used as a biomarker.

Advantageously, all of the biomarkers used in accordance with the first aspect of the invention are expressed in samples from blastocysts for potential embryo transfer.

Detection for the presence of the 1 or more genes, gene transcripts, expression products, or fragments thereof, in the sample, is preferably carried out in vitro or ex vivo.

Throughout this disclosure, "blastocyst implantation success" may refer to an increased likelihood of blastocyst implantation success or a reduced likelihood of blastocyst implantation success.

Samples used in this disclosure may consist of, or comprise material, such as cells, tissues, extracellular vesicles, or fluid that is, or has previously been, collected from a blastocyst for potential embryo transfer. Suitable sample material may consist of or comprise, for example, material from the blastocyst trophectoderm, such as one or more trophoblast cells, material from the blastocoele, such as from a blastocentesis sample, material from media in which the blastocyst has been cultured, material consisting of, or comprising extracellular exosomes produced by the blastocyst and/or combination of the above.

Suitable blastocysts may be obtained from mothers having any reproductive age. In particular, blastocysts may be obtained from mothers having an age of greater than 30, greater than 35, or greater than 40 years old, such as, for example, having an age between 30 and 40 years old. In preferred embodiments, the mother's age may be 31, 32, 33, 34, 35, 36, 37, 38, or 39. Preferably the mother has an age between 31 and 39, 32 and 38, 33 and 37, or 34 and 36. The mother may have an age of greater than 35, 40, 45, or 50, for example.

The inventors have found that the biomarkers listed in Table A and Table C have an increased level of expression in samples identified as having a young chronological (YMA) and/or young biological reproductive maternal age (rba-YMA) relative to the level of expression in samples identified as having an advanced chronological (AMA) and/or advanced biological reproductive maternal age (rba-AMA).

Thus, in some embodiments, there is provided the use of at least one gene or gene transcript selected from those listed in Table A or Table C, or an expression product or fragment thereof, as a biomarker for increased likelihood of blastocyst implantation success. Preferably, the at least one gene or gene transcript is selected from those listed in Table A* or Table C*, or an expression product or fragment thereof. More preferably, there is provided the use of at least 2, 3, 4, 5, 6, 7, 8, 9, 10, 12, 15, 18, 20, 25, 30, 35, 40, 45, or 50 genes and/or gene transcripts selected from any of those listed in Table A or Table C, or expression products or fragments thereof, as biomarkers, or in a biomarker panel, for blastocyst implantation success, wherein at least one of the genes and/or gene transcripts or fragment thereof, such as at least 2, 3, 4, 5, 6, 7, 8, 9, 10, 12, 15, 18, 20, 25, 30, 35, 40, 45, or 50 genes and/or gene transcripts or fragments thereof is selected from Table A* and/or Table C*.

In some embodiments, there is provided the use of at least 35 genes and/or gene transcripts selected from any of those listed in Table A and/or Table C, or expression products or fragments thereof, in or as a biomarker panel for increased likelihood of blastocyst implantation success. Preferably, there is provided the use of at least 40, 45, 50, or all, genes and/or gene transcripts selected from any of those listed in Table A and/or Table C, or expression products or fragments thereof, in or as a biomarker panel for increased likelihood of blastocyst implantation success.

The inventors have found that the biomarkers in Table A have a particularly statistically significantly increased level of expression in samples that have a young chronological (YMA) and/or young biological reproductive maternal age (rba-YMA) relative to the level of expression in samples identified as having an advanced chronological (AMA) and/or advanced biological reproductive maternal age (rba-AMA) (false discovery rate - FDR of <0.01).

Thus, in preferred embodiments, there is provided the use of at least one gene or gene transcript selected from those listed in Table A, or an expression product or fragment thereof, as a biomarker for increased likelihood of blastocyst implantation success. In some embodiments, there is provided the use of at least 35 genes and/or gene transcripts selected from any of those listed in Table A, or expression products or fragments thereof, in or as a biomarker panel for increased likelihood of blastocyst implantation success. Preferably, there is provided the use of at least 40, 45, 50, or all, genes and/or gene transcripts selected from any of those listed in Table A, or expression products or fragments thereof, in or as a biomarker panel for increased likelihood of blastocyst implantation success.

The inventors have found that the biomarkers listed in Table B and Table D have an increased level of expression in samples identified as having an advanced chronological (AMA) and/or advanced biological reproductive maternal age (rba-AMA) relative to the level of expression in samples identified as having a young chronological (YMA) and/or young biological reproductive maternal age (rba-YMA).

Thus, in some embodiments, there is provided the use of at least one gene or gene transcript selected from those listed in Table B or Table D, or an expression product or fragment thereof, as a biomarker for reduced likelihood of blastocyst implantation success.

The inventors have found that the biomarkers in Table B have a particularly statistically significantly increased level of expression in samples that have an advanced chronological (AMA) and/or advanced biological reproductive maternal age (rba-AMA) relative to the level of expression in samples identified as having a young chronological (YMA) and/or young biological reproductive maternal age (rba-YMA) (FDR of <0.01).

Thus, in preferred embodiments, there is provided the use of at least one gene or gene transcript selected from those listed in Table B, or an expression product or fragment thereof, as a biomarker for reduced likelihood of blastocyst implantation success.

References to the level of expression of the biomarkers may refer to the level of gene or gene transcript expression, such as, for example, measured in terms of the level of nucleic acid, such as the amount of mRNA or cDNA in the sample, or may refer to the expression product, such as the level of polypeptide or protein in the sample. Suitable methods for determining these different measures of the level of expression are described below and will be known to the skilled person. Any suitable method known in the art may be used.

The inventors have found that the biomarkers having a statistically significantly increased level of expression in samples that have a young chronological (YMA) and/or young biological reproductive maternal age (rba-YMA) relative to the level of expression in samples identified as having an advanced chronological (AMA) and/or advanced biological reproductive maternal age (rba-AMA) may be either protein coding transcripts or non-coding transcripts. On this basis, Table A is made up of Table A1, referred to as "Table A protein coding transcripts", and Table A2, referred to as "Table A non-coding transcripts".

Protein coding transcripts that have been found to have a statistically significantly increased level of expression in samples that have a young chronological (YMA) and/or young biological reproductive maternal age (rba-YMA) relative to the level of expression in samples identified as having an advanced chronological (AMA) and/or advanced biological reproductive maternal age (rba-AMA) are listed in Table A1.

Thus, in some embodiments, there is provided the use of at least one gene or gene transcript selected from those listed in Table A1, or an expression product or fragment thereof, as a biomarker for increased likelihood of blastocyst implantation success.

In some embodiments, there is provided the use of at least one gene expression product or fragment thereof selected from those listed in Table A1, as a biomarker for increased likelihood of blastocyst implantation success.

In some embodiments, there is provided the use of at least one gene transcript or identifying fragment thereof selected from those listed in Table A1, as a biomarker for increased likelihood of blastocyst implantation success.

Non-coding transcripts that have been found to have a statistically significantly increased level of expression in samples that have a young chronological (YMA) and/or young biological reproductive maternal age (rba-YMA) relative to the level of expression in samples identified as having an advanced chronological (AMA) and/or advanced biological reproductive maternal age (rba-AMA) are listed in Table A2.

Thus, in some embodiments, there is provided the use of at least one gene transcript or identifying fragment thereof selected from those listed in Table A2, as a biomarker for increased likelihood of blastocyst implantation success.

The inventors have also found that the biomarkers having a statistically significantly increased level of expression in samples that have an advanced chronological (AMA) and/or advanced biological reproductive maternal age (rba-AMA) relative to the level of expression in samples identified as having a young chronological (YMA) and/or young biological reproductive maternal age (rba-YMA) may be either protein coding transcripts or non-coding transcripts. On this basis, Table B is made up of Table B1, referred to as "Table B protein coding transcripts", and Table B2, referred to as "Table B non-coding transcripts".

Protein coding transcripts that have been found to have a statistically significantly increased level of expression in samples having an advanced chronological (AMA) and/or advanced biological reproductive maternal age (rba-AMA) relative to the level of expression in samples identified as having a young chronological (YMA) and/or young biological reproductive maternal age (rba-YMA) are listed in Table B1.

Thus, in some embodiments, there is provided the use of at least one gene or gene transcript selected from those listed in Table B1, or an expression product or fragment thereof, as a biomarker for reduced likelihood of blastocyst implantation success.

In some embodiments, there is provided the use of at least one gene expression product or fragment thereof selected from those listed in Table B1, as a biomarker for reduced likelihood of blastocyst implantation success.

In some embodiments, there is provided the use of at least one gene transcript or identifying fragment thereof selected from those listed in Table B1, as a biomarker for reduced likelihood of blastocyst implantation success.

Non-coding transcripts that have been found to have a statistically significantly increased level of expression in samples having an advanced chronological (AMA) and/or advanced biological reproductive maternal age (rba-AMA) relative to the level of expression in samples identified as having a young chronological (YMA) and/or young biological reproductive maternal age (rba-YMA) are listed in Table B2.

In some embodiments, there is provided the use of at least one gene transcript or identifying fragment thereof selected from those listed in Table B2, as a biomarker for reduced likelihood of blastocyst implantation success.

The use of a plurality of transcripts selected from those listed in Tables A-D as biomarkers as described may provide improved statistical power for the prediction of blastocyst implantation success.

Thus, in some embodiments, there is provided the use of at least two genes and/or gene transcripts selected from those listed in Table A, Table B, Table C, and/or Table D, or expression products or fragments thereof, in or as a biomarker panel for blastocyst implantation success.

Throughout this disclosure, the use of a plurality of genes, gene transcripts, expression products, and/or fragments thereof as described herein in or as a biomarker panel for blastocyst implantation success encompasses the use of the plurality of genes, gene transcripts, expression products, and/or fragments thereof in a biomarker panel consisting of the plurality of genes, gene transcripts, expression products, and/or fragments thereof, and the use of the plurality of genes, gene transcripts, expression products, and/or fragments thereof in a biomarker panel comprising the plurality of genes, gene transcripts, expression products, and/or fragments thereof.

In some embodiments, there is provided the use of at least two genes and/or gene transcripts selected from any of those listed in Table A and/or Table C, or expression products or fragments thereof, in or as a biomarker panel for increased likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A and/or Table C, or expression products or fragments thereof, in or as a biomarker panel for increased likelihood of blastocyst implantation success.

In some embodiments, there is provided the use of at least two genes and/or gene transcripts selected from any of those listed in Table A, or expression products or fragments thereof, in or as a biomarker panel for increased likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A, or expression products or fragments thereof, in or as a biomarker panel for increased likelihood of blastocyst implantation success.

In some embodiments, there is provided the use of at least two genes and/or gene transcripts selected from any of those listed in Table A1, or expression products or fragments thereof, in or as a biomarker panel for increased likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A1, or expression products or fragments thereof, in or as a biomarker panel for increased likelihood of blastocyst implantation success.

In some embodiments, there is provided the use of at least two gene transcripts selected from any of those listed in Table A2, or fragments thereof, in or as a biomarker panel for increased likelihood of blastocyst implantation success. For example, in some embodiments, there is provided the use of at least 3, 4, 5, 6, 7, 8, 9, 10, 12, 15, 18, 20, or 21 gene transcripts selected from any of those listed in Table A2, or fragments thereof, in or as a biomarker panel for increased likelihood of blastocyst implantation success.

In some embodiments, there is provided the use of at least two genes and/or gene transcripts selected from any of those listed in Table B and/or Table D, or expression products or fragments thereof, in or as a biomarker panel for reduced likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table B and/or Table D, or expression products or fragments thereof, in or as a biomarker panel for reduced likelihood of blastocyst implantation success.

In some embodiments, there is provided the use of at least two genes and/or gene transcripts selected from any of those listed in Table B, or expression products or fragments thereof, in or as a biomarker panel for reduced likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table B, or expression products or fragments thereof, in or as a biomarker panel for reduced likelihood of blastocyst implantation success.

In some embodiments, there is provided the use of at least two genes and/or gene transcripts selected from any of those listed in Table B1, or expression products or fragments thereof, in or as a biomarker panel for reduced likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table B1, or expression products or fragments thereof, in or as a biomarker panel for reduced likelihood of blastocyst implantation success.

In some embodiments, there is provided the use of at least two gene transcripts selected from any of those listed in Table B2, or fragments thereof, in or as a biomarker panel for reduced likelihood of blastocyst implantation success. For example, in some embodiments, there is provided the use of at least 3, 4, 5, 6, 7, 8, 9, 10, 12, 15, 18, or 19 gene transcripts selected from any of those listed in Table B2, or fragments thereof, in or as a biomarker panel for reduced likelihood of blastocyst implantation success.

The YMA/rba-YMA coding genes/transcripts in Table A1 and the non-coding transcripts in Table A2 are listed in order of statistical significance, illustrating a greater confidence of their gene expression levels to be significantly higher compared with the level of expression of the same gene or gene transcript in AMA/rba-AMA samples. YMA/rba-YMA samples correspond to increased likelihood of implantation and/or embryonic developmental success, based on FDRs determined as described in the examples below. All of the genes listed in Table A1 and Table A2 have FDRs of less than 0.01. The most statistically significant gene (i.e. having the lowest FDR) is listed first in each table, with the subsequently listed genes having sequentially lower levels of statistical significance.

Thus, in preferred embodiments, there is provided the use of the most statistically significantly enriched transcript from Table A1 (which is transcript NM_001079862) or Table A2 (which is NR_144397), or an expression product or fragment of either transcript, as a biomarker for increased likelihood of blastocyst implantation success.

Throughout this disclosure, the term "enriched" may refer to an increased level of transcription, translation, or expression of a gene or gene transcript or an expression product or fragment thereof.

In some embodiments, there is provided the use of a plurality of the most statistically significantly enriched transcripts from Table A1, or expression products or fragments thereof, in or as a biomarker panel for increased likelihood of blastocyst implantation success. For example, in some embodiments, there is provided the use of at least the first two (i.e. most statistically significantly enriched) transcripts from Table A1, or expression products or fragments thereof, in or as a biomarker panel for increased likelihood of blastocyst implantation success. For example, in some embodiments, there is provided the use of at least the first 3, 4, 5, 6, 7, 8, 9, 10, 12, 15, 18, 20, 25, 30, 35, 40, 45, or 50 (i.e. most statistically significantly enriched) transcripts from Table A1, or expression products or fragments thereof, in or as a biomarker panel for increased likelihood of blastocyst implantation success. In some embodiments, there is provided the use of at least 2, 3, 4, 5, 6, 7, 8, 9, or 10 of the first 10 (i.e. most statistically significantly enriched) transcripts from Table A1, or expression products or fragments thereof, in or as a biomarker panel for increased likelihood of blastocyst implantation success. In some embodiments, there is provided the use of at least 2, 3, 4, 5, 6, 7, 8, 9, 10, 12, 15, 18, 20, 25, 30, 35, 40, 45, or 50 of the first 50 (i.e. most statistically significantly enriched transcripts) in Table A1, or expression products or fragments thereof, in or as a biomarker panel for increased likelihood of blastocyst implantation success. In some embodiments, there is provided the use of at least 35 genes and/or gene transcripts selected from any of those listed in Table A and/or Table C, or expression products or fragments thereof, in or as a biomarker panel for increased likelihood of blastocyst implantation success, wherein the selected genes and/or gene transcripts, expression products, or fragments thereof, comprise at least 2, such as at least 3, 4, 5, 6, 7, 8, 9, 10, 12, 15, 18, 20, 25, 30, or 35, of the first 50 genes or gene transcripts in Table A1, or expression products or fragments thereof. In some embodiments, there is provided the use of at least 35, such as at least 40, 45, 50, or all, genes and/or gene transcripts selected from any of those listed in Table A and/or Table C, or expression products or fragments thereof, in or as a biomarker panel for increased likelihood of blastocyst implantation success, wherein the selected genes and/or gene transcripts, expression products, or fragments thereof, comprise at least 2, of the first 50 genes or gene transcripts in Table A1, or expression products or fragments thereof.

In some embodiments, there is provided the use of at least the two most statistically significantly correlated transcripts from Table A2, or expression products or fragments thereof, in or as a biomarker panel for increased likelihood of blastocyst implantation success. For example, in some embodiments, there is provided the use of at least the first 3, 4, 5, 6, 7, 8, 9, 10, 12, 15, or 18, or 20 (i.e. most statistically significantly enriched) transcripts from Table A2, or expression products or fragments thereof, in or as a biomarker panel for increased likelihood of blastocyst implantation success. In some embodiments, there is provided the use of at least 2, 3, 4, 5, 6, 7, 8, 9, or 10 of the first 10 (i.e. most statistically significantly enriched) transcripts from Table A2, or expression products or fragments thereof, in or as a biomarker panel for increased likelihood of blastocyst implantation success. In some embodiments, there is provided the use of at least 2, 3, 4, 5, 6, 7, 8, 9, 10, 12, 15, 18, or 20 of the first 20 (i.e. most statistically significantly correlated) transcripts from Table A2, or expression products or fragments thereof, in or as a biomarker panel for increased likelihood of blastocyst implantation success.

In some embodiments, there is provided the use of all of the genes or gene transcripts listed in Table A and Table C in combination, or expression products or fragments thereof, in or as a biomarker panel for increased likelihood of blastocyst implantation success.

In some embodiments, there is provided the use of all of the genes or gene transcripts listed in Table A, or expression products or fragments thereof, in or as a biomarker panel for increased likelihood of blastocyst implantation success.

In some embodiments, there is provided the use of all of the genes or gene transcripts listed in Table C, or expression products or fragments thereof, in or as a biomarker panel for increased likelihood of blastocyst implantation success.

In some embodiments, there is provided the use of all of the genes or gene transcripts listed in Table A1, or expression products or fragments thereof, in or as a biomarker panel for increased likelihood of blastocyst implantation success.

In some embodiments, there is provided the use of all of the genes or gene transcripts listed in Table A2, or expression products or fragments thereof, in or as a biomarker panel for blastocyst implantation success, and specifically, as a biomarker for increased likelihood of blastocyst implantation success.

The AMA/rba-AMA coding genes/transcripts in Table B1 and the non-coding transcripts in Table B2 are also listed in order of the statistical significance, illustrating a greater confidence of their gene expression levels to be significantly higher compared with the expression of the same gene or gene transcript in the YMA/rba-YMA samples. AMA/rba-AMA samples correspond to a reduced likelihood of implantation and/or embryonic developmental success, based on FDRs determined as described in the examples below. All of the genes listed in Table B1 and Table B2 have FDRs of less than 0.01. The most statistically significant gene (i.e. having the lowest FDR) is listed first in each table, with the subsequently listed genes having sequentially lower levels of statistical significance.

Thus, in preferred embodiments, there is provided the use of the most statistically significantly enriched transcript from Table B1 (which is transcript NM_001188) or Table B2 (which is NR_126449), or an expression product or fragment of either transcript, as a biomarker for reduced likelihood of blastocyst implantation success.

In some embodiments, there is provided the use of a plurality of the most statistically significantly enriched transcripts from Table B1, or expression products or fragments thereof, in or as a biomarker panel for reduced likelihood of blastocyst implantation success. For example, in some embodiments, there is provided the use of at least the first two (i.e. most statistically significantly correlated) transcripts from Table B1, or expression products or fragments thereof, in or as a biomarker panel for reduced likelihood of blastocyst implantation success. For example, in some embodiments, there is provided the use of at least the first 3, 4, 5, 6, 7, 8, 9, 10, 12, 15, 18, 20, 25, 30, 35, 40, 45, or 50 (i.e. most statistically significantly correlated) transcripts from Table B1, or expression products or fragments thereof, in or as a biomarker panel for reduced likelihood of blastocyst implantation success. In some embodiments, there is provided the use of at least 2, 3, 4, 5, 6, 7, 8, 9, or 10 of the first 10 (i.e. most statistically significantly enriched) transcripts from Table B1, or expression products or fragments thereof, in or as a biomarker panel for reduced likelihood of blastocyst implantation success. In some embodiments, there is provided the use of at least 2, 3, 4, 5, 6, 7, 8, 9, 10, 12, 15, 18, 20, 25, 30, 35, 40, 45, or 50 of the first 50 (i.e. most statistically significantly correlated) transcripts from Table B1, or expression products or fragments thereof, in or as a biomarker panel for reduced likelihood of blastocyst implantation success.

In some embodiments, there is provided the use of at least the two most statistically significantly enriched transcripts from Table B2, or expression products or fragments thereof, in or as a biomarker panel for reduced likelihood of blastocyst implantation success. For example, in some embodiments, there is provided the use of at least the first 3, 4, 5, 6, 7, 8, 9, 10, 12, 15, or 18 (i.e. most statistically significantly correlated) transcripts from Table B2, or expression products or fragments thereof, in or as a biomarker panel for reduced likelihood of blastocyst implantation success. In some embodiments, there is provided the use of at least 2, 3, 4, 5, 6, 7, 8, 9, or 10 of the first 10 (i.e. most statistically significantly correlated) transcripts from Table B2, or expression products or fragments thereof, in or as a biomarker panel for reduced likelihood of blastocyst implantation success. In some embodiments, there is provided the use of at least 2, 3, 4, 5, 6, 7, 8, 9, 10, 12, 15, or 18 of the first 18 (i.e. most statistically significantly enriched) transcripts from Table B2, or expression products or fragments thereof, in or as a biomarker panel for reduced likelihood of blastocyst implantation success.

Throughout this disclosure, references to "enrichment" or "enriched" genes or gene transcripts refers generally to genes, gene transcripts, expression products, and fragments thereof statistically significantly higher expressed in the YMA/rba-YMA and/or AMA/rba-AMA samples. "Enrichment" or "enriched" also refers to gene ontologies, biological processes or cellular components of statistical significance (FDR<0.05).

In some embodiments, there is provided the use of all of the genes or gene transcripts listed in Table B and Table D in combination, or expression products or fragments thereof, in or as a biomarker panel for reduced likelihood of blastocyst implantation success.

In some embodiments, there is provided the use of all of the genes or gene transcripts listed in Table B, or expression products or fragments thereof, in or as a biomarker panel for reduced likelihood of blastocyst implantation success.

In some embodiments, there is provided the use of all of the genes or gene transcripts listed in Table D, or expression products or fragments thereof, in or as a biomarker panel for reduced likelihood of blastocyst implantation success.

In some embodiments, there is provided the use of all of the genes or gene transcripts listed in Table B1, or expression products or fragments thereof, in or as a biomarker panel for reduced likelihood of blastocyst implantation success.

In some embodiments, there is provided the use of all of the genes or gene transcripts listed in Table B2, or expression products or fragments thereof, in or as a biomarker panel for reduced likelihood of blastocyst implantation success.

In some embodiments, there is provided the use of a combination of at least two genes selected from those listed in Table A and Table B or expression products or fragments thereof, as these biomarkers have been reported as differentially expressed between the YMA/rba-YMA and AMA/rba-AMA groups with high confidence (FDR<0.01), suggesting that these biomarkers could best characterise the YMA/rba-YMA and AMA/rba-AMA samples.

Thus, in some embodiments, there is provided the use of at least two genes and/or gene transcripts selected from any of those listed in Table A and Table B, or expression products or fragments thereof, in or as a biomarker panel for blastocyst implantation success or for determining the likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A and Table B, or expression products or fragments thereof, in or as a biomarker panel for blastocyst implantation success or for determining the likelihood of blastocyst implantation success.

In some embodiments. there is provided the use of at least one gene, gene transcript, expression product, or fragment thereof selected from Table A in combination with at least one gene, gene transcript, expression product, or fragment thereof selected from Table B, in or as a biomarker panel, for blastocyst implantation success or for determining the likelihood of blastocyst implantation success.

In some embodiments. there is provided the use of all of the genes, gene transcripts, expression products, or fragments thereof selected from Table A in combination with all of the genes, gene transcripts, expression products, or fragments thereof selected from Table B, in or as a biomarker panel, for blastocyst implantation success or for determining the likelihood of blastocyst implantation success.

In some embodiments, there is provided the use of at least two genes and/or gene transcripts selected from any of those listed in Table A1 and Table B1, or expression products or fragments thereof, in or as a biomarker panel for blastocyst implantation success or for determining the likelihood of blastocyst implantation success. For example, in some embodiments, there is provided the use of at least 3, 4, 5, 6, 7, 8, 9, 10, 12, 15, 18, 20, 25, 30, 35, 40, 45, or 50 genes and/or gene transcripts selected from any of those listed in Table A1 and Table B1, or expression products or fragments thereof, in or as a biomarker panel for blastocyst implantation success or for determining the likelihood of blastocyst implantation success.

In some embodiments, there is provided the use of at least one gene, gene transcript, expression product, or fragment thereof selected from Table A1 in combination with at least one gene, gene transcript, expression product, or fragment thereof selected from Table B1, in or as a biomarker panel, for blastocyst implantation success or for determining the likelihood of blastocyst implantation success.

In some embodiments, there is provided the use of all of the genes, gene transcripts, expression products, or fragments thereof selected from Table A1 in combination with all of the genes, gene transcripts, expression products, or fragments thereof selected from Table B1, in or as a biomarker panel, for blastocyst implantation success or for determining the likelihood of blastocyst implantation success.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A, Table B, Table C, and/or Table D, or expression products or fragments thereof, as biomarkers for blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A*, Table B*, Table C*, and/or Table D*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A or Table C, or expression products or fragments thereof, for use as biomarkers for increased likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A* and/or Table C*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table B or Table D, or expression products or fragments thereof, for use as biomarkers for reduced likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table B* and/or Table D*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A, or expression products or fragments thereof, in a biomarker panel for increased likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A1, or expression products or fragments thereof, in a biomarker panel for increased likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A1*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table B, or expression products or fragments thereof, in a biomarker panel for increased likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table B*.

In some embodiments, there is provided the use of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table B1, or expression products or fragments thereof, in a biomarker panel for increased likelihood of blastocyst implantation success, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table B1*.

A gene or gene transcript, or a panel of genes or gene transcripts, may be selected for use as a biomarker or in a biomarker panel in accordance with the first aspect, on the basis of biological function.

Thus, in some embodiments, there is provided the use of a combination of at least two genes selected from those listed in Table A, Table B, Table C, and/or Table D, or expression products or fragments thereof, that are involved in the same specific biological process, in or as a biomarker panel for blastocyst implantation success. The specific biological process may be or may comprise, for example, exosome function, embryo-endometrial communication, and/or apoptotic process.

In some embodiments, there is provided the use of a combination of at least two genes selected from those listed in Table A, Table B, Table C, and/or Table D, or expression products or fragments thereof, that are involved in the same specific biological process, together with at least one further gene selected from those listed in Table A, Table B, Table C, and/or Table D, or expression products or fragments thereof, that are not directly involved or known to be involved in the same specific biological process, in or as a biomarker panel for blastocyst implantation success. This does not exclude the possibility of using at least two genes and/or gene transcripts selected from those listed in Table A, Table B, Table C, and/or Table D, or expression products or fragments thereof, without being involved in any specific ontologies or biological processes.

Table E1 lists genes selected from Table A and Table C that are involved in exosome function.

Thus, in some embodiments, there is provided the use of at least 1 gene or gene transcript selected from any of those listed in Table E1, or expression products or fragments thereof, as a biomarker or biomarker panel for increased likelihood of blastocyst implantation success. For example, in some embodiments, there is provided the use of at least 2, 3, 4, 5, 6, 7, 8, 9, 10, 12, 15, 18, 20, 25, 30, 35, 40, 45, 50 or all of the genes or gene transcripts selected from any of those listed in Table E1, or expression products or fragments thereof, in or as a biomarker panel for blastocyst implantation success.

The genes PEBP1, PSMA5, UBB, DAB2, HSP90AA1, HSPB11, WNT7A, and PRDX5, which are involved in exosome function and listed in Table E1, are thought to be particularly useful as biomarkers, in or as a biomarker panel, or for inclusion in a biomarker panel, for increased likelihood of blastocyst implantation success.

Thus, in some embodiments, there is provided the use of at least 1 gene selected from PEBP1, PSMA5, UBB, DAB2, HSP90AA1, HSPB11, WNT7A, and PRDX5, or expression products or fragments thereof, as a biomarker, or for inclusion in a biomarker panel, for increased likelihood of blastocyst implantation success. In some embodiments, there is provided the use of at least 2, 3, 4, 5, 6, 7, or all 8 of these genes, gene transcripts, expression products, or fragments thereof, in or as a biomarker panel for increased likelihood of blastocyst implantation success.

In some embodiments, there is provided the use of at least 35 genes and/or gene transcripts selected from any of those listed in Table A and/or Table C, or expression products or fragments thereof, in or as a biomarker panel for increased likelihood of blastocyst implantation success, wherein the selected genes and/or gene transcripts, expression products, or fragments thereof, comprise at least 7, such as at least 8, 9, 10, 12, 15, 18, 20, 25, 30, or 35, genes or gene transcripts in Table E1, or expression products or fragments thereof. In some embodiments, there is provided the use of at least 35, such as at least 40, 45, 50, or all, genes and/or gene transcripts selected from any of those listed in Table A and/or Table C, or expression products or fragments thereof, in or as a biomarker panel for increased likelihood of blastocyst implantation success, wherein the selected genes and/or gene transcripts, expression products, or fragments thereof, comprise at least 7 of the genes or gene transcripts in Table E1, or expression products or fragments thereof.

Table E2 lists genes selected from Table A or Table C that have been identified in knock-out mouse studies to be involved in repressing and/or compromising early embryo development and/or implantation process when absent. Thus, the genes/transcripts listed in Table E2 are not only involved in successful embryonic implantation, but are likely to also have an increased level of expression in embryos with better viability during the early developmental stages, with this outcome represented in the implantation success. Combination of our human along with mouse knockout findings, support the current findings on the effect of Table E2 factors in implantation success and/or embryonic development. Combination of our human along with mouse knockout findings, support the current findings on the effect of Table E2 factors in implantation success and/or embryonic development.

Thus, in some embodiments, there is provided the use of at least 1 gene or gene transcript selected from any of those listed in Table E2, or expression products or fragments thereof, as a biomarker, or for inclusion in a biomarker panel, for increased likelihood of blastocyst implantation success. For example, in some embodiments, there is provided the use of at least 3, 4, 5, 6, 7, 8, 9, 10, 11, or all of the genes or gene transcripts selected from any of those listed in Table E2, or expression products or fragments thereof, in or as a biomarker panel for increased likelihood of blastocyst implantation success.

Table E3 lists genes selected from Table A or Table C that have been identified as being involved in embryo-endometrial communication, based on the analysis conducted in the current invention.

Without wishing to be bound by any specific theory, it appears that the significantly more highly expressed genes and gene transcripts in the YMA/rba-YMA group (but not the AMA/rba-AMA) have potential molecular interactions with the significantly more highly expressed genes in the receptive endometrium (when compared with the non-receptive). The genes/transcripts provided in Table E3, in particular, that are identified as having an increased level of expression in the YMA/rba-YMA group, may participate in embryo-endometrial interactions. Considering that human embryo-endometrial communications cannot be studied *in vivo* due to various ethical restrictions, this is one of the closest methods to a functional approach that it is possible to get, combining the transcriptome data from the trophectoderm/embryo together with the receptive endometrial data. Despite the fact that the main differentially expressed transcripts are reported in both YMA/rba-YMA and AMA/rba-AMA groups, only YMA/rba-YMA genes/transcripts (and not the AMA/rba-AMA) correspond to gene ontologies/biological processes that could delineate successful embryo-endometrial communication that could be considered as an additional "proof" of their potential role during implantation.

Thus, in some embodiments, there is provided the use of at least 1 gene or gene transcript selected from any of those listed in Table E3, or expression products or fragments thereof, as a biomarker or for inclusion in a biomarker panel for increased likelihood of blastocyst implantation success. For example, in some embodiments, there is provided the use of at least 2, 3, 4, 5, 6, 7, 8, 9, 10, 12, 15, 18, 20, 25, 30, 35, 40, 45, 50 or all of the genes or gene transcripts selected from any of those listed in Table E3, or expression products or fragments thereof, in or as a biomarker panel for blastocyst implantation success.

In particular, a number of gene products (proteins) of the genes listed in Table E3 have previously been found to be present in the extracellular area of the blastocoele (cavity inside the human blastocyst) following blastocentesis (process of retrieving the blastocyst fluid from the internal part of the blastocyst) (Poli et al., 2015). This study characterised the blastocoele protein content secreted by individual human blastocysts, although the results were not associated with the maternal age nor the implantation success of the corresponding embryos as defined in the current study. The extracellular exosome related PRDX2, PSMA5, HSP90AA1, PSMA7, TAGLN2 and FABP5 gene products reported in the current study, are present in the blastocyst fluid at the protein level. The identification of 8 YMA/rba-YMA and only 3 AMA/rba-AMA profile gene products (proteins) in the blastocoele, support the hypothesis that the reported genes participate in embryo-endometrial communication, considering that they are present in the extracellular space.

Thus, in some embodiments, there is provided the use of at least 1 gene selected from HSP90AA1, PRDX2, PSMA5, TAGLN2, FABP5 and PSMA7, or an expression product or fragment thereof, as a biomarker or for inclusion in a biomarker panel for increased likelihood of blastocyst implantation success. For example, in some embodiments, there is provided the use of at least 2, 3, 4, 5, or all 6 of these genes, genes transcripts, expression products, or fragments thereof, in or as a biomarker panel for blastocyst implantation success. The use of one or more of these genes as a biomarker or in a biomarker panel for blastocyst implantation success may be particularly advantageous when the sample consists of or comprises material that is, or has previously been, collected from the blastocoele, and in particular, from the blastocyst fluid using blastocentesis and/or the culture media, where the blastocyst secreted proteins and/or nucleic acids, such as coding and non-coding transcripts.

The genes DAB2, DBI, FABP3, and IFITM3, which are involved in embryo-endometrial communication and listed in Table E3, are thought to be particularly useful as biomarkers, or for inclusion in a biomarker panel, for increased likelihood of blastocyst implantation success.

Thus, in some embodiments, there is provided the use of at least 1 gene selected from DAB2, DBI, FABP3, and IFITM3, or an expression product or fragment thereof, as a biomarker, or for inclusion in a biomarker panel, for increased likelihood of blastocyst implantation success. In some embodiments, there is provided the use of at least 2, 3, or all 4 of these genes, or expression products or fragments thereof, in or as a biomarker panel for blastocyst implantation success.

Table E4 lists genes and gene transcripts selected from Tables B and Table D that have been identified as being involved in the apoptotic process.

Thus, in some embodiments, there is provided the use of at least 1 gene or gene transcript selected from any of those listed in Table E4, or expression products or fragments thereof, as a biomarker, or for inclusion in a biomarker panel, for reduced likelihood of blastocyst implantation success. For example, in some embodiments, there is provided the use of at least 2, 3, 4, 5, 6, 7, 8, 9, 10, 12, 15, 18, 20, 25, or all of the genes or gene transcripts selected from any of those listed in Table E4, or expression products or fragments thereof, as a biomarker, or for inclusion in a biomarker panel, for blastocyst implantation failure, hence suggesting the blastocysts with lower likelihood to implant.

In particular, BAK1 has a significantly higher level of expression in non-implanted blastocysts (AMA/rba-AMA group). Without wishing to be bound by any specific theory, it is thought that BAK1 and/or other relevant transcripts, could be associated with an underlying apoptotic process. Such a process may take place at the molecular level, without being obvious in the morphological assessment of the blastocyst (performed by the clinical embryologists).

Thus, in some embodiments, there is provided the use of BAK1 or an expression product or fragment thereof, as a biomarker for blastocyst implantation success, and specifically, as a biomarker for reduced likelihood of blastocyst implantation success.

The more genes, gene transcripts, expression products, or fragments thereof that are used as biomarkers or in a biomarker panel of the first aspect, the greater the accuracy and reliability with which the likelihood of implantation success of the blastocyst can be predicted.

The inventors have developed a high throughput methodology comprising blastocyst samples representing the full spectrum of maternal reproductive ages with embryo implantation potential examined in relation to the transcriptome dynamics and to the reproductive maternal age.

The use of at least 1 gene and/or gene transcript selected from those listed in Table A, Table B, Table C, and/or Table D, or an expression product or fragment thereof, as a biomarker, or in or as a biomarker panel, for blastocyst implantation success in accordance with the first aspect may involve any suitable method or analysis.

Therefore, according to a second aspect, there is provided a method of identifying the likelihood of implantation success of a blastocyst for potential embryo transfer, the method comprising:
determining, in a test sample obtained from the blastocyst, the level of expression of at least 1 gene or gene transcript selected from the genes and gene transcripts listed in Table A, Table B, Table C, and/or Table D, or an expression product or fragment thereof;
comparing the level of expression of the at least 1 gene, gene transcript, expression product, or fragment thereof, in the test sample, with the level of expression of the at least 2 genes, gene transcripts, expression products, or fragments thereof, in a reference sample;
wherein the level of expression of the at least 1 gene, gene transcript, expression product, or fragment thereof, in the test sample relative to the level of expression in the reference sample identifies the likelihood of implantation success of the blastocyst.

In some embodiments, the method comprises:
determining, in a test sample obtained from the blastocyst, the level of expression of at least 1 gene or gene transcript selected from the genes and gene transcripts listed in Table A and/or Table C, or an expression product or fragment thereof;
comparing the level of expression of the at least 1 gene, gene transcript, expression product, or fragment thereof, in the test sample, with the level of expression of the corresponding at least one gene, gene transcript, expression product, or fragment thereof, in a reference sample;
wherein the level of expression of the at least 1 gene, gene transcript, expression product, or fragment thereof, in the test sample relative to the level of expression in the reference sample identifies the blastocyst as having an increased likelihood of implantation success.

In some embodiments, the method comprises:
determining, in a test sample obtained from the blastocyst, the level of expression of at least 1 gene or gene transcript selected from the genes and gene transcripts listed in Table B and/or Table D, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample;
wherein the level of expression of the at least 1 gene, gene transcript, expression product, or fragment thereof, in the test sample relative to the level of expression in the reference sample identifies the blastocyst as having a reduced likelihood of implantation success.

The choice of the at least 1 gene or gene transcript selected from the genes and gene transcripts listed in Table A, Table B, Table C, and/or Table D, or an expression product or fragment thereof in relation to the method of the second aspect is as defined in relation to the use of the first aspect.

Thus, in some embodiments, a method of the second aspect as set out above may comprise the use of any gene/transcript or combination of genes/transcripts or expression products or fragments thereof listed in Table A and/or Table C, as defined in accordance with the first aspect above, as a biomarker to identify a blastocyst as having an increased likelihood of implantation success. For example, the method may comprise the use as defined in accordance with the first aspect as a biomarker to identify a blastocyst as having a young reproductive chronological (YMA) and/or biological maternal age (rba-YMA), and thereby as having an increased likelihood of implantation success.

In other embodiments, a method of the second aspect as set out above may comprise the use of any gene/transcript or combination of genes/transcripts or expression products or fragments thereof listed in Table B and/or Table D, as defined in accordance with the first aspect above, as a biomarker to identify a blastocyst as having a reduced likelihood of implantation success. For example, the method may comprise the use as defined in accordance with the first aspect as a biomarker to identify a blastocyst as having an advanced reproductive chronological (AMA) and/or biological maternal age (rba-AMA), and thereby as having a reduced likelihood of implantation success.

In preferred embodiments, any of the methods of this disclosure may or may not include as an initial step testing the samples for aneuploidy and the removal of aneuploid samples from the embryo transfer process, such as by using the Preimplantation Genetic Testing for Aneuploidy (PGT-A) method.

In a preferred embodiment, the test and reference samples may be analysed under the same or substantially similar experimental conditions.

In some embodiments, the reference and test samples may consist of the same sample type, and suitable sample types are described above in relation to the first aspect.

In other embodiments, the reference sample may be a different type of sample to the test sample, and may comprise, for example, a preparation of cDNA, RNA, protein, and/or other material such as a preparation of extracellular exosomes.

In some embodiments, the reference sample may comprise a control that corresponds to the level of expression of the at least 1 gene, gene transcript, expression product, or fragment thereof in a sample that is known to be representative of a blastocyst having a young reproductive chronological (YMA) and/or biological maternal age (rba-YMA) or an advanced reproductive chronological (AMA) and/or biological maternal age (rba-AMA), as appropriate.

In some embodiments, a correlation or similarity in the level of expression of the at least 1 gene, gene transcript, expression product, or fragment thereof in the test sample with the level of expression in the reference sample identifies the test sample as being in the same YMA/rba-YMA or AMA/rba-AMA group as the reference sample.

Conversely, in some embodiments, a significant difference in the level of expression of the at least 1 gene, gene transcript, expression product, or fragment thereof in the test sample with the level of expression in the reference sample identifies the test sample as being in the opposite YMA/rba-YMA or AMA/rba-AMA group to the reference sample.

Thus, in some embodiments, the method comprises:
determining, in a test sample obtained from the blastocyst, the level of expression of at least 1 gene or gene transcript selected from the genes and gene transcripts listed in Table A and/or Table C, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample, wherein the reference sample comprises a control that corresponds to the level of expression of the at least 1 gene, gene transcript, expression product, or fragment thereof in a sample that is known to be representative of a blastocyst having a young reproductive chronological (YMA) and/or biological maternal age (rba-YMA);
wherein a corresponding level of expression of the at least 1 gene, gene transcript, expression product, or fragment thereof, in the test sample relative to the level of expression in the reference sample identifies the blastocyst as having an increased likelihood of implantation success.

In some embodiments, the method comprises:
determining, in a test sample obtained from the blastocyst, the level of expression of at least 1 gene or gene transcript selected from the genes and gene transcripts listed in Table A and/or Table C, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample, wherein the reference sample comprises a control that corresponds to the level of expression of the at least 1 gene, gene transcript, expression product, or fragment thereof in a sample that is known to be intermediate or average between the levels of expression in blastocysts representative of YMA/rba-YMA and AMA/AMA-rba;
wherein a corresponding or increased level of expression of the at least 1 gene, gene transcript, expression product, or fragment thereof, in the test sample relative to the level of expression in the reference sample identifies the blastocyst as having an increased likelihood of implantation success.

In some embodiments, the method comprises:
determining, in a test sample obtained from the blastocyst, the level of expression of at least 1 gene or gene transcript selected from the genes and gene transcripts listed in Table A and/or Table C, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample, wherein the reference sample comprises a control that corresponds to the level of expression of the at least 1 gene, gene transcript, expression product, or fragment thereof in a sample that is known to be representative of a blastocyst having an advanced reproductive chronological (AMA) and/or biological age (rba-AMA);
wherein an increased level of expression of the at least 1 gene, gene transcript, expression product, or fragment thereof, in the test sample relative to the level of expression in the reference sample identifies the blastocyst as having an increased likelihood of implantation success.

In some embodiments, the method comprises:
determining, in a test sample obtained from the blastocyst, the level of expression of at least 1 gene or gene transcript selected from the genes and gene transcripts listed in Table B and/or Table D, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample, wherein the reference sample comprises a control that corresponds to the level of expression of the at least 1 gene, gene transcript, expression product, or fragment thereof in a sample that is known to be representative of a blastocyst having a young reproductive chronological (YMA) and/or biological maternal age (rba-YMA);
wherein an increased level of expression of the at least 1 gene, gene transcript, expression product, or fragment thereof, in the test sample relative to the level of expression in the reference sample identifies the blastocyst as having a reduced likelihood of implantation success.

In some embodiments, the method comprises:
determining, in a test sample obtained from the blastocyst, the level of expression of at least 2 genes or gene transcripts selected from the genes and gene transcripts listed in Table B and/or Table D, or an expression product or fragment thereof;
comparing the level of expression of the at least 2 genes, gene transcripts, expression products, or fragments thereof, in the test sample, with the level of expression of the corresponding at least 2 genes, gene transcripts, expression products, or fragments thereof, in a reference sample, wherein the reference sample comprises a control that corresponds to the level of expression of the at least 2 genes, gene transcripts, expression products, or fragments thereof in a sample that is known to be intermediate or average between the levels of expression in blastocysts representative of YMA/rba-YMA and AMA/AMA-rba;
wherein an increased level of expression of the at least 2 genes, gene transcripts, expression products, or fragments thereof, in the test sample relative to the level of expression in the reference sample identifies the blastocyst as having a reduced likelihood of implantation success.

In some embodiments, the method comprises:
determining, in a test sample obtained from the blastocyst, the level of expression of at least 1 gene or gene transcript selected from the genes and gene transcripts listed in Table B and/or Table D, or an expression product or fragment thereof;
comparing the level of expression of the at least 1 gene, gene transcript, expression product, or fragment thereof, in the test sample, with the level of expression of the corresponding at least 1 gene, gene transcript, expression product, or fragment thereof, in a reference sample, wherein the reference sample comprises a control that corresponds to the level of expression of the at least 1 gene, gene transcript, expression product, or fragment thereof in a sample that is known to be representative of a blastocyst having an advanced reproductive chronological (AMA) and/or biological age (rba-AMA);
wherein a corresponding level of expression of the at least 1 gene, gene transcript, expression product, or fragment thereof, in the test sample relative to the level of expression in the reference sample identifies the blastocyst as having a reduced likelihood of implantation success.

In any of these methods, the choice of the at least 1 gene or gene transcript selected from the genes and gene transcripts listed in Table A, Table B, Table C, and/or Table D, or an expression product or fragment thereof may be as defined in relation to the use of the first aspect, for example selected from one or a combination of genes, gene transcripts, expression products, or fragments thereof selected from the genes and gene transcripts listed in Table A, Table B, Table C, and/or Table D, and /or Table A*, Table B*, Table C*, and/or Table D*.

In some embodiments, the method comprises:
determining, in a test sample obtained from the blastocyst, the level of expression of at least 35, such as at least 40, 45, 50, or all, of the genes or gene transcripts selected from the genes and gene transcripts listed in Table A and/or Table C, or expression products or fragments thereof;
comparing the level of expression of the selected genes, gene transcripts, expression products, or fragments thereof, in the test sample, with the level of expression of the corresponding selected genes, gene transcripts, expression products, or fragments thereof, in a reference sample;
wherein the level of expression of the selected genes, gene transcripts, expression products, or fragments thereof, in the test sample relative to the level of expression in the reference sample identifies the likelihood of implantation success of the blastocyst.

In some embodiments, the method further comprises:
determining, in the test sample, the level of expression of at least one stably expressed gene, gene transcript, expression product, or fragment thereof;
comparing the level of expression of the at least one stably expressed gene, gene transcript, expression product, or fragment thereof in the test sample, with the level of expression of the at least one stably expressed gene, gene transcript, expression product, or fragment thereof, in a reference sample;
wherein a difference in the relative level of expression of the corresponding selected genes, gene transcripts, expression products, or fragments thereof listed in Table A and/or Table C, versus the relative level of expression of the at least one stably expressed gene, gene transcript, expression product, or fragment thereof, identifies the likelihood of implantation success of the blastocyst.

The at least one stably expressed gene, gene transcript, expression product, or fragment thereof may include any one, such as at least any 2, 3, 4, 5, 6, 7, 8, 9, 10, 12, 15, 18, 20, 25, 30, 35, 40, 45, 50, or all, of the genes listed in Table F.

The more genes, gene transcripts, expression products, or fragments thereof that are analysed in the disclosed methods, the greater the expected accuracy and reliability with which the likelihood of implantation success of the blastocyst can be predicted.

Thus, in some embodiments, the method may comprise the use of two or more reference samples. The reference samples may represent the same YMA/rba-YMA or AMA/rba-AMA groups. Each reference sample may comprise a number of controls corresponding to each of the at least 1 gene, gene transcript, expression product, or fragment thereof used in the method.

Thus, in some embodiments, the method may comprise the use of two or more reference samples, including at least a first reference sample comprising a control that corresponds to the level of expression of the at least 2 genes, gene transcripts, expression products, or fragments thereof in the YMA/rba-YMA group and at least a second reference sample comprising a control that corresponds to the level of expression of the at least 2 genes, gene transcripts, expression products, or fragments thereof in the AMA/rba-AMA group. Thus, a correlation in the level of expression of the at least 2 genes, gene transcripts, expression products, or fragments thereof in the test sample with the level of expression in one of the first or second reference samples, and a significant difference in the level of expression relative to the other reference sample, identifies the test sample as being in the YMA/rba-YMA or AMA/rba-AMA group with which the level of expression is correlated.

In some embodiments, the reference sample may comprise a control that corresponds to the gene expression profile or transcriptome or proteome of one of the YMA/rba-YMA or AMA/rba-AMA groups.

In some embodiments, the reference sample or samples may comprise a plurality of controls that corresponds to the gene expression profiles or transcriptomes or proteomes of both YMA/rba-YMA and AMA/rba-AMA groups.

In some embodiments, the reference sample may correspond to the average (or median) level of expression of the at least 1 gene, gene transcript, expression product, or fragment thereof, between the YMA/rba-YMA and AMA/rba-AMA groups. The deviation of the level of expression between the test sample and such a reference sample may be used to determine the likelihood of implantation success. For example, when the at least 1 gene, gene transcript, expression product, or fragment thereof, in the test sample that is greater than the expression in this reference sample, may identify the at least 1 gene, gene transcript, expression product, or fragment thereof as having an increased level of expression.

Such a reference sample comprising, or representative of, the mean (or median) level of expression of the at least 1 gene, gene transcript, expression product, or fragment thereof, between the YMA/rba-YMA and AMA/rba-AMA groups, may be produced, for example, by mixing samples from similar gene expression profiles to the YMA/rba-YMA and AMA/rba-AMA groups and/or samples having similar gene expression profiles to the YMA/rba-YMA and AMA/rba-AMA groups. For example, samples of different maternal ages, covering the whole age spectrum between YMA/rba-YMA and AMA/rba-AMA groups, may be mixed together to provide gene expression profile(s) equivalent to an average implantation competency. The reference sample may comprise, or be representative of, a sample of the intermediate reproductive maternal age (intermediate maternal age - IMA group).

In some embodiments, the reference sample may comprise, may correspond to, and/or may be representative of an expression level of the at least 1 gene, gene transcript, expression product, or fragment thereof, that is between the levels of expression in the YMA/rba-YMA and AMA/rba-AMA groups, for example, as reported in the current study. For example, in some embodiments, according to the current dataset, a reference gene expression level/profile of the at least 1 gene, gene transcript, expression product, or fragment thereof may be derived by mixing (computationally or not) samples from YMA/rba-YMA and AMA/rba-AMA groups or use of the YMA/rba-YMA and/or AMA/rba-AMA datasets as references.

Comparing the level of expression of the at least 1 gene, gene transcript, expression product, or fragment thereof in the test sample with the level of expression of the corresponding at least 1 gene, gene transcript, expression product, or fragment thereof in a reference sample may comprise directly comparing the gene expression levels, such as, for example, the absolute numbers of transcripts and/or normalised number of transcripts and/or number of normalised transcripts alone. The absolute levels of expression may be those provided in the current disclosure and set out below. Thus, the values set out in Tables A-D below may be used as reference samples defining the YMA/rba-YMA and AMA/rba-AMA groups, against which absolute levels of expression and/or normalised numbers of transcripts of the at least 1 gene, gene transcript, expression product, or fragment thereof, in the test sample may be compared, to determine the relative level of expression and thereby an increased or decreased likelihood of implantation success, respectively.

In some embodiments, the level of expression of the at least 1 gene, gene transcript, expression product, or fragment thereof in the test sample and/or reference sample may be normalised. For example, in some embodiments, the level of expression may be normalised against at least one selected housekeeping gene, using for example the ΔCt method (Livak and Schmittgen, 2001), or at least 2, 3, 4, 5, 6, 7, 8, 9, or 10 housekeeping genes using i.e. the mean or geometric mean, for example, when a qRT-PCR method is used. In some embodiments, the level of expression may be normalised against a large number of genes or transcripts, such as the whole transcriptome or large part of it, for example, when an RNA-Seq method is employed to detect the investigated transcripts.

In some embodiments, at least 2 housekeeping genes may be used for normalisation.

Throughout this disclosure, references to "housekeeping" genes refers generally to genes, gene transcripts, expression products, and fragments thereof that may be used for reference or control purposes in the disclosed methods, for example due to being expressed at substantially the same level in the YMA/rba-YMA and AMA/rba-AMA groups. Thus, the term "housekeeping" in relation to such genes, gene transcripts, expression products, and/or fragments thereof may be used interchangeably with the terms "stably expressed", "reference", and/or "control", for example.

The housekeeping genes that may be used include any one or any combination of the genes and gene transcripts listed in Table F. The genes and gene transcripts in Table F have been found to be expressed at substantially the same level in both the YMA/rba-YMA and AMA/rba-AMA groups (coefficient of variation (CV) <20%). Other housekeeping genes that are known in the art may additionally or alternatively be used, such as for example, including *H3F3B, GAPDH, ACTB* and/or *HNRNPC.* The latter housekeeping genes have been demonstrated by the current study in the context of a qRT-PCR approach, that was used to determine the levels of expression of transcripts significantly higher expressed in the YMA/rba-YMA and/or AMA-rba-AMA group.

The RNA-Seq data analysis described in the Examples below was confirmed by qRT-PCR verifying the data derived from the high-throughput gene expression analysis. Different metrics were investigated as part of the differential expression analysis (edgeR package in R) between YMA/rba-YMA and AMA/rba-AMA groups, including among others the raw and normalised read counts (assigned into features i.e. genes), the fold-change (FC), copies-per-million (CPM) and/or their logarithmic transformations, such as logFC and logCPM, the p-value and false discovery rate adjusted p-value (FDR). FC, for example, is expressed here either as a ratio or as a logarithm of the ratio. These metrics are provided for each significantly differentially expressed marker.

Thus, throughout this disclosure, in some embodiments, the disclosed methods may comprise among others the use of the FC, CPM and/or their logarithmic (or other) transformations, such as logFC and logCPM; the Reads Per Kilobase of transcript per Million mapped reads (RPKM); the Fragments Per Kilobase of transcript per Million mapped reads (FPKM); and Transcripts Per Million (TPM) metrics. For example, in some embodiments, FC, CPM, RPKM/FPKM and TPM metrics may be used separately for determining the level of expression of the at least one gene, gene transcript, expression product, fragment thereof in the test sample and/or reference sample.

In other embodiments, FC and CPM or RPKM/FPKM or TPM metrics may be used in combination for determining the level of expression of the at least one gene, gene transcript, expression product, or fragment thereof in the test sample and/or reference sample.

The FC metric shows the difference in the level of expression of the at least one gene, gene transcript, expression product, or fragment thereof between YMA/rba-YMA and AMA/rba-AMA groups and is depicted as fold-change, reporting an approximation of the minimum difference of a particular gene/transcript in order for the tested sample(s) to be considered as implantation competent (or not competent).

Thus, in some embodiments, the FC metric may be used to determine the fold change difference between the YMA/rba-YMA and AMA/rba-AMA samples. For example, separate control samples representative of each of the YMA/rba-YMA and AMA/rba-AMA and/or an averagely expressed reference sample may be used to determine a measure for the relative difference in the level of expression of the at least one gene, gene transcript, expression product, or fragment thereof in the control samples. Using this measure, the test sample may be classified in the YMA/rba-YMA or AMA/rba-AMA group, for example, by comparing the level of expression of the at least one gene, gene transcript, expression product, or fragment thereof in the test sample to the level of expression in the YMA/rba-YMA and/or AMA/rba-AMA control groups. Thus, the test sample may be identified as being in the YMA/rba-YMA or AMA/rba-AMA group with which the level of expression is compared and/or correlated.

In some embodiments, the CPM/logCPM metric may be used to determine the relative difference between the level of expression of the at least one gene, gene transcript, expression product, or fragment thereof and one or more housekeeping genes or transcripts in the test and reference samples. The logCPM relies on the fact that the read distribution of the reported genes is about the same and corresponds to the Fragments Per Kilobase of transcript per Million mapped reads (FPKM) plus the log(2) of gene length. For example, the relative difference of the CPM between the at least one gene, expression product or fragment thereof and one or more housekeeping genes in a sample may be compared with the relative difference of the CPM between the same at least one gene, expression product or fragment thereof and one or more housekeeping genes in one or both of the YMA/rba-YMA and AMA/rba-AMA groups and the stable expressed housekeeping gene(s) in the same groups. In some embodiments, instead of the CPM metric, normalised read counts may be employed or raw (not normalised) read counts that may be normalised according to the gene expression levels of certain housekeeping (reference) genes or gene transcripts, such as the ones described herein and/or those reported in Table F.

The inventors have also found that samples may be identified as having either an increased or reduced likelihood of implantation success without comparison to a reference sample, for example, by relying on the use set out and described above in accordance with the first aspect, of the significantly differentially expressed YMA/rba-YMA and AMA/rba-AMA biomarkers, and comparing the level of expression of these biomarkers to the level of expression of genes that are known to be substantially uniformly expressed in both the YMA/rba-YMA and AMA/rba-AMA groups. Thus, the relative expression of the YMA/rba-YMA and/or AMA/rba-AMA biomarkers with respect to certain stably expressed genes, such as housekeeping genes, as well as in the context of the existing YMA/rba-YMA and AMA/rba-AMA datasets, may be used to identify and determine the likelihood of implantation success.

Thus, according to a third aspect, there is provided a method of identifying the likelihood of implantation success of a blastocyst for potential embryo transfer, the method comprising:
determining, in a test sample obtained from the blastocyst, the level of expression of at least one biomarker gene or transcript selected from the genes and transcripts listed in Table A, Table B, Table C, and/or Table D, or an expression product or fragment thereof;
determining, in the test sample, the level of expression of at least one stably expressed gene, gene transcript, expression product, or fragment thereof; and
comparing the level of expression of the at least one biomarker gene, gene transcript, expression product, or fragment thereof, in the test sample, with the level of expression of at least one stably expressed gene, gene transcript, expression product, or fragment thereof;
wherein the level of expression of the at least one biomarker gene, gene transcript, expression product, or fragment thereof, relative to the level of expression of the at least one stably expressed gene, gene transcript, expression product, or fragment thereof, identifies the likelihood of implantation success of the blastocyst.

In some embodiments, comparing the level of expression of the at least one gene, gene transcript, expression product, or fragment thereof in the test sample, selected from the genes and transcripts listed in Table A, Table B, Table C, and/or Table D, with the level of expression of the at least one stably expressed gene, gene transcript, expression product, or fragment thereof in the same sample, may comprise directly comparing the gene expression levels. Such methods may comprise comparing, for example, the number of transcripts, the quantity of the fluorescent dye(s) during PCR amplification including for example, the detected number of PCR cycles, such as Ct or Cp, as reported by the detection method used, for example qRT-PCR. In this regard, comparing the normalised level of expression of the at least one gene, gene transcript, expression product, or fragment thereof in the test sample with the level of expression of the at least one stably expressed gene, gene transcript, expression product, or fragment thereof in the same sample could be reported for example as normalised ΔCt value (Livak and Schmittgen, 2001).

In some embodiments, comparing the level of expression of the at least one biomarker gene, gene transcript, expression product, or fragment thereof, in the test sample, with the level of expression of at least one stably expressed gene, gene transcript, expression product, or fragment thereof comprises comparing the normalised gene expression levels.

In some embodiments, the method comprises normalising the levels of expression of the at least one gene, gene transcript, expression product, or fragment thereof with the level of expression of the at least one stably expressed gene, gene transcript, expression product, or fragment in the test sample. In some embodiments, such normalised levels of expression may be compared, for example, with the same method applied to an YMA/rba-YMA and/or AMA/rba-AMA reference sample and/or the corresponding datasets reported in the current invention, allowing the determination of an increased or decreased likelihood of implantation success.

All references to normalising the level of gene expression, and methods for normalising are as defined in relation to the method of the second aspect, and indeed, such references may be applied throughout this disclosure.

In some embodiments, the method comprises comparing the difference in the level of expression of the at least one biomarker gene, gene transcript, expression product, or fragment thereof in the test sample versus a reference level of expression, with the difference in the level of expression of at least one stably expressed gene, gene transcript, expression product, or fragment thereof in the test sample versus a reference level of expression.

Thus, in some embodiments, the method comprises:
determining, in a test sample obtained from the blastocyst, the level of expression of at least one biomarker gene or transcript selected from the genes and transcripts listed in Table A, Table B, Table C, and/or Table D, or an expression product or fragment thereof;
determining, the level of expression of the at least one biomarker gene or transcript, or expression product or fragment thereof in a reference sample;
determining the relative level of expression of the at least one biomarker gene or transcript, or expression product or fragment thereof in the test sample versus the reference sample;
determining, in the test sample, the level of expression of at least one stably expressed gene, gene transcript, expression product, or fragment thereof;
determining, in the reference sample, the level of expression of the at least one stably expressed gene, gene transcript, expression product, or fragment thereof;
determining the relative level of expression of the at least one stably expressed gene, gene transcript, expression product, or fragment thereof in the test sample versus the reference sample;
comparing the relative level of expression of the at least one biomarker gene, gene transcript, expression product, or fragment thereof with the relative level of expression of the at least one stably expressed gene, gene transcript, expression product, or fragment thereof;
wherein a difference in the relative level of expression of the at least one biomarker gene, gene transcript, expression product, or fragment thereof, versus the relative level of expression of the at least one stably expressed gene, gene transcript, expression product, or fragment thereof, identifies the likelihood of implantation success of the blastocyst.

The reference level of expression may be the level of the expression in a reference sample. The reference sample may be a reference sample as defined in relation to the method of the second aspect, and indeed, such reference samples may be used in any of the methods disclosed throughout this disclosure.

For example, in some embodiments the reference sample may be representative of a YMA/rba-YMA or AMA/rba-AMA sample. In some embodiments, the reference sample may be representative of the datasets provided herein.

Thus, in some embodiments, the method comprises comparing the relative levels of expression of the at least one biomarker gene, gene transcript, expression product, or fragment thereof, in the test sample versus a reference sample, with the relative level of expression of at least one stably expressed gene, gene transcript, expression product, or fragment thereof in the test sample versus a reference sample.

The choice of the at least one gene selected from the genes listed in Table A, Table B, Table C, and/or Table D, or an expression product or fragment thereof in relation to the method of the third aspect is as defined in relation to the use of the first aspect, and/or in relation to the method of the second aspect.

In related methods, the level of expression of the at least one biomarker gene, gene transcript, expression product, or fragment thereof, relative to the level of expression of the at least one stably expressed gene, gene transcript, expression product, or fragment thereof, in relation to the gene expression levels and/or the overall expression levels of the datasets reported in the current study, identifies the likelihood of implantation success of the blastocyst.

In some embodiments, a method of the third aspect as set out above may comprise the use of any gene or gene transcript, or combination of genes, gene transcripts, expression products, or fragments thereof listed in Table A and/or Table C, as defined in accordance with the first aspect above, as a biomarker to identify a blastocyst as having an increased likelihood of implantation success. For example, the method may comprise the use as defined in accordance with the first aspect as a biomarker to identify a blastocyst as having a young reproductive chronological (YMA) and/or biological maternal age (rba-YMA), and thereby as having an increased likelihood of implantation success. Thus, in a method of the third aspect as set out above, an increased level of expression of at least one biomarker gene, gene transcript, expression product, or fragment thereof listed in Table A and/or Table C, relative to the level of expression of the at least one stably expressed gene, gene transcript, expression product, or fragment thereof, identifies the blastocyst as having an increased likelihood of implantation success.

In other embodiments, a method of the third aspect as set out above may comprise the use of any gene or gene transcript, or combination of genes, gene transcripts, expression products, or fragments thereof listed in Table B and/or Table D, as defined in accordance with the first aspect above, as a biomarker to identify a blastocyst as having a reduced likelihood of implantation success. For example, the method may comprise the use as defined in accordance with the first aspect as a biomarker to identify a blastocyst as having an advanced reproductive chronological (AMA) and/or biological age (rba-AMA), and thereby as having a reduced likelihood of implantation success. Thus, in a method of the third aspect as set out above, an increased level of expression of at least one biomarker gene, gene transcript, expression product, or fragment thereof listed in Table B and/or Table D, relative to the level of expression of the at least one stably expressed gene, gene transcript, expression product, or fragment thereof, identifies the blastocyst as having a reduced likelihood of implantation success.

Deviation in the level of expression between the level of expression of the biomarker gene/transcript and the level of expression of the stably expressed gene/transcript may be used to determine the likelihood of implantation success. For example, a relative increase in level of expression of the at least one biomarker gene, gene transcript, expression product, or fragment thereof, in the test sample against the level of expression of the at least one stably expressed gene, gene transcript, expression product, or fragment thereof, may identify the at least one gene, gene transcript, expression product, or fragment thereof as having an increased level of expression. For example, YMA/rba-YMA and/or AMA/rba-AMA and/or the datasets reported in the current invention can be used as references.

In some embodiments, the stably expressed gene may be a housekeeping gene. Any suitable housekeeping gene may be used.

In some embodiments, a plurality of stably expressed genes, gene transcripts, expression products, or fragments thereof, may be used. In such embodiments, the mean level of expression of the stably expressed genes, gene transcripts, expression products, or fragments thereof, may be determined and used for comparison to the level of expression of the at least one biomarker gene, gene transcript, expression product, or fragment thereof, to identify the likelihood of implantation success of the blastocyst.

In some embodiments, the stably expressed gene or genes that may be used include any one or combination of the genes and gene transcripts listed in Table F. The genes and gene transcripts in Table F have been found to be expressed at substantially the same level in both the YMA/rba-YMA and AMA/rba-AMA groups (coefficient of variation (CV) <20%). Housekeeping genes that are known in the art may also be used, such as for example, including *H₃F₃B, HNRNPC, ACTB* and *GAPDH.*

Throughout this disclosure, any suitable method for determining the level of expression of the one or more genes or gene transcripts may be used. For example, to measure the level of gene expression of one or more genes or gene transcripts, cDNA may be generated from poly(A) RNA extracted from a sample such as a trophoblast cell taken from a blastocyst of interest, and primers designed to amplify test sequences using a quantitative form of Polymerase Chain Reaction, such as, for example, qRT-PCR. Suitable methods are described below and will be known to the skilled person. Suitable methods may include absolute quantification methods.

The second and third aspects relate to methods of identifying the likelihood of implantation success of a blastocyst for potential embryo transfer. Having identified a blastocyst as having an increased or decreased likelihood of implantation success, therefore, the skilled person may subsequently use the blastocyst, or avoid using the blastocyst, in a uterine transfer procedure.

Therefore, according to a fourth aspect, there is provided a method of embryo transfer of a blastocyst, the method comprising:
determining, in a test sample obtained from the blastocyst, the level of expression of at least one gene or gene transcript selected from the genes and gene transcripts listed in Table A, Table B, Table C, and/or Table D, or an expression product or fragment thereof;
comparing the level of expression of the at least one gene, gene transcript, expression product, or fragment thereof, in the test sample, with the level of expression of the at least one gene, gene transcript, expression product or fragment thereof, in a reference sample;
wherein the level of expression of the at least one gene, gene transcript, expression product, or fragment thereof, in the test sample relative to the level of expression in the reference sample identifies the likelihood of implantation success of the blastocyst, and
using a blastocyst identified as having an increased likelihood of implantation success, or as not having a reduced likelihood of implantation success, in a uterine transfer procedure.

In some embodiments, the method is a method of embryo transfer of a blastocyst identified as having an increased likelihood of implantation success, and the method comprises:
determining, in a test sample obtained from the blastocyst, the level of expression of at least one gene or gene transcript selected from the genes and transcripts listed in Table A and/or Table C, or an expression product or fragment thereof;
comparing the level of expression of the at least one gene, gene transcript, expression product, or fragment thereof, in the test sample, with the level of expression of the corresponding at least one gene, gene transcript, expression product, or fragment thereof, in a reference sample;
wherein the level of expression of the at least one gene, gene transcript, expression product, or fragment thereof, in the test sample relative to the level of expression in the reference sample identifies the blastocyst as having an increased likelihood of implantation success, and
using a blastocyst identified as having an increased likelihood of implantation success in a uterine transfer procedure.

In some embodiments, the method is a method of embryo transfer of a blastocyst identified as not having a reduced likelihood of implantation success, and the method comprises:
determining, in a test sample obtained from the blastocyst, the level of expression of at least one gene or gene transcript selected from the genes and transcripts listed in Table B and/or Table D, or an expression product or fragment thereof;
comparing the level of expression of the at least one gene, gene transcript, expression product, or fragment thereof, in the test sample, with the level of expression of the corresponding at least one gene, gene transcript, expression product, or fragment thereof, in a reference sample;
wherein the level of expression of the at least one gene, gene transcript, expression product, or fragment thereof, in the test sample relative to the level of expression in the reference sample identifies the blastocyst as having a reduced likelihood of implantation success, and
using a blastocyst that is identified as not having a reduced likelihood of implantation success in a uterine transfer procedure.

The choice of the at least one gene or gene transcript selected from the genes and gene transcripts listed in Table A, Table B, Table C, and/or Table D, or an expression product or fragment thereof in relation to the method of the fourth aspect is as defined in relation to the use of the first aspect.

The method of the fourth aspect corresponds to the method of the second aspect but additionally includes using a blastocyst that is identified as having an increased likelihood of implantation success, or that is identified as not having a reduced likelihood of implantation success, in a uterine transfer procedure. Thus, methods of the fourth aspect are as defined in relation to the method of the second aspect but additionally include the use of the blastocyst in a uterine transfer procedure.

Thus, in some embodiments, a method of the fourth aspect as set out above may comprise the use of any gene or gene transcript, or combination of genes or gene transcripts, expression products, or fragments thereof listed in Table A and/or Table C, as defined in accordance with the first aspect above, as a biomarker to identify a blastocyst as having an increased likelihood of implantation success, and then using the blastocyst in a uterine transfer procedure. For example, the method may comprise the use as defined in accordance with the first aspect as a biomarker to identify a blastocyst as having a young reproductive chronological (YMA) and/or young biological maternal age (rba-YMA), and thereby as having an increased likelihood of implantation success, and then using the blastocyst in a uterine transfer procedure.

Thus, in some embodiments, a method of the fourth aspect as set out above may comprise the use of any gene or gene transcript, or combination of genes or gene transcripts, expression products, or fragments thereof listed in Table B and/or Table D, as defined in accordance with the first aspect above, as a biomarker to identify a blastocyst as having a reduced likelihood of implantation success, and then using a blastocyst that has not been identified as having a reduced likelihood of implantation success in a uterine transfer procedure. For example, the method may comprise the use as defined in accordance with the first aspect as a biomarker to identify a blastocyst as having an advanced reproductive chronological (AMA) and/or advanced biological maternal age (rba-AMA), and thereby as having a reduced likelihood of implantation success, and then using a blastocyst that has not been identified as AMA/rba-AMA in a uterine transfer procedure.

Thus, the method of the fourth aspect may correspond to any of the methods described above in relation to the second aspect, and further comprises the use of a blastocyst identified as having an increased likelihood of implantation success, or as having an increased likelihood of implantation success, in a uterine transfer procedure.

Thus, the method of the fourth aspect may correspond to any of the methods described above in relation to the third aspect, and further comprises the use of a blastocyst identified as having an increased likelihood of implantation success, or as not having a reduced likelihood of implantation success, in a uterine transfer procedure.

Throughout this disclosure, references to using a blastocyst in a uterine transfer procedure refer to the process of implanting a blastocyst into the uterus.

According to a fifth aspect, there is provided a method of embryo transfer of a blastocyst, the method comprising:
determining, in a test sample obtained from the blastocyst, the level of expression of at least one biomarker gene or gene transcript selected from the genes and gene transcripts listed in Table A, Table B, Table C, and/or Table D, or an expression product or fragment thereof;
determining, in the test sample, the level of expression of at least one stably expressed gene, gene transcript, expression product, or fragment thereof;
comparing the level of expression of the at least one biomarker gene, gene transcript, expression product, or fragment thereof, in the test sample, with the level of expression of the at least one stably expressed gene, gene transcript, expression product, or fragment thereof;
wherein the level of expression of the at least one biomarker gene, gene transcript, expression product, or fragment thereof, relative to the level of expression of the at least one stably expressed gene, gene transcript, expression product, or fragment thereof, identifies the likelihood of implantation success of the blastocyst, and
using a blastocyst identified as having an increased likelihood of implantation success, or as not having a reduced likelihood of implantation success, in a uterine transfer procedure.

The method of the fifth aspect corresponds to the method of the third aspect but additionally includes using a blastocyst that is identified as having an increased likelihood of implantation success, or that is identified as not having a reduced likelihood of implantation success, in a uterine transfer procedure.

Thus, methods of the fifth aspect are as defined in relation to the method of the third aspect but additionally include the use of the blastocyst in a uterine transfer procedure.

The choice of the at least one gene or gene transcript selected from the genes and gene transcripts listed in Table A, Table B, Table C, and/or Table D, or an expression product or fragment thereof in relation to the method of the fifth aspect is as defined in relation to the use of the first aspect, or as defined in relation to the method of any of the second, third and/or fourth aspects.

The at least one stably expressed gene, gene transcript, expression product, or fragment thereof that may be used in the methods of the second, third, fourth and fifth aspects include any one or combination of the genes and gene transcripts listed in Table F. The genes and gene transcripts in Table F have been found to be expressed at substantially the same level in both the YMA/rba-YMA and AMA/rba-AMA groups (coefficient of variation (CV) <20%). Other housekeeping genes that are known in the art may additionally or alternatively be used, such as for example, including *H₃F₃B, GAPDH, ACTB* and/or *HNRNPC.*

Thus, in some embodiments, a method of the fifth aspect as set out above may comprise the use of any gene or gene transcript, or combination of genes, gene transcripts, expression products, or fragments thereof listed in Table A and/or Table C, as defined in accordance with the first aspect above, as a biomarker to identify a blastocyst as having an increased likelihood of implantation success, and then using the blastocyst in a uterine transfer procedure. For example, the method may comprise the use as defined in accordance with the first aspect as a biomarker to identify a blastocyst as having a young reproductive chronological (YMA) and/or young biological maternal age (rba-YMA), and thereby as having an increased likelihood of implantation success, and then using the blastocyst in a uterine transfer procedure.

In other embodiments, a method of the fifth aspect as set out above may comprise the use of any gene or gene transcript, or combination of genes or gene transcripts, expression products, or fragments thereof listed in Table B and/or Table D, as defined in accordance with the first aspect above, as a biomarker to identify a blastocyst as having a reduced likelihood of implantation success, and then using a blastocyst that has not been identified as having a reduced likelihood of implantation success in a uterine transfer procedure. For example, the method may comprise the use as defined in accordance with the first aspect as a biomarker to identify a blastocyst as having an advanced reproductive chronological (AMA) and/or advanced biological age (rba-AMA), and thereby as having a reduced likelihood of implantation success, and then using a blastocyst that has not been identified as AMA/rba-AMA in a uterine transfer procedure.

Thus, the method of the fifth aspect may correspond to any of the methods described above in relation to the third aspect, and further comprises the use of a blastocyst identified as having an increased likelihood of implantation success, or as not having a reduced likelihood of implantation success, in a uterine transfer procedure.

In a sixth aspect, there is provided a method of identifying the likelihood of implantation success of a blastocyst for potential embryo transfer, the method comprising:
determining, in a test sample obtained from the blastocyst, the gene expression profile of a plurality of genes and/or gene transcripts selected from the genes and gene transcripts listed in Table A, Table B, Table C, and/or Table D;
comparing the gene expression profile of the plurality of genes and/or gene transcripts in the test sample, with the gene expression profile of the plurality of genes and/or gene transcripts in a reference sample;
wherein a correlation in the gene expression profiles, or the absence of a correlation, identifies the likelihood of implantation success of the blastocyst.

Normalisation of the gene expression levels may be applied. The choice of normalisation method, for example, based on housekeeping genes such as the genes and/or gene transcripts listed in Table F, is as defined in relation to the use of the first aspect and/or the methods of any of the second, third, fourth, and/or fifth aspects.

Gene expression profile data relating to the disclosed genes and gene transcripts may be found at the Gene Expression Omnibus (GEO; https://www.ncbi.nlm.nih.gov/geo/) under the accession number GSE133592.

Any suitable method of statistical analysis may be used to identify a correlation. Suitable methods include, for example, Spearman's correlation.

In some embodiments, comparing the gene expression profile of the plurality of genes and/or gene transcripts in the test sample, with the gene expression profile of the plurality of genes and/or gene transcripts in a reference sample and/or the YMA/rba-YMA and/or AMA/rba-AMA dataset profiles reported herein, may comprise the use of the disclosed gene expression levels, optionally normalised as described herein (edgeR), using for example the CPM or RPKM/FPKM or TPM metric, or the raw counts normalised against the not normalised (by edgeR) gene expression levels of the disclosed stably expressed genes.

The choice of the reference sample or reference samples in relation to the method of the sixth aspect is as defined in relation to the method of any of the second, third, fourth, and/or fifth aspects.

For example, in some embodiments, the reference sample may be representative of a blastocyst having a young reproductive chronological (YMA) and/or biological age (rba-YMA). Thus, a correlation between the gene expression profile of the test sample and the gene expression profile of the reference sample identifies the blastocyst as having a high or increased likelihood of implantation success. Conversely, the absence of a correlation identifies the blastocyst as having a low or reduced likelihood of implantation success.

In some embodiments, the reference sample may be representative of a blastocyst having an advanced reproductive chronological (AMA) and/or biological age (rba-AMA). Thus, a correlation between the gene expression profile of the test sample and the gene expression profile of the reference sample identifies the blastocyst as having a low or reduced likelihood of implantation success. Conversely, the absence of a correlation identifies the blastocyst as having a high or increased likelihood of implantation success.

In some embodiments, the method may comprise the use of two or more reference samples, including at least a first reference sample that is representative of a blastocyst having a young reproductive chronological (YMA) and/or biological age (rba-YMA) and a second reference sample that is representative of a blastocyst having an advanced reproductive chronological (AMA) and/or biological age (rba-AMA). Thus, a correlation between the gene expression profile of the test sample with the gene expression profile of the first or second reference samples, and a significant difference with the gene expression profile of the other reference sample, identifies the test sample as being in the YMA/rba-YMA or AMA/rba-AMA group with which the level of expression is correlated.

In some embodiments, the method may comprise a first reference sample that is representative of a blastocyst having a young reproductive chronological (YMA) and/or biological age (rba-YMA) and a second reference sample that is representative of a blastocyst having an advanced reproductive chronological (AMA) and/or biological age (rba-AMA);
wherein a closer correlation between the gene expression profile of the test sample with the gene expression profile of the first reference sample than with the gene expression profile of the second reference sample identifies the blastocyst as having a high or increased likelihood of implantation success; and
wherein a closer correlation between the gene expression profile of the test sample with the gene expression profile of the second reference sample than with the gene expression profile of the first reference sample identifies the blastocyst as having a low or reduced likelihood of implantation success.

In other embodiments, the method comprises an absolute quantification method. For example, the method may comprise the absolute quantification of the number of copies and/or molecules of each biomarker. Such methods may comprise, for example, using known concentrations of this biomarker, by using serially diluted standards to generate a standard curve (absolute quantification). This may be appropriate when a known number of blastocyst cell(s) is/are used. This absolute quantification approach can be used in comparison with known absolute quantification values, determined using YMA/rba-YMA and/or AMA/rba-AMA and/reference samples. Correlation of the absolute quantification between test and reference samples, may be employed to identify increased or decreased levels of expression to thereby identify the likelihood of implantation success.

According to a seventh aspect, there is provided a method of identifying a blastocyst having the greatest likelihood of implantation success for potential embryo transfer from a plurality of blastocysts from the same mother, the method comprising:
determining, in a first test sample obtained from a first blastocyst, the level of expression of at least one biomarker gene or transcript selected from the genes and transcripts listed in Table A, Table B, Table C, and/or Table D, or an expression product or fragment thereof;
determining, in a second test sample obtained from a second blastocyst, from the same mother as the first blastocyst, the level of expression of the at least one biomarker gene, gene transcript, expression product or fragment thereof;
comparing the level of expression of the at least one biomarker gene, gene transcript, expression product, or fragment thereof, in the first test sample, with the level of expression of the at least one biomarker gene, gene transcript, expression product, or fragment thereof, in the second test sample;
wherein the level of expression of the at least one biomarker gene, gene transcript, expression product, or fragment thereof in the first test sample, relative to the level of expression of the at least one biomarker gene, gene transcript, expression product, or fragment thereof in the second test sample, identifies the blastocyst having the greatest likelihood of implantation success.

In some embodiments, there is provided a method of identifying a blastocyst having the greatest likelihood of implantation success for potential embryo transfer from a plurality of blastocysts from the same mother, the method comprising:
determining, in a first test sample obtained from a first blastocyst, the level of expression of at least one biomarker gene or transcript selected from the genes and transcripts listed in Table A, Table B, Table C, and/or Table D, or an expression product or fragment thereof;
determining, in a nth test sample obtained from a nth blastocyst, from the same mother as the first blastocyst, the level of expression of the at least one biomarker gene, gene transcript, expression product or fragment thereof;
comparing the level of expression of the at least one biomarker gene, gene transcript, expression product, or fragment thereof, in the first test sample, with the level of expression of the at least one biomarker gene, gene transcript, expression product, or fragment thereof, in the nth test sample;
wherein the level of expression of the at least one biomarker gene, gene transcript, expression product, or fragment thereof in the first test sample, relative to the level of expression of the at least one biomarker gene, gene transcript, expression product, or fragment thereof in the nth test sample, identifies the blastocyst having the greatest likelihood of implantation success.

In some embodiments, the same process may be repeated until all blastocysts are compared, or part of them, for example, until 1 or 2 competent blastocysts are determined with high likelihood of implantation success.

As nth blastocyst is considered each blastocyst out of the total number of blastocysts produced by a woman, determined by an ordinal number. Any suitable number of blastocysts may be tested, for example, up to 10, 12, 15, 18, 20 or more blastocysts may be tested. In some embodiments, 2, 3, 4, 5, 6, 7, 8, 9, 10, 11, 12, 13, 14, 15 or more blastocysts may be tested.

Comparison of the level of expression of the at least one biomarker gene, gene transcript, expression product or fragment thereof, of the blastocysts derived from the same woman may take place among all the blastocysts tested or part of them, for example, until 1 or 2 implantation competent blastocysts are determined, and/or until the blastocyst, or 2, 3, or 4 blastocysts, having the greatest likelihood of implantation success is/are identified.

According to an eighth aspect, there is provided a method of embryo transfer of a blastocyst, the method comprising:
determining, in a first test sample obtained from a first blastocyst, the level of expression of at least one biomarker gene or transcript selected from the genes and transcripts listed in Table A, Table B, Table C, and/or Table D, or an expression product or fragment thereof;
determining, in a second test sample obtained from a second blastocyst from the same mother as the first blastocyst, the level of expression of the at least one biomarker gene, gene transcript, expression product or fragment thereof;
comparing the level of expression of the at least one biomarker gene, gene transcript, expression product, or fragment thereof, in the first test sample, with the level of expression of the at least one biomarker gene, gene transcript, expression product, or fragment thereof, in the second test sample;
wherein the level of expression of the at least one biomarker gene, gene transcript, expression product, or fragment thereof in the first test sample, relative to the level of expression of the at least one biomarker gene, gene transcript, expression product, or fragment thereof in the second test sample, identifies the blastocyst having the greatest likelihood of implantation success; and
using the blastocyst identified as having the greatest likelihood of implantation success in a uterine transfer procedure.

In some embodiments, there is provided a method of embryo transfer of a blastocyst, the method comprising:
determining, in a first test sample obtained from a first blastocyst, the level of expression of at least one biomarker gene or transcript selected from the genes and transcripts listed in Table A, Table B, Table C, and/or Table D, or an expression product or fragment thereof;
determining, in a nth test sample obtained from a nth blastocyst from the same mother as the first blastocyst, the level of expression of the at least one biomarker gene, gene transcript, expression product or fragment thereof;
comparing the level of expression of the at least one biomarker gene, gene transcript, expression product, or fragment thereof, in the first test sample, with the level of expression of the at least one biomarker gene, gene transcript, expression product, or fragment thereof, in the nth test sample;
wherein the level of expression of the at least one biomarker gene, gene transcript, expression product, or fragment thereof in the first test sample, relative to the level of expression of the at least one biomarker gene, gene transcript, expression product, or fragment thereof in the nth test sample, identifies the blastocyst having the greatest likelihood of implantation success;
the same process may be repeated until all blastocysts are compared, or part of them, for example, until 1 or 2 competent blastocysts are determined with high likelihood of implantation success, and
using the blastocyst identified as having the greatest likelihood of implantation success in a uterine transfer procedure.

As nth blastocyst is considered each blastocyst out of the total number of blastocysts produced by a woman. Any suitable number of blastocysts may be tested, for example, up to 10, 12, 15, 18, 20 or more blastocysts may be tested. In some embodiments, 2, 3, 4, 5, 6, 7, 8, 9, 10, 11, 12, 13, 14, 15 or more blastocysts may be tested.

Comparison of the level of expression of the at least one biomarker gene, gene transcript, expression product or fragment thereof, of the blastocysts derived from the same woman may take place among all the blastocysts tested or part of them, for example, until 1 or 2 implantation competent blastocysts are determined, and/or until the blastocyst, or 2, 3, or 4 blastocysts, having the greatest likelihood of implantation success is/are identified.

The methods of the seventh and eighth aspects correspond in all of the relevant respects to the methods of the second, third, fourth, fifth, and sixth aspects.

In particular, the choice of the at least one gene or gene transcript selected from the genes and gene transcripts listed in Table A, Table B, Table C, and/or Table D, or an expression product or fragment thereof in relation to the methods of the seventh and eighth aspects is as defined in relation to the use of the first aspect, or as defined in relation to the method of any of the second, third, fourth, fifth, and/or sixth aspects.

In addition, the choice of the reference sample or reference samples in relation to the methods of the seventh or eighth aspects is as defined in relation to the method of any of the second, third, fourth, fifth, and/or sixth aspects.

According to a ninth aspect, there is provided a method of identifying the likelihood of implantation success of a blastocyst for potential embryo transfer, the method comprising:
determining, in a test sample obtained from the blastocyst, the level of expression of at least one biomarker gene or transcript selected from the genes and transcripts listed in Table A, Table B, Table C, and/or Table D, or an expression product or fragment thereof, wherein determining the level of expression comprises determining the number of molecules present in the sample;
comparing the level of expression of the at least one biomarker gene, gene transcript, expression product, or fragment thereof, in the test sample, with a reference;
wherein the level of expression of the at least one biomarker gene, gene transcript, expression product, or fragment thereof, relative to the reference identifies the likelihood of implantation success of the blastocyst.

In some embodiments, the method comprises comparing the level of expression of the at least one biomarker gene, gene transcript, expression product, or fragment thereof, in the test sample with the levels of known concentrations of the biomarker. For example, the method may comprise the use of serially diluted standards to generate a standard curve (absolute quantification), allowing the detection of the exact number of molecules of the investigated biomarker (no housekeeping genes). The level of absolute quantification of the at least one biomarker gene, gene transcript, expression product, or fragment thereof, identifies the likelihood of implantation success of the blastocyst. Such methods may be particularly suitable when a known number of blastocyst cells are used in the test sample.

In other embodiments, an absolute quantification approach may be used in comparison with known absolute quantification values, determined by YMA/rba-YMA and/or AMA/rba-AMA reference samples and/or the corresponding datasets of the current invention, identifying the blastocyst having an increased likelihood of implantation success.

According to a tenth aspect, there is provided a method of embryo transfer of a blastocyst, the method comprising:
determining, in a test sample obtained from the blastocyst, the level of expression of at least one biomarker gene or transcript selected from the genes and transcripts listed in Table A, Table B, Table C, and/or Table D, or an expression product or fragment thereof, wherein determining the level of expression comprises determining the number of molecules present in the sample;
comparing the level of expression of the at least one biomarker gene, gene transcript, expression product, or fragment thereof, in the test sample, with a reference;
wherein the level of expression of the at least one biomarker gene, gene transcript, expression product, or fragment thereof, relative to the reference identifies the likelihood of implantation success of the blastocyst; and
using the blastocyst identified as having the greatest likelihood of implantation success in a uterine transfer procedure.

The methods of the ninth and tenth aspects correspond in all of the relevant respects to the methods of the second, third, fourth, fifth, sixth, seventh, and/or eighth aspects.

In particular, the choice of the at least one gene or gene transcript selected from the genes and gene transcripts listed in Table A, Table B, Table C, and/or Table D, or an expression product or fragment thereof in relation to the methods of the ninth and tenth aspects is as defined in relation to the use of the first aspect, or as defined in relation to the method of any of the second, third, fourth, fifth, sixth, seventh, and/or eighth aspects.

In addition, the choice of the reference sample or reference samples in relation to the methods of the ninth or tenth aspects is as defined in relation to the method of any of the second, third, fourth, fifth, sixth, seventh, and/or eighth aspects.

In an eleventh aspect, there is provided an algorithm that identifies blastocysts having a high or increased likelihood of implantation success and/or a low or reduced likelihood of implantation success.

In some embodiments, the algorithm may incorporate information relating to:
(i) the expression levels of one or more biomarkers of the YMA/rba-YMA and/or AMA/rba-AMA groups;
(ii) the expression of housekeeping (stably expressed) genes;
(iii) known control samples; and/or datasets of known gene expression levels based on the maternal age and/or the implantation potential, as described herein.

Considering also the implantation and/or pregnancy outcomes, artificial intelligence can be employed, allowing the determination of biomarkers with potential greater importance in prioritising blastocysts, associating all previous parameters with the implantation potential. These approaches can ultimately be used to accurately estimate the implantation success potential of blastocysts of known gene expression profiles, while following a learning process, such as deep learning (machine learning), to gradually train the algorithm(s) used to more accurately prioritise the blastocysts with higher likelihood to implant into the uterus, based on the gene expression profiles. The datasets of the current invention could be considered as part of the training process.

In a further aspect, any one or combination of the molecular biomarkers reported herein, such as for example the biomarkers listed in Table A or Table C, as defined above in relation to the use of the first aspect, can be employed to enrich the environment where the blastocysts grow or lie, including for example the culture media and/or embryo transfer media prior to or upon embryo transfer. These factors could be used separately or in groups at the RNA and/or protein level, either as whole peptides and/or fragments, or instead they can be used in the context of an exosome-related methodology that relies on the findings of the current invention.

In some embodiments, the YMA/rba-YMA gene expression profiles reported herein, illustrate significantly higher expressed extracellular exosome-related genes/transcripts compared with the AMA/rba-AMA samples, such as those described in Table E1. This suggests that the presence of YMA/rba-YMA exosome-related biomarkers and/or exosomes lie in the blastocysts with a higher likelihood to implant into the uterus, such as the YMA/rba-YMA. For example, as reported in the current invention, extracellular vesicles (exosomes) of samples classified as YMA/rba-YMA or other YMA/rba-YMA-like samples, could be used to predict enhanced implantation success of the tested blastocysts. In this regard, the use of these biomarkers in the context of naturally- or artificially- derived extracellular exosomes/vesicles (as part of the blastocyst's secretome), could enhance the likelihood of implantation success especially in the blastocysts of AMA/rba-AMA profiles. According to the findings of the current invention, this can be beneficial for women of advanced chronological and/or biological maternal age and so it can be used in women over 40 years and over 30 years, respectively. Although perhaps 35 years could be used more appropriately as the minimum age of women that this approach is more beneficial (compromised fertility), without prior knowledge of the expression profile per blastocyst (i.e. YMA/rba-YMA or AMA/rba-AMA).

Thus, in a twelfth aspect, there is provided at least one gene or gene transcript or fragment thereof selected from those listed in Table A and/or Table C, or an expression product or fragment thereof, for use as a medicament. In some embodiments, there is provided a composition comprising at least 35, such as at least 40, 45, 50, or all, genes selected from those listed in Table A and/or Table C, or expression products or fragments thereof, for use as a medicament.

In a thirteenth aspect, there is provided at least one gene or gene transcript, or fragment thereof selected from those listed in Table A and/or Table C, or an expression product or fragment thereof, for use in enriching a blastocyst. In some embodiments, there is provided a composition comprising at least 35, such as at least 40, 45, 50, or all, genes selected from those listed in Table A and/or Table C, or expression products or fragments thereof, for use in enriching a blastocyst.

Throughout this disclosure, references to "enriching a blastocyst" refer to the administration of at least one gene or gene transcript or expression product or fragment thereof to a blastocyst prior to the use, or upon use, of the blastocyst in an embryo transfer procedure. For example, in some embodiments, the at least one gene or gene transcript or expression product or fragment thereof may be added in an appropriate amount to culture media and/or embryo transfer media containing the blastocyst.

The twelfth and/or thirteenth aspects preferably comprise in *vitro,* in *vivo* or *ex vivo* use.

The choice of the at least one gene or gene transcript or expression product or fragment thereof in relation to the twelfth and/or thirteenth aspect is as defined in relation to the use of the first aspect, or as defined in relation to a method of any of the second, third, fourth, fifth, sixth, seventh, eighth, ninth, and/or tenth aspects.

In some embodiments, the twelfth and/or thirteenth aspect may be in relation to a blastocyst identified as having a low or reduced likelihood of implantation success, for example using a method of any of the second, third, sixth, seventh, ninth, and/or eleventh aspects.

The inventors have developed a kit which is useful for identifying the likelihood of implantation success of a blastocyst.

Accordingly, in a fourteenth aspect, there is provided a kit for identifying the likelihood of implantation success of a blastocyst, the kit comprising:
a reference sample that is known to be representative of a blastocyst having a young reproductive chronological (YMA) and/or young biological age (rba-YMA) or an advanced reproductive chronological (AMA) and/or advanced biological age (rba-AMA) and/or an intermediate sample of average gene expression levels; and
means for determining the level of expression of at least one gene or gene transcript selected from those listed in Table A, Table B, Table C and/or Table D or an expression product or fragment thereof.

Intermediate is a sample that is representative of the average level of gene expression between the levels of expression in blastocysts representative of YMA/rba-YMA and AMA/AMA-rba. In some embodiments, the reference sample may correspond to the average (or median) level of expression of the at least one gene, gene transcript, expression product, or fragment thereof, between the YMA/rba-YMA and AMA/rba-AMA groups.

The choice of the reference sample or reference samples in relation to the kit of the fourteenth aspect is as defined in relation to the method of any of the second, third, fourth, fifth, sixth, seventh, eighth, ninth, and/or tenth aspect.

The choice of the housekeeping gene or genes in relation to the kit of the fourteenth aspect is as defined in relation to the method of the third, fourth, fifth, sixth, seventh, eighth, ninth, and/or tenth aspect.

In some embodiments, there is provided a kit for identifying the likelihood of implantation success of a blastocyst, the kit comprising:
at least one reference sample that is known to be representative of a blastocyst having a young reproductive chronological (YMA) and/or young biological maternal age (rba-YMA) or an advanced reproductive chronological (AMA) and/or advanced biological maternal age (rba-AMA) and/or a sample that is representative of an intermediate profile between YMA/rba-YMA and AMA/rba-AMA samples; and
means for determining the level of expression of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A, Table B, Table C, and/or Table D, or expression products or fragments thereof, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A*, Table B*, Table C*, and/or Table D*.

In some embodiments, there is provided a kit for identifying the likelihood of implantation success of a blastocyst, the kit comprising:
a reference sample that is known to be representative of a blastocyst having a young reproductive chronological (YMA) and/or young biological maternal age (rba-YMA) or an advanced reproductive chronological (AMA) and/or advanced biological maternal age (rba-AMA) and/or a sample that is representative of an intermediate between YMA/rba-YMA and AMA/rba-AMA samples; and
means for determining the level of expression of at least 35, such as at least 40, 45, 50, or all, genes or gene transcripts selected from those listed in Table A and/or Table C, or expression products or fragments thereof.

In a fifteenth aspect, there is provided a kit for identifying the likelihood of implantation success of a blastocyst, the kit comprising:
means for determining the level of expression of at least one gene or gene transcript selected from those listed in Table A, Table B, Table C and/or Table D or an expression product or fragment thereof
means for determining the level of expression of at least one stably expressed gene, gene transcript, expression product, or fragment thereof.

The choice of the at least one stably expressed gene, gene transcript, expression product, or fragment thereof in relation to the kit of the fifteenth aspect is as defined in relation to the use of the first aspect and/or method of the second, third, fourth, fifth, sixth, seventh, eighth, ninth, tenth, eleventh, twelfth, and/or fourteenth aspect. Thus, the at least one stably expressed gene, gene transcript, expression product, or fragment thereof that may be used in the kit of the fifteenth aspect includes any one or combination of the genes and gene transcripts listed in Table F. The genes and gene transcripts in Table F have been found to be expressed at substantially similar levels in both the YMA/rba-YMA and AMA/rba-AMA groups (coefficient of variation (CV) <20%). Other housekeeping genes that are known in the art may additionally or alternatively be used, such as for example, including *H3F3B, GAPDH, ACTB* and/or *HNRNPC.*

In some embodiments, there is provided a kit for identifying the likelihood of implantation success of a blastocyst, the kit comprising:
means for determining the level of expression of at least 2, such as at least 3, 4, 5, 6, 7, 8, 9, 10, 12, 15, 18, 20, 25, 30, 35, 40, 45, 50, or all, genes, gene transcripts, expression products, or fragments thereof selected from those listed in Table A, Table B, Table C, and/or Table D, or expression products or fragments thereof, wherein at least one, such as at least 2, 3, 4, 5, 6, 7, 8, 9, 10, 12, 15, 18, 20, 25, 30, 35, 40, 45, 50, or all, of the genes, gene transcripts, expression products, or fragments thereof is selected from those listed in Table A*, Table B*, Table C*, and/or Table D*; and
means for determining the level of expression of at least one stably expressed gene, gene transcript, expression product, or fragment thereof selected from panel F.

The choice of the at least one gene or gene transcript selected from the genes listed in Table A, Table B, Table C, and/or Table D or expression product or fragment thereof in relation to the kits of the fourteenth or fifteenth aspect is as defined in relation to the use of the first aspect. In some embodiments, there is provided the use of at least 2, 3, 4, 5, 6, 7, 8, 9, 10, 12, 15, 18, 20, 25, 30, 35, 40, 45, or 50 genes and/or gene transcripts selected from any of those listed in Table A, Table B, Table C and/or Table D, or expression products or fragments thereof.

In some embodiments, there is provided a kit for identifying the likelihood of implantation success of a blastocyst, the kit comprising:
means for determining the level of expression of at least 35, such as at least 40, 45, 50, or all, genes or gene transcripts selected from those listed in Table A and/or Table C, or expression products or fragments thereof; and
means for determining the level of expression of at least one stably expressed gene, gene transcript, expression product, or fragment thereof selected from panel F.

In some embodiments, the kits of the fourteenth and/or fifteenth aspects may comprise means for absolute quantification of the number of copies and/or molecules of each biomarker. For example, in some embodiments, the kits comprise means for producing a series of standards to generate a standard curve (absolute quantification). Such embodiments may be appropriate when known number of blastocyst cell(s) is/are used. This absolute quantification approach may be used in comparison with known absolute quantification values, determined, for example, by YMA/rba-YMA and/or AMA/rba-AMA reference samples.

The kits of the fourteenth and/or fifteenth aspects may be arranged for use in any of the methods of the second, third, fourth, fifth, sixth, seventh, eighth, ninth, tenth, and/or eleventh aspects.

For example, it will be appreciated that the inventors have determined that an increased level of expression of each of the genes and/or gene transcripts listed in Table A and Table C is statistically significantly associated with an increased likelihood of blastocyst implantation success, and the kits of the fourteenth and/or fifteenth aspects may comprise means for detecting the levels of expression of any one or more of the genes or gene transcripts listed in Table A and/or Table C, or expression product or fragment thereof, as a biomarker for increased likelihood of blastocyst implantation success, using any of the methods disclosed herein.

The inventors have also determined that an increased level of expression of each of the genes and/or gene transcripts listed in Table B and Table D is statistically significantly associated with a reduced likelihood of blastocyst implantation success, and the kits of the fourteenth and/or fifteenth aspects may comprise means for detecting the levels of expression of any one or more of the genes and/or gene transcripts listed in Table B and/or Table D, or an expression product or fragment thereof, as a biomarker for reduced likelihood of blastocyst implantation success, using any of the methods disclosed herein.

The kits of the fourteenth and/or fifteenth aspects may comprise any suitable materials and methods for determining gene and/or protein expression and suitable methods are discussed below.

In some embodiments, the kits comprise PCR methods and suitable primers for use in such methods are described below, for example in Tables 1 and 2.

### Nucleic acid assays

Significantly more highly expressed biomarkers in the YMA/rba-YMA samples (Table A, Table C) are associated with higher embryonic implantation success and/or developmental competency, while AMA/rba-AMA biomarkers (Table B, Table D) suggest significantly reduced embryonic implantation potential and/or decreased developmental competency. These biomarkers can be detected in trophectoderm (TE) cells; fluids such as blastocyst fluid and/or culture media where blastocysts have been incubating and secreting these biomarkers including cell-free nucleic acids, proteins and extracellular vesicles. All sources could be assayed using the biomarkers reported in the current invention and can be analysed using various methodological approaches at the nucleic acid and/or protein level per blastocyst. A blastocyst biopsy is consisted of mainly by trophectoderm cells along with a portion of cell culture media/blastocyst fluids that include part of the molecular biomarkers reported in the current study and especially the biomarkers significantly higher present in the YMA/rba-YMA samples.

Nucleic acids are detected primarily by hybridization, potential amplification and identification. Poly (A) RNA deriving from single human blastocysts, for example, could be employed to detect these biomarkers. Oligo(dT) and/or random oligos (i.e. random hexamers) can be used to hybridize the nucleic acids of interest (biomarkers), although longer oligo sequences of various lengths up to 1-2 kilobases, or more, can also be employed. Hybridization molecules could be designed to generically target any poly(A) and/or other transcripts, or they could be custom designed to specifically target the biomarkers of this invention.

These oligonucleotide sequences may be used as primers for amplification of the nucleic acids/targets, or to form duplex molecules with the nucleic acids based on their complementarity. Their nucleic acid sequences can be derived synthetically by fragment production using chemical means or through amplification of selected sequences using vectors. "Primer" refers to any nucleic acid that could prime the synthesis of another nucleic acid in a template specific manner.

Depending on the application, the selection of nucleic acids may require different levels of hybridization stringency and the experimental conditions could be adapted accordingly. The detection of DAB2 and/or BAK1 biomarkers, for example, may require higher stringency, allowing confidence in detecting these transcripts, specfically, and the same strategy could be applied if all of the YMA/rba-YMA and/or AMA/rba-AMA biomarkers are targeted. In certain occasions, these sequences may be combined with appropriate means, such as a label, that could detect successful hybridization, including among others, enzymatic, radioactive, fluorescent, or other ligands, such as biotin.

These approaches could be used among others in the context of a PCR reaction, including but not limited to SYBR green real-time PCR, TaqMan real-time PCR assay, microfluidics PCR; Northern, Southern blot; RNA fluorescent in situ hybridisation (RNA FISH); cDNA microarray; RNA sequencing and other approaches. The nucleic acids may include genomic DNA or RNA/cDNA. In the context of PCR as an oligonucleotide sequence amplification method, multiple rounds of amplification ("cycles") usually take place to produce sufficient amount of product that can be more easily detected/quantified. In the case of RNA, reverse transcription PCR amplification could be performed to amplify the RNA after conversion into cDNA and again this process is followed by polymerisation. Among other methods, isothermal amplification could be used as an alternative approach, which employs a polymerase with strand displacement activity. RT-PCR and qRT-PCR could be applied in the context of a microfluidics system that could be used to reverse transcribe and amplify the transcripts/biomarkers of interest on a chip.

For example, a simple kit involving primers (oligos) that target the most important YMA/rba-YMA and/or AMA/rba-AMA biomarkers, could be employed to reveal the blastocyst with greater likelihood to implant into the uterus and/or those having a greater developmental potential. As also reported in Tables A-E (below), the primers of the molecular biomarkers shown in the following table (Table 1), belong to YMA/rba-AMA (AA: 1-14) or AMA/rba-AMA (AA: 15-20) groups. These particular primers (Table 1; AA: 1-14), target YMA/rba-AMA biomarkers with overlapping properties. The majority of them are extracellular exosome-related, they can be involved in embryo-endometrial communication, with FABP5, PSMA5, TAGLN2 and PRDX2 being present at the protein level in the blastocyst fluid, illustrating higher confidence that these biomarkers participate in embryo-endometrial communication with higher confidence. The primers targeting the AMA/rba-AMA biomarkers (Table 1; AA: 15-20) are also involved in apoptotic process, illustrating a biological significance while classifying test samples in the AMA/rba-AMA category; hence lower likelihood to implant into the uterus. These primers may be amplified in annealing temperature set at 60°C.

**Table 1.**

| ***AA*** | ***Gene name (Biomarker)*** | ***Primer (Forward)*** | ***Primer (Reverse)*** |
|---|---|---|---|
| 1 | ACAT2 | GCGGACCATCATAGGTTCCTT SEQ ID No. 25 | TGCTGCCAAGACATGTCCAA SEQ ID No. 26 |
| 2 | DAB2 | *Also reported in Table 2* | *Also reported in Table 2* |
| 3 | DBI | *Also reported in Table 2* | *Also reported in Table 2* |
| 4 | FABP3 | *Also reported in Table 2* | *Also reported in Table 2* |
| 5 | FABP5 | CATGAAGGAGCTAGGAGTGGG SEQ ID No. 27 | CCCAGGGTACAAGAAAACTGTG SEQ ID No. 28 |
| 6 | HINT1 | TTGAGGATGACCGGTGCCTT SEQ ID No. 29 | AGCAGCACATTTCTTGCCAAC SEQ ID No. 30 |
| 7 | HSP90AA1 | CGTTTCTGAGAAGCAGGGCA SEQ ID No. 31 | GGGAATGCAGAGACGTGGAA SEQ ID No. 32 |
| 8 | IFITM3 | CGCCTACTCCGTGAAGTCTA SEQ ID No. 33 | CCATAGGCCTGGAAGATCAGC SEQ ID No. 34 |
| 9 | LRP2 | CGTCGCGGAGATGGATCG SEQ ID No. 35 | CGCACTGTCACATTCTTGGC SEQ ID No. 36 |
| 10 | PEBP1 | CACCAGCATTTCGTGGGATG SEQ ID No. 37 | CAGGAAATGATGCCATTCTCTGT SEQ ID No. 38 |
| 11 | PRDX5 | GGATGTTCCAAGGTTCGGCT SEQ ID No. 39 | AGGGCCTTCACTATGCCATC SEQ ID No. 40 |
| 12 | PSMA5 | TTAGTACTGCGGCCGTGTG SEQ ID No. 41 | AGTATTCACGCCCCTGTCGTA SEQ ID No. 42 |
| 13 | TAGLN2 | GCTTGAACGCTCCCCG SEQ ID No. 43 | TCCACTGGATCAGGATCTGC SEQ ID No. 44 |
| 14 | PRDX2 | CGAGCATGGGGAAGTTTGTC SEQ ID No. 45 | GGCACAAGCTCACTATCCGT SEQ ID No. 46 |
| 15 | ATG3 | CCCCAGGATGCAGAATGTGA SEQ ID No. 47 | TCCAGCTGCCACAAACTCTT SEQ ID No. 48 |
| 16 | BAK1 | *Also reported in Table 2* | *Also reported in Table 2* |
| 17 | MCL1 | AACAAAGAGGCTGGGATGGG SEQ ID No. 49 | TGCCAAACCAGCTCCTACTC SEQ ID No. 50 |
| 18 | MTMR14 | CGCGGCTCTAAGGTTGA SEQ ID No. 51 | GATGTGCCGGGGATAGTGG SEQ ID No. 52 |
| 19 | PI4KB | GGGTCCCCCAGAGGATCAA SEQ ID No. 53 | CAAAACTTGCTCCGGCCTTC SEQ ID No. 54 |
| 20 | PPP1R15A | GCTTGAACGCTCCCCGC SEQ ID No. 55 | ATCCTTTCGGCACTGGETG SEQ ID No. 56 |

High throughput approaches, including RNA sequencing and cDNA microarrays could be used to detect, identify and quantify a large number of biomarkers that belong to the YMA/rba-YMA and/or AMA/rba-AMA samples. These approaches could be used on test samples to determine these biomarkers investigating the whole transcriptome or the poly(A) coding and non-coding transcripts. Alternatively, custom panels targeting specifically the biomarkers of the current invention could be developed. Ultrahigh multiplexed PCR approaches could be tailored with RNA sequencing to determine the expression levels of the biomarkers of interest in a single panel that could allow the determination of blastocysts with higher implantation potential associated with the appropriate expression levels of selected biomarkers reported herein.

### Protein assays

The biomarkers of the current invention can be detected at the protein level using various methods, including among others electrochemical approaches, optical methods, gas phase ion spectrometry, atomic force microscopy.

For example, YMA/rba-YMA and AMA/rba-AMA biomarkers could be tested at the protein level using differential expression profiling analyses, such as the surface enhanced laser desorption/ionisation (SELDI) or matrix-assisted laser desorption/ionization (MALDI) methods (i.e. ProteinChip^{®} arrays). In particular, biomolecules/biomarkers can be separated using high performance liquid chromatography (HPLC) followed by gas phase ion spectrometry to detect the biomarkers using for example SELDI or MALDI, with SELDI biochips being able to capture the biomarkers of the current invention.

Another approach would be to use specific antibodies targeting selected biomarkers. These could be employed in the context of a bioassay of polyclonal or monoclonal antibodies that could target the protein(s) in question, detecting a complex of antibodies selectively bound to the biomarkers that could possibly isolate and/or quantify the target protein/antigen. Typically, selective antibody reactions may require for example 10 to 100 times more signal compared with the background/noise or at least twice as much signal. The formation of an antibody-biomarker complex may be detected using a detection reagent, including but not limited to a secondary antibody with a detectable label, such as fluorescent dye(s); enzymes including alkaline phosphatase, horse radish peroxide etc; magnetic beads, such as Dynabeads; and colorimetric labels including colloidal gold. A second labelled antibody could be used in other cases to indirectly target the antibody-biomarker complex, alternatively.

The antibody-biomarker complex can be measured among others by chemiluminescent, luminescent, fluorescent and absorbance approaches; optical methods such as non-confocal or confocal microscopy; radio frequency approaches such as multipolar resonance spectroscopy; electrochemical methods including voltammetry and others. Commonly used assays include Western blot, radioimmune (RIA) and enzyme-linked immunosorbent (ELISA) assays.

Immunoassays can quantify selected biomarkers determining the amount of antibody-biomarker complex, the relative expression of which can be compared to a known compound or protein (standard/reference), instead of being measured in absolute units. In this regard, YMA/rba-YMA and/or AMA/rba-AMA biomarkers can be targeted at the protein level, the expression of which can inform the likelihood of implantation success and developmental competency or implantation and developmental failure, accordingly, using, for example, trophectoderm cells or organelles or homogenised tissue extract and/or different fluids in contact with the blastocyst, such as blastocyst fluid or other blastocyst secreted cell-free molecules found within the embryo culture media, or extracellular exosomes derived from the human blastocyst. These sources could be used individually or combined for the selection of the most competent blastocyst for embryo transfer into the uterus.

Immunoassays include among others, RIA, ELISA, immunocytochemistry, Western blot, chemiluminescent and other assays. Immunobinding assays include the formation of antibody-biomarker complexes or (immunocomplexes). In the current invention, the presence (or absence) of various YMA/rba-YMA and/or AMA/rba-AMA biomarkers or presence/absence of the most crucial ones, may be detected and/or the formed immunocomplexes quantified.

Western blot can be employed for protein detection following protein separation by electrophoresis usually in a polyacrylamide gel, which is then transferred onto, for example, a nylon membrane. The first antibody targeting the antigen is followed, for example, by a second labelled antibody as mentioned before. Apart from the commonly used RIA and ELISA binding immunoassays, other assays include fluorescence-activated cell sorting (FACS), which could be applied, for example, on trophectoderm biopsies, targeting expression profiles related with YMA/rba-YMA or AMA/rba-AMA samples. Immunocytochemistry may be used primarily in the context of detecting the presence or absence of YMA/rba-YMA and/or AMA/rba-AMA biomarkers, which are indicative of blastocyst implantation competency, or they could be tailored to a quantification approach that would measure the signal intensity of targeted biomarkers used as prognostic biomarkers.

Protein microarray technology (or protein chip) is another method for high throughput screening, that could be used to determine the expression levels of the biomarkers reported in the current invention, along with the potential investigation of protein functions, that include binding with different macromolecules, ligand-receptor interactions and enzyme activity. This approach may include protein chip assays such as the ProteinChip approach that is based on SELDI methodology, enabling studies of protein-protein interactions, immunoassays, ligand binding assays etc. Protein-protein interactions could be tested in the context of the current invention if endometrial proteins (enhanced in the receptive endometrium) and embryonic proteins are used to define these interactions. Based on this approach, exosome-related biomarkers such as the ones reported in Table E for example, may be tested for potential protein-protein interactions with receptive endometrial proteins.

Microfluidic chip immunoassays can be used for processing proteomic applications requiring much lower volumes, such as the ones required to detect the biomarkers of the current invention, either, for example, at the cellular or extracellular/exosome level.

Exosomes could be isolated from individual blastocyst fluids and/or culture media where blastocysts have been cultured. Different methods could be applied in this regard, including among others ultracentrifugation, precipitation, chromatography and magnetic beads, targeting specific exosomal proteins. Magnetic beads are highly efficient at concentrating markers from low yield mixtures and small exosome volumes, hence appropriate for this invention, considering the small number of exosomes secreted by the individual blastocysts. The isolated exosomes could then be used for nucleic acid and protein isolation/quantification allowing the detection of the YMA/rba-YMA and AMA/rba-AMA biomarkers. The molecules contained therein could then be analysed using among others, any of the aforementioned methodological approaches that could target the biomarkers at the nucleic acid and/or protein level.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be illustrated by reference to specific Examples which are not intended to be limiting. Data from the Examples is presented in the Figures, in which:

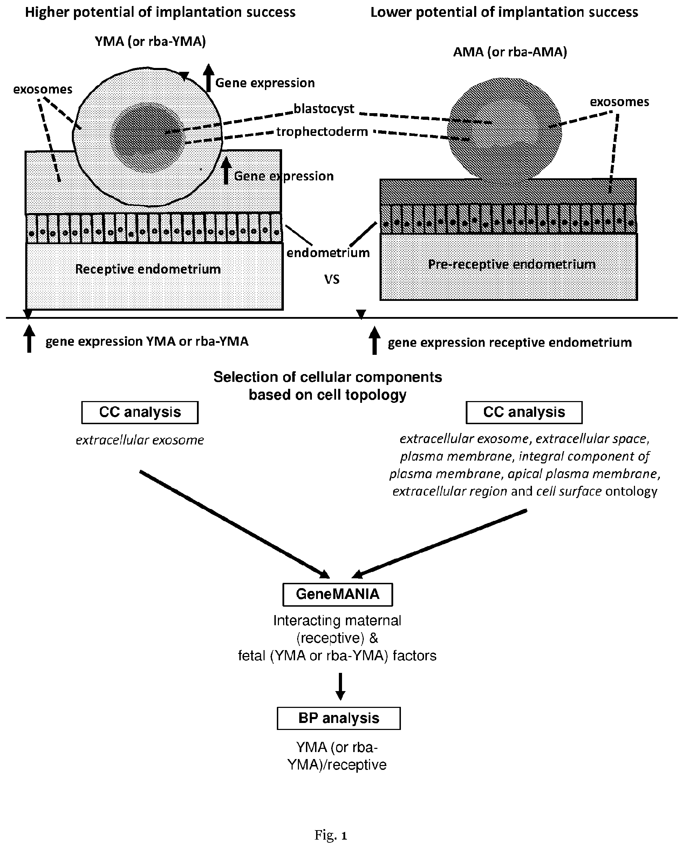

**Figure 1** is a diagram showing a molecular embryo-endometrial interaction model, assessing trophectoderm transcriptomes from AMA and YMA cohorts along with the endometrial pre-receptive and receptive RNA sequencing data. Trophectoderm cellular component (CC) analysis is applied after differential gene expression analysis. Ontologies potentially supporting maternal-fetal communication were selected. Exosome related ontologies were used in GeneMANIA module (Cytoscape). Similarly, plasma membrane-related and exosome ontologies from pre-receptive and receptive endometrial genes and plasma membrane- and exosome-related ontologies from YMA trophectoderm genes were subjected to GeneMANIA and the interacting factors altogether to biological process analysis (BP).
**Figure 2A** (parts A-D) shows eSNP-karyotyping analysis on trophectoderm RNA sequencing data, illustrating loss of heterozygosity (LOH) (right side) and allelic ratio (left side), along with the corresponding p-values corrected for multiple testing by false discovery rate (FDR).
**Figure 2B** (parts E-H) shows eSNP-karyotyping analysis on trophectoderm RNA sequencing data, illustrating loss of heterozygosity (LOH) (right side) and allelic ratio (left side), along with the corresponding p-values corrected for multiple testing by false discovery rate (FDR).
**Figure 2C** (parts I-L) shows eSNP-karyotyping analysis on trophectoderm RNA sequencing data, illustrating loss of heterozygosity (LOH) (right side) and allelic ratio (left side), along with the corresponding p-values corrected for multiple testing by false discovery rate (FDR).
**Figure 2D** (parts M-P) shows eSNP-karyotyping analysis on trophectoderm RNA sequencing data, illustrating loss of heterozygosity (LOH) (right side) and allelic ratio (left side), along with the corresponding p-values corrected for multiple testing by false discovery rate (FDR).
**Figure 2E** shows eSNP-karyotyping analysis on trophectoderm RNA sequencing data, illustrating loss of heterozygosity (LOH) (right side) and allelic ratio (left side), along with the corresponding p-values corrected for multiple testing by false discovery rate (FDR). The figure illustrates chromosome 2 aneuploidy (and in particular a non-viable trisomy), and such samples are preferably excluded from use in the disclosed methods.
**Figure 3A** shows a clustered heatmap for trophectoderm gene expression for the YMA, IMA and AMA cohorts. Samples are ordered by chronological maternal age. The gene expression levels represent YMA vs AMA and IMA vs AMA comparisons.
**Figure 3B** shows hierarchical clustering of the trophectoderm gene expression shown in Figure 3A, with samples ordered based on hierarchical clustering analysis that determined the biological reproductive maternal age (rba-YMA and rba-AMA).
**Figure 4A** shows a singular value decomposition (SVD) plot representing the trophectoderm transcriptome of chronological maternal age samples based on the first two Principal Components (PC1, PC2).
**Figure 4B** shows a visualization of biological maternal age samples, based on the first two Principal Components (PC1, PC2).
**Figure 5** shows a histogram illustrating fold-changes based on the gene expression patterns of selected genes in YMA samples using RNA-Seq (dark grey) and qRT-PCR (black) and in advanced maternal age (AMA) samples by RNA-Seq (light grey) and qRT-PCR (grey) methods.
**Figure 6A****.** Spearman's correlation was used to generate a heatmap of distance matrix. The colour gradient ranges from light (maximum distance) to dark grey (minimum distance). Horizontal bars (top of heatmap) represent the maternal age class, while the top and left dendrograms indicate cluster identification.
**Figure 6B****.** Unsupervised MDS plot was used to illustrate rba-YMA (squares), rba-IMA (triangles) and rba-AMA (circles) samples classified by reproductive maternal age.

### Examples

The inventors have developed a high throughput methodology comprising trophectoderm samples representing the full spectrum of maternal reproductive ages with embryo implantation potential examined in relation to trophectoderm transcriptome dynamics, reproductive maternal age and the implantation success following embryo transfer into the uterus. To explore potential embryo-endometrial interactions, the inventors considered trophectoderm from young women with a correspondingly receptive uterine environment that represents the most 'fertile' environment for successful embryo implantation, in comparison with the less 'fertile' system including blastocysts that derived from advanced maternal age women. In this regard, the current analysis included blastocysts without aneuploidies (that could affect implantation success). This resulted in a more targeted analysis that reduced the gene expression variation, increasing the statistical power of the study, hence the confidence of the reported biomarkers. This methodology highlighted informative molecular biomarkers of development and embryo-endometrial communication, revealing potential roles for processes involved in the regulation of apoptosis and embryonic metabolism, alongside extracellular exosomes. Blastocyst biopsies were consisted mainly by trophectoderm cells along with smaller quantities of RNA biomarkers present in the secretome of the analysed blastocysts i.e. blastocoel fluid and embryo culture media that the blastocysts were cultured in. The data analysis reported in this study provides methods useful for the selection of the blastocysts for embryo transfer that have a high likelihood of implantation success.

### Materials and Methods

### Patient recruitment

The current study was ethically approved by the A' Administration of the Health District of Attica, National Health System, General Children Hospital 'Aghia Sofia', Greece (Reference - Protocol Number: 19964/04-09-2014); the bioethics committee of Genesis Athens clinic; and the National Authority of Assisted Reproduction (Greece). Patients attending infertility treatment at Genesis Athens Clinic (Reproductive Medicine Unit) in Greece, provided informed consent to participate in the current study.

### Embryo culture, blastocyst biopsy procedure and clinical outcome

Intracytoplasmic sperm injection (ICSI) was used to fertilise all oocytes participating in the current study. Fertilised oocytes were cultured to the blastocyst stage in continuous single culture media supplemented with 5% Human Serum Albumin (HSA) (Irvine Scientific, CA, USA). Trophectoderm and inner cell mass (ICM) quality along with the degree of expansion were used to evaluate/assess the blastocysts participating in the current study, which were categorised as excellent (3-5AA), good (3-5B*) and average quality (3-5BB), while C* was considered as poor quality (Balaban et al., 2011, Gardner and Schoolcraft, 1999). The minimum morphological grade used was average quality (Gardner and Schoolcraft, 1999).

During trophectoderm biopsy, the blastocyst was oriented on the side opposite the biopsy pipette and in a way that the ICM was clearly visible (Cook Medical, Bloomington, USA) (Kokkali et al., 2007). Trophectoderm cells from all maternal age blastocyst groups (YMA, less than 30 years old; IMA, 30-39 years old; AMA over 40 years old) were gently aspirated into the biopsy pipette, before transfer into (10X) lysis buffer containing RNase Inhibitor (Clontech, USA).

Embryo implantation was determined by serum beta human Chorionic Gonadotropin levels (≥25 mIU/ml) (Roche Diagnostics International, Switzerland) on Day 16 following oocyte retrieval. Implantation was confirmed by demonstration of a gestational sac, 4 weeks after embryo transfer, by ultrasound scan. The presence of a fetal heartbeat beyond 7 weeks of gestation was defined as clinical pregnancy.

### Library construction and RNA sequencing

According to the manufacturer's instructions, SMART-Seq v4 Ultra Low Input RNA method (Clontech, USA) was used for library construction. Locked nucleic acid (LNA) technology was used for synthesizing the first cDNA strand and full-length cDNA was amplified by long distance PCR (LD-PCR). An optimised constant number of 13 PCR cycles was applied to all samples to permit accurate comparisons across samples, while minimising PCR bias. Agencourt Ampure XP Beads (Beckman Coulter, USA) purified the amplified cDNA.

The cDNA was quantified using the Qubit dsDNA HS (high sensitivity) fluorometric assay (Thermo Fisher Scientific, USA). A cDNA quantity of 150 pg was used with Nextera XT DNA library preparation, according to the manufacturer's protocol (Illumina, USA). Simultaneous tagging and DNA fragmentation was applied to the (full-length) cDNA in a single step, allowing a unique adapter index combination per library. The distribution of the size per cDNA library was assessed using the high sensitivity DNA assay (Agilent, USA). Paired-end deep RNA sequencing generated 150 bp reads on a Hi-Seq 3000 instrument (Illumina, USA), following equimolar pooling.

Sequence quality was assessed using FastQC tool (Andrews, 2010). Quality and Nextera adapter trimming were employed using Trim galore! (Krueger, 2015), with the reads passing quality control (QC) being mapped to the (hg38) human reference genome using the HISAT2 aligner (v2.1.0) (Pertea et al., 2016). Unmapped and unpaired reads were removed using Samtools (v1.8) (Li et al., 2009) and Picard tools v2.1.1 tagged PCR duplicates (Broad Institute. 2010. Available online at http://broadinstitute.github.io/picard). Only uniquely mapped, non-duplicated and correctly paired reads were considered as part of this analysis. Using an hg38 annotation file (UCSC) as a guide, StringTie was used to detect potentially novel transcripts (Pertea et al., 2015). The differential expression analysis robustness was assessed using two different approaches for assigning and quantifying mapped reads to known or predicted genes. The first approach used a python script (PythonScript, Accessed on March 2018) to extract read counts from the HISAT2/StringTie pipeline in a format suitable as input for the edgeR (v3.9) Bioconductor R package (Robinson and Oshlack, 2010), while the second approach employed the featureCounts function of the Subread Bioconductor R package to generate tables of counts (Liao et al., 2013).

### Bioinformatics analysis, molecular interactions and functional annotation

Trophectoderm transcriptomes derived from different maternal age groups were clustered by Multidimension Scaling (MDS) approach using edgeR (Robinson and Oshlack, 2010) and by principal component analysis (PCA) using Clustvis (Metsalu and Vilo, 2015, Available online at https://biit.cs.ut.ee/clustvis/).

The chronological maternal age was initially employed to construct a heatmap for visualising the differential gene expression patterns among the three maternal age cohorts (YMA, IMA and AMA), using the heatmap.plus R package (Day, 2012). Reproductive biological age (rba) (rba-YMA, rba-IMA and rba-AMA samples), was determined by unsupervised hierarchical clustering analysis of the differentially expressed trophectoderm transcripts, suggesting that trophectoderm transcriptome patterns of intermediate maternal age (30-40 years) may cluster with the YMA or AMA, forming the rba-YMA and rba-AMA groups. Logarithm fold-change (logFC) data was employed for conducting hierarchical clustering analysis. The reproductive biological age classification was supported both by the transcriptome analysis of YMA/rba-YMA and AMA/rba-AMA samples and the implantation success per group.

Computational analysis also employed the DaMiRseq (R package) approach to determine molecular biomarkers characteristic of the YMA/rba-YMA and AMA/rba-AMA samples (Chiesa et al., 2018). A correlation cutoff of 0.3 was used for normalisation and FSelect function was employed for partial least-square feature selection, considering gene expression in (at least) half of the samples of the rba-AMA group. The number of selected surrogate variables explained at least 90% of the variation. Default values were used for other functions. Distance matrix and multi-dimensional scaling plots were generated using different R packages (Wickham, 2016, Kolde and Kolde, 2015).

A methodological approach was designed to reveal potential relationships between the trophectoderm and endometrial expressed factors (Figure 1). DAVID performed ontological analysis on the YMA/rba-YMA, AMA/rba-AMA samples (Huang et al., 2009a, Huang et al., 2009b), investigating for enriched cellular components and biological processes. Multiple comparison corrections applied to all analyses and FDR (or FDRs) reported. More detailed consideration was provided to the significantly enriched ontologies with the potential to participate in maternal-fetal communication/interactions, such as extracellular exosomes, cell adhesion etc. Further investigation of the most significantly enriched YMA/rba-YMA cellular component (gene ontologies) were analysed in conjunction with the significantly more highly enriched cellular components of the receptive endometria (luteinizing hormone LH+7 or LH+8) (Figure 1).

Cytoscape GeneMANIA (v3.4.1) module assessed the gene expression products participating in the reported gene ontologies (Mostafavi et al., 2008, Warde-Farley et al., 2010). To focus on maternal-fetal interactions, this approach employed shared common pathways and/or known physical interactions that include protein coding factors expressed by the trophectoderm and the endometrium. In addition, biological process analysis between the potentially interacting endometrial (maternal) and trophectoderm (fetal) factors were investigated using DAVID (Huang et al., 2009a, Huang et al., 2009b), with the biological processes of maternal and fetal origin factors (that could be in close proximity), potentially being enriched. STRING (Jensen et al., 2009) was also used to further investigate the maternal and fetal interactions that participate in the resulting biological processes.

### Meta-analysis of endometrial factors

Using RNA sequencing, Hu (Hu et al., 2014) reported the differentially expressed transcripts between receptive (LH+7) and pre-receptive (LH+2) and Altmae (Altmäe et al., 2017) between receptive (LH+8) and pre-receptive (LH+2) endometria. RNA sequencing (Altmae) data was downloaded from Gene Expression Omnibus (GEO) database (www.nchi.nlm.nih.gov/geo/; accession number GSE98386), focusing on the significantly highly expressed genes in the receptive endometria and their potential interactions with the trophectoderm factors.

### Statistical analysis

The sequencing reads/counts were normalized and analysed statistically using EdgeR. The edgeR calcNormFactors function normalized read counts, based on the trimmed mean M value (TMM) (Robinson and Oshlack, 2010). The gene expression thresholds for inclusion in the differential expression analysis were 5 counts-per-million (cpm) reads in at least 4 samples. The analysis was performed both with and without the inclusion of non-coding transcripts. Statistical significance level was set at FDR<0.05.

EdgeR's generalized linear model (GLM) was employed for trophectoderm analysis between rba-YMA and rba-AMA or YMA/IMA and AMA samples, and exact test was used for endometrial differential expression analysis between receptive and non/pre-receptive endometria (Robinson and Oshlack, 2010). FDR was set at 0.05. The blastocyst developmental stage was examined in the three maternal age groups using the Kruskal Wallis test (kruskal.test() function) in R (Team, 2019). ANOVA was employed for examining the mean paternal age in the maternal age groups using aov() function in R. Normality (age and blastocyst grade) was assessed by Shapiro-Wilks test (Stats package). The association between trophectoderm gene expression with respect to implantation success and maternal age was investigated using Fisher's exact test (Stats package). RnaSeqSampleSize package calculated the power of the trophectoderm samples (32) that were recruited by the current study (Zhao et al., 2018). As a confirmation of the EdgeR results, DaMiRseq was used to analyse the gene expression levels between rba-YMA and rba-AMA groups, illustrating the robustness of the approach.

### Aneuploidy detection analysis by RNA sequencing data

The expressed single nucleotide polymorphism (eSNP) karyotyping approach was employed to determine the blastocyst ploidy status from the RNA sequencing data (Ntostis et al., 2019, Weissbein et al., 2016). Correctly paired and mapped reads by HISAT2 generated variant calls. Picard MarkDuplicates tool removed PCR duplicates and Picard AddOrReplaceReadGroups was used to add read group information (accessible at https://broadinstitute.github.io/picard/). SplitNCigarReads and HaplotypeCaller tools (GATK v4.1.0.0) created vcf files for downstream analyses (adapted from the eSNP-karyotyping approach) (Weissbein et al., 2016). The reference snp151Common table was downloaded from the UCSC Browser. During aneuploidy analysis, some reported varied allelic ratios around peri-centromeric regions could be explained by the low trophectoderm RNA input, potential mosaicism and the lower expression levels of peri-centromerically located genes and together with viable trisomies, were retained in the analysis, given that implantation outcome is not compromised. The complete workflow used for eSNP-karyotyping analysis is available online (https://github.com/daveiles/human_TEbiopsy_eSNPanalysis).

### Quantitative RT-PCR validation

Reverse transcription of trophectoderm RNA was employed using the ultra-low RNA input SMART protocol (Takara-Clontech, USA) to validate the RNA sequencing data. Briefly, long distance PCR (LD-PCR) amplification generated sufficient cDNA quantities and reverse transcription quantitative PCR (qRT-PCR) was performed on a LC480 thermal cycler (Roche, Switzerland). Three biological replicates were used per ageing group, with the annealing temperature set at 60°C (Table 2). Different endogenous controls genes were used, including GAPDH, ACTB, HNRNPC and H3F3B. The PCR data was normalized as described previously (Livak and Schmittgen, 2001).

**Table 2. DNA oligo sequences (primers) used to validate the RNA sequencing results by qRT-PCR. Gene names are reported together with the forward/reverse primer sequences and in all cases the melting temperature (Tm) was 60°C. ACTB, GAPDH, H3F3B and HNRNPC were used as reference genes (endogenous controls).**

| **Gene name** | **Primer (Forward)** | **Primer (Reverse)** |
|---|---|---|
| *ACTB* | AAGCCACCCCACTTCTCTCT SEQ ID No. 1 | CTATCACCTCCCCTGTGTGG SEQ ID No. 2 |
| *BAK1* | TTCACCAAGATTGCCACCAG SEQ ID No. 3 | ATGCTGGTAGACGTGTAGGG SEQ ID No. 4 |
| *BRAP* | TGACTGAACTCCCCAAGTGC SEQ ID No. 5 | GGCAAACAGGACACGTGGTA SEQ ID No. 6 |
| *CCPG1* | CCCCGAGTGCTATACCGAAC SEQ ID No. 7 | CAGACATCTTTCAGGTCATATGGAT SEQ ID No. 8 |
| *DAB2* | CTAGCTATTGCAAATGAGGGAAG SEQ ID No. 9 | GGTAATACTACTTGAACCCAGGAGCA SEQ ID No. 10 |
| *DBI* | GCTGCGAAGTGTAGACCCTTGT SEQ ID No. 11 | ATGGCTTGGTCTTAAGGTGCC SEQ ID No. 12 |
| *FABP3* | CGCCTGCTCTCTTGTAGCTT SEQ ID No. 13 | ATATGAGCGAGTGACTTCATGT SEQ ID No. 14 |
| *GAPDH* | TTGTCAAGCTCATTTCCTGGTAT SEQ ID No. 15 | TCTCTCTTCCTCTTGTGCTCTTG SEQ ID No. 16 |
| *H3F3B* | TGCTGGTTTTTCGCTCGTCG SEQ ID No. 17 | GCCATTTTCTTTCACCCAACGC SEQ ID No. 18 |
| *HNRNPC* | GCCAGCAACGTTACCAACAA SEQ ID No. 19 | TGAACAGAGCAGCCCACAAT SEQ ID No. 20 |
| *IFITM3* | CTGGGCTTCATAGCATTCGCCT SEQ ID No. 21 | AGATGTTCAGGCACTTGGCGGT SEQ ID No. 22 |
| *ZFANDS* | GTCCATCAGTTTCTCAGCCCA SEQ ID No. 23 | GGCAGTCAAACCCTGTAAGAC SEQ ID No. 24 |

### Production of Tables A-F

Biomarkers identified using the analytical methods described above are presented in Tables A-F. Each transcript is identified using the NCBI Reference Sequence ("refSeq ID") along with the corresponding associated gene name, and the following information is included:
- logFC: logarithm fold change of a particular transcript/gene, which is expressed at a significantly higher level in the group that is reported (e.g. YMA/rba-YMA), compared with the opposite group (e.g AMA/rba-AMA), as calculated by the edgeR package in R (for more information, see: https://www.bioconductor.org/packages/release/bioc/vignettes/edgeR/inst/doc/edgeRUsersGuide.pdf);
- FC: fold change. The FC number represents the value 2^logFC;
- logCPM: logarithm counts-per-million mapped reads (calculated by edgeR package in R);
- CPM: counts-per-million mapped reads. The CPM represents the value 2^logCPM;
- FDR: false discovery rate - adjusted P-value (calculated by edgeR package in R)

The purpose of the following examples is to demonstrate certain embodiments of the invention. It will be appreciated that the methods/techniques reported herein, represent approaches that could function well in the practice of the invention and can constitute models for practice. However, it may be appreciated that various alterations can be made in the methodologies and embodiments reported herein, and still obtain similar results within the scope of the current invention.

### Example 1

### Clinical samples and ageing molecular biomarkers

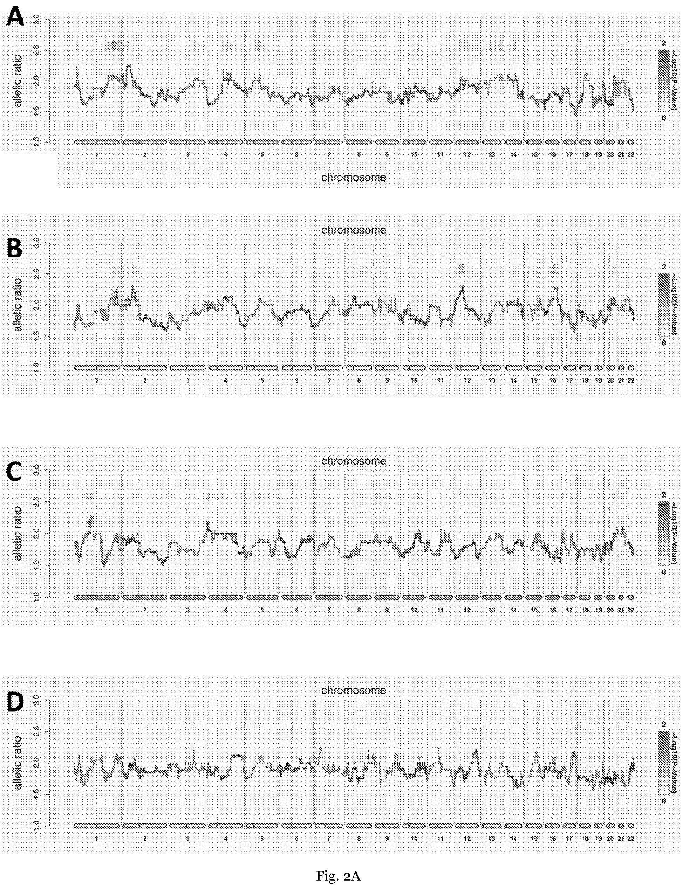

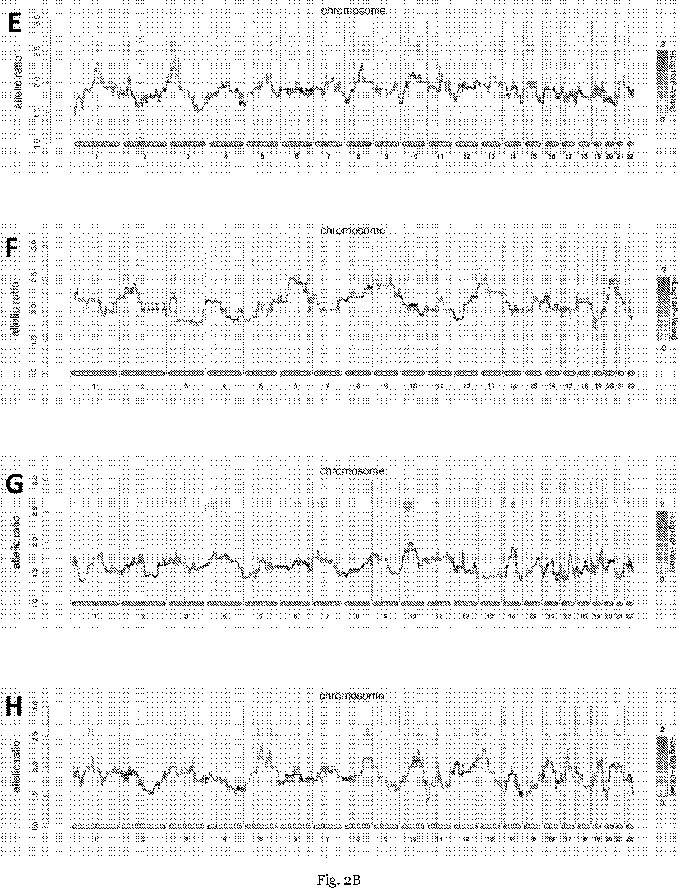

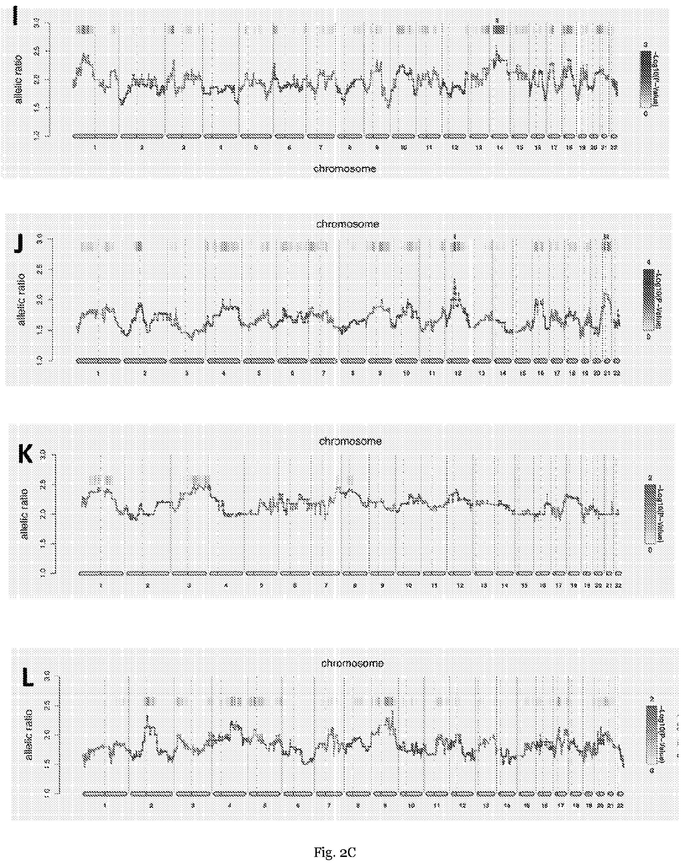

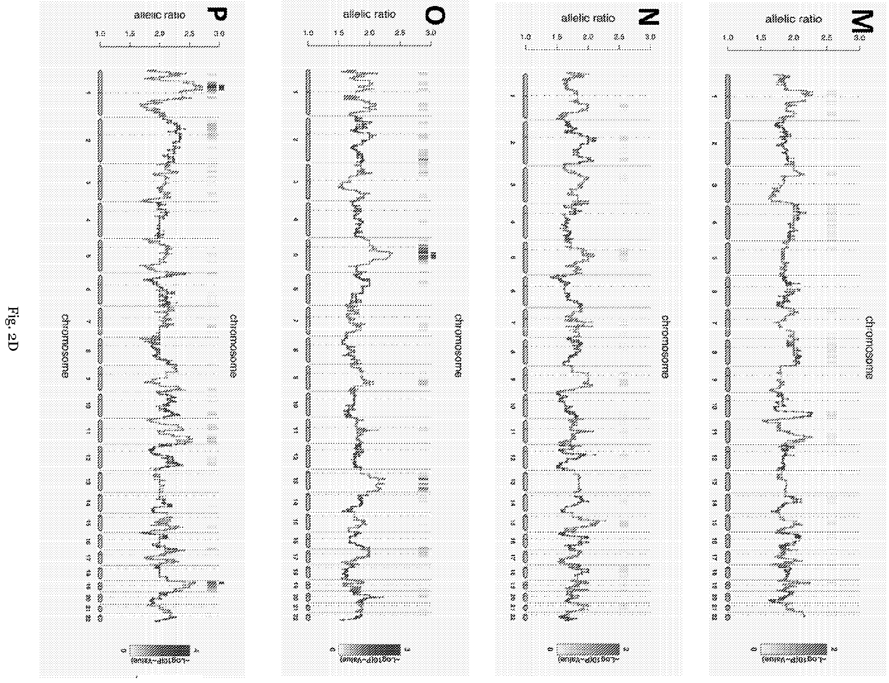

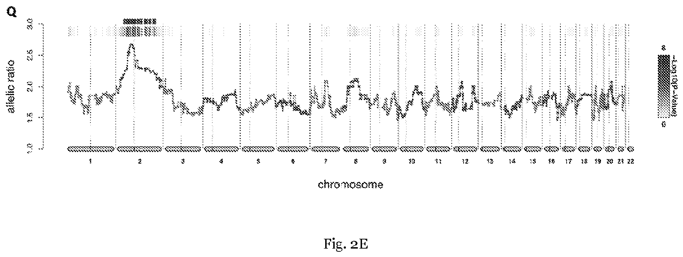

The inventors obtained trophectoderm biopsies from 36 individual blastocysts (Day 5), which were then used to generate whole transcriptome profiles. The RNA sequencing data was used in an eSNP-karyotyping approach (Weissbein et al., 2016, Ntostis et al., 2019) to identify the ploidy status of the analysed blastocysts, with the loss of heterozygosity (LOH) (Figure 2 - right side) and the allelic ratio (Figure 2 - left side) being reported (per plot) for each trophectoderm sample. Samples with aneuploidies that were indicative of a non-viable embryo, were excluded from the analysis (e.g. Figure 2E), with 32 samples being finally included (e.g. Figure 2A-D).

The young maternal age (YMA), intermediate maternal age (IMA) and advanced maternal age (AMA) groups were composed of 4 women (<30 years old), 8 women (30-39 years old) and 3 women (at least 40 years), respectively. In particular, YMA, IMA and AMA groups included 10, 16 and 6 trophectoderm biopsies/samples, respectively, with mean (SD) maternal ages of 24.4 (2.0) years, 34.3 (2.6) years and 42 (1.1) years. The corresponding paternal mean (SD) ages for YMA, IMA and AMA groups were 43.6 (5.7) years, 40.3 (6.5) years and 41.3 (2.5) years, respectively, indicating no statistical significance from the paternal age perspective. The distributions of the developmental stages of the analysed blastocysts were also similar.

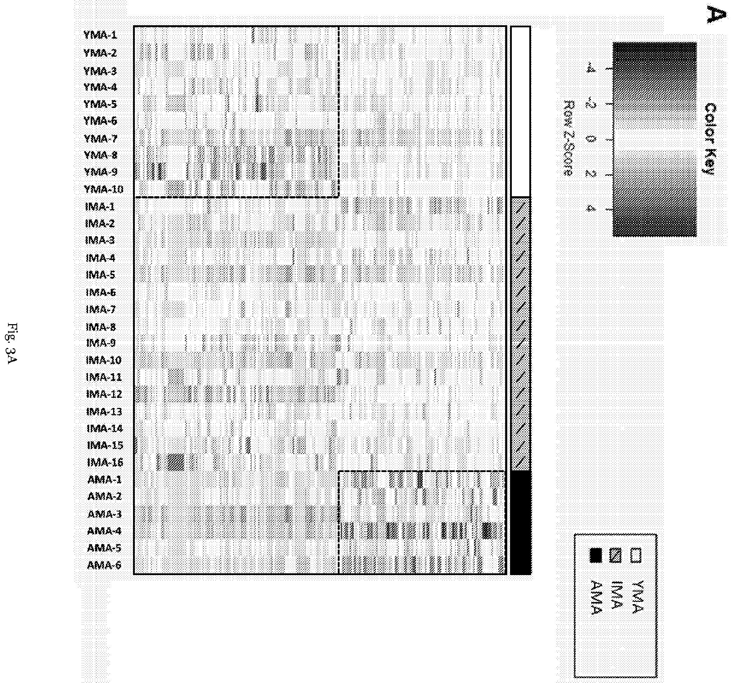

Ruling out the factors that may affect trophectoderm gene expression levels, hence normal embryo development and/or implantation success, the inventors performed differential expression analysis among the YMA, IMA and AMA chronological maternal age groups. The analysis that included the protein-coding transcripts, illustrated 580 differentially expressed transcripts (or 233 unique genes) when YMA and AMA samples were compared, of which 312 transcripts (or 111 unique genes) were significantly higher expressed in the YMA and the remaining 268 transcripts (122 unique genes) higher expressed in the AMA cohort. No differentially expressed transcripts were detected when YMA and IMA groups were compared. These results were used to generate a clustered heatmap that includes the differential expression between YMA vs AMA and IMA vs AMA chronological age comparisons (Figure 3A), illustrated as Z-scores, with the mean expression level shown in white. The higher gene expression levels between YMA and AMA samples are included in the bottom left (YMA) and top right (AMA) squared (dashed) boxes.

The gene expression patterns of the IMA samples illustrated either similarities with the expression patterns of the YMA or AMA groups (more extreme ageing groups) or a distinct profile. Trophectoderm gene expression patterns of the YMA and AMA samples showed more homogeneous gene expression patterns within each group, although there were significant differences between the expression patterns of these two groups, supporting the effect of maternal age in the trophectoderm gene expression (Figure 3A).

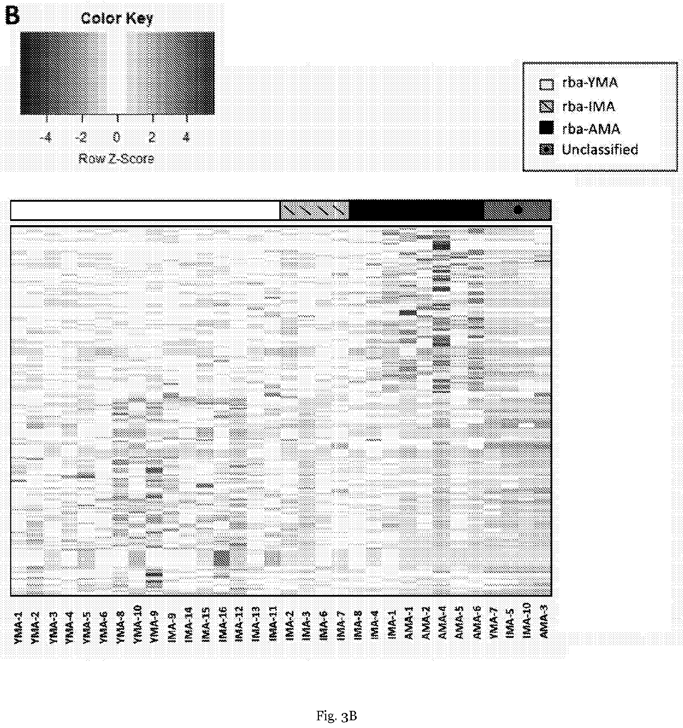

Hierarchical clustering analysis was performed using the above as a guide, categorising the samples in 4 main distinct groups based on their trophectoderm gene expression patterns, including the reproductive young biological maternal age (rba-YMA; 16 samples); the reproductive biological advanced maternal age (rba-AMA; 8 samples); the reproductive biological intermediate maternal age (rba-IMA; 4 samples) group that seems to be a transition between the rba-YMA and rba-AMA; and the 'unclassified' group (Figure 3B). The latter could be either a transition state between rba-YMA and rba-AMA or (for various reasons) an unclassified group of distinct gene expression levels. The main focus, however, was between the rba-YMA and rba-AMA samples that better described the trophectoderm gene expression patterns as a function of (biological) age and which overlapped to a great extent with the gene expression patterns of the YMA or AMA samples. In this regard, the inventors' findings suggest that the trophectoderm transcriptome could be a useful predictor/indicator of maternal biological age irrespective of the exact chronological age, especially in samples of women aged between 30 and 40 years (transition reproductive period).

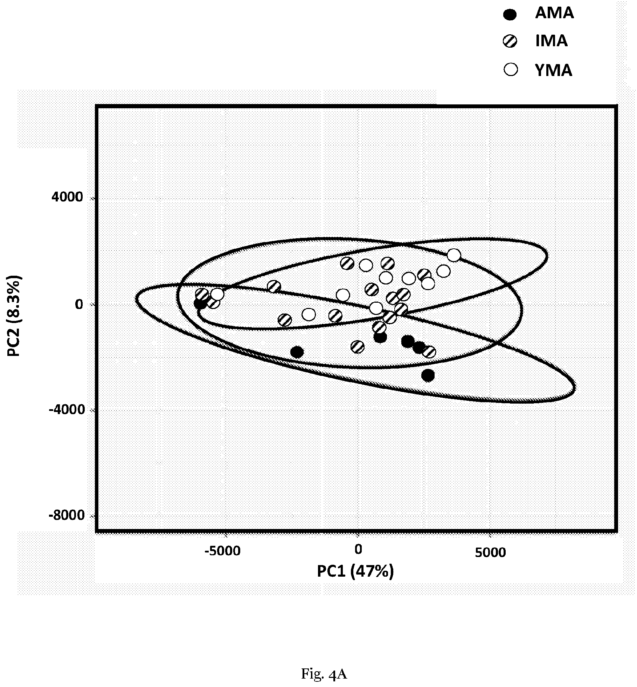

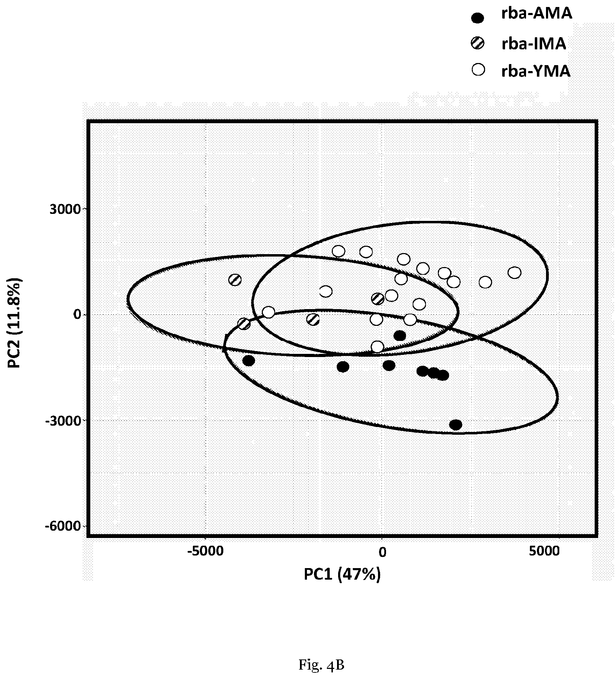

Principal Component Analysis (PCA) and Multidimension Scaling (MDS) analysis illustrated additional insights into the relationships among the transcriptome profiles/patterns of the different age groups, with a gradual shift illustrated both when chronological (YMA to IMA to AMA) and biological (rba-YMA to rba-IMA to rba-AMA) age were considered (Figure 4). Moreover, the YMA and rba-YMA always lie further apart from the AMA and rba-AMA samples, respectively, compared with the IMA and rba-IMA samples that illustrate transcriptome patterns more closely related (overlapping) with the young maternal age group. Differential expression analysis illustrated approximately 75% overlap between chronological and biological maternal age transcripts, also referred to as YMA/rba-YMA and AMA/rba-AMA groups. The statistical variability when rba-YMA and rba-AMA samples were compared, was approximately 0.39 (cpm>5), corresponding to a study powered to 70%.

The genes and gene transcripts in Table A have been found to have an elevated level of expression that is statistically significantly associated with trophoblasts having a young chronological/biological reproductive age and are listed in Table A in order of statistical significance expressed by false discovery rate adjusted p-value (FDR), with FDR<0.05 considered as significant level. The expression level of any one or more of the genes listed in Table A can, therefore, be used as a biomarker to identify blastocysts having an increased likelihood of implantation success.

The term Table* is used to define the genes shown with an asterisk in each corresponding table, for example Table A* means all the genes of Table A having an asterisk in the 6^{th} or 12^{th} column. At least one of these genes has to be included in the invention, when for example "at least 1 or 2 or 3 etc genes" are used to indicate markers supporting a specific implantation outcome. These genes are preferentially selected, given some parameters that include among others the thermodynamics of the oligos/primers the target them, the importance of these genes and their biological processes they are involved. Hence, in preferred embodiments, at least one of the genes shown with an asterisk (*) in Table A, Table B, Table C and/or Table D are selected for use in the claimed invention.

"Table*" consists of all of the genes in Tables A-D that are highlighted in column P using an asterisk (*). Thus, "Table A*" consists of all of the genes in Table A that are highlighted in column P using an asterisk, "Table A1*" consists of all of the genes in Table A1 that are highlighted in column P using an asterisk, "Table C*" consists of all of the genes in Table C that are highlighted in column P using an asterisk and likewise for each of the other Tables B and and Tables D-G. The genes highlighted with an asterisk in column P have been found to be particularly preferred for use in the invention, due to the inventors having found them to have particularly advantageous parameters that include among others the thermodynamics of the oligos/primers that target these genes. In view of the observed advantages of the genes indicated with an asterisk in column P, preferred embodiments of the invention involve the use of at least one of these genes.

Table A is made up of Table A1, referred to as "Table A protein coding transcripts", and Table A2, referred to as "Table A non-coding transcripts". Differential expression analyses with and without considering the non-coding transcripts revealed a few hundreds of differentially expressed factors between the YMA/rba-YMA and AMA/rba-AMA groups with various levels of significance. The protein-coding YMA/rba-YMA biomarkers with higher likelihood (FDR<0.01) to describe the maternal age and developmental potential, are illustrated in Table A1 and the corresponding non-coding biomarkers in Table A2, reporting 416 (147 genes) and 21 non-coding transcripts, respectively.

**Table A1**

| **YMA/rba-YMA present in both analyses (i.e. with and without non-coding NR_) & Qvalue<0.01** | | | | | | | | | | | |
|---|---|---|---|---|---|---|---|---|---|---|---|
| **refSeq** | **gene name** | **logFC** | **log CPM** | **FDR** | **P** | **refSeq** | **gene name** | **logFC** | **log CPM** | **FDR** | **P** |
| NM_001079862 | DBI | 1,59 | 6,87 | 1,57E-08 | # | NM_001130851 | CDKN3 | 1,18 | 4,37 | 1,17E-03 | * |
| NM_001079863 | DBI | 1,59 | 6,87 | 1,57E-08 | # | NM_001330173 | CDKN3 | 1,18 | 4,37 | 1,20E-03 | * |
| NM_001178017 | DBI | 1,59 | 6,87 | 1,57E-08 | # | NM_002790 | PSMA5 | 0,70 | 7,07 | 1,21E-03 | * |
| NM_001178041 | DBI | 1,59 | 6,87 | 1,57E-08 | # | NM_006365 | C1orf61 | 2,34 | 2,42 | 1,23E-03 | * |
| NM_001178042 | DBI | 1,59 | 6,87 | 1,57E-08 | # | NM_001199772 | PSMA5 | 0,70 | 7,07 | 1,23E-03 | * |
| NM_001178043 | DBI | 1,59 | 6,87 | 1,57E-08 | # | NM_001199773 | PSMA5 | 0,70 | 7,07 | 1,23E-03 | * |
| NM_001282633 | DBI | 1,59 | 6,87 | 1,57E-08 | # | NM_001199774 | PSMA5 | 0,70 | 7,07 | 1,23E-03 | * |
| NM_001282634 | DBI | 1,59 | 6,87 | 1,57E-08 | # | NM_000786 | CYP51A1 | 2,12 | 4,77 | 1,26E-03 | # |
| NM_001282635 | DBI | 1,59 | 6,87 | 1,57E-08 | # | NM_001146152 | CYP51A1 | 2,11 | 4,73 | 1,39E-03 | # |
| NM_001282636 | DBI | 1,59 | 6,87 | 1,57E-08 | # | NM_001293273 | MCCC1 | 3,62 | 1,42 | 1,41E-03 | * |
| NM_001352432 | DBI | 1,59 | 6,87 | 1,57E-08 | # | NM_020166 | MCCC1 | 3,62 | 1,42 | 1,41E-03 | * |
| NM_020548 | DBI | 1,59 | 6,87 | 1,57E-08 | # | NM_001320453 | C1orf61 | 2,40 | 2,30 | 1,46E-03 | * |
| NM_001320996 | FABP3 | 3,64 | 6,36 | 1,66E-08 | # | NM_001135055 | TKT | 1,14 | 5,98 | 1,60E-03 | * |
| NM_004102 | FABP3 | 3,64 | 6,36 | 1,66E-08 | # | NM_001202450 | ADK | 1,65 | 5,20 | 1,63E-03 | * |
| NM_001098272 | HMGCS1 | 2,93 | 5,37 | 6,09E-08 | * | NM_006721 | ADK | 1,65 | 5,21 | 1,63E-03 | * |
| NM_001324219 | HMGCS1 | 2,93 | 5,37 | 6,09E-08 | * | NM_001096 | ACLY | 1,34 | 5,98 | 1,75E-03 | * |
| NM_001324220 | HMGCS1 | 2,93 | 5,37 | 6,09E-08 | * | NM_001303274 | ACLY | 1,34 | 5,98 | 1,75E-03 | * |
| NM_001324222 | HMGCS1 | 2,93 | 5,37 | 6,09E-08 | * | NM_001303275 | ACLY | 1,34 | 5,98 | 1,75E-03 | * |
| NM_001324223 | HMGCS1 | 2,93 | 5,37 | 6,09E-08 | * | NM_198830 | ACLY | 1,34 | 5,98 | 1,75E-03 | * |
| NM_001324224 | HMGCS1 | 2,93 | 5,37 | 6,09E-08 | * | NM_001202449 | ADK | 1,65 | 5,21 | 1,80E-03 | * |
| NM_001330663 | HMGCS1 | 2,93 | 5,37 | 6,09E-08 | * | NM_001123 | ADK | 1,64 | 5,21 | 1,80E-03 | * |
| NM_002130 | HMGCS1 | 2,93 | 5,37 | 6,09E-08 | * | NM_024051 | GGCT | 1,29 | 4,47 | 1,82E-03 | * |
| NM_000413 | HSD17B1 | 3,45 | 5,05 | 3,41E-07 | # | NM_001199816 | GGCT | 1,29 | 4,46 | 1,85E-03 | * |
| NM_001330219 | HSD17B1 | 3,45 | 5,05 | 3,41E-07 | # | NM_001199815 | GGCT | 1,29 | 4,47 | 1,94E-03 | * |
| NM_002927 | RGS13 | 7,70 | 2,56 | 4,57E-07 | * | NM_001199817 | GGCT | 1,28 | 4,42 | 2,22E-03 | * |
| NM_144766 | RGS13 | 7,68 | 2,55 | 4,57E-07 | * | NM_001010980 | NCMAP | 3,22 | 1,60 | 2,22E-03 | * |
| NM_001135099 | TMPRSS2 | 5,82 | 2,73 | 2,54E-06 | # | NM_004255 | COX5A | 0,68 | 7,63 | 2,34E-03 | * |
| NM_005656 | TMPRSS2 | 5,82 | 2,73 | 2,54E-06 | # | NM_014412 | CACYBP | 0,66 | 7,52 | 2,47E-03 | * |
| NM_021034 | IFITM3 | 3,06 | 5,75 | 4,47E-06 | * | NM_001884 | HAPLN1 | 7,51 | 1,33 | 2,50E-03 | * |
| NM_005891 | ACAT2 | 1,48 | 7,49 | 4,62E-06 | * | NM_000431 | MVK | 2,29 | 2,23 | 2,50E-03 | * |
| NM_001303253 | ACAT2 | 1,32 | 7,68 | 5,59E-06 | * | NM_001114185 | MVK | 2,29 | 2,23 | 2,50E-03 | * |
| NM_006645 | STARD10 | 1,56 | 6,21 | 1,07E-05 | * | NM_001301182 | MVK | 2,29 | 2,23 | 2,50E-03 | * |
| NM_001145160 | TPM4 | 1,85 | 6,91 | 1,73E-05 | # | NM_001007214 | CACYBP | 0,66 | 7,51 | 2,52E-03 | * |
| NM_003290 | TPM4 | 1,85 | 6,92 | 1,73E-05 | # | NM_001190329 | ATP5MC3 | 0,63 | 8,05 | 2,55E-03 | * |
| NM_014365 | HSPB8 | 7,85 | 2,82 | 2,12E-05 | # | NM_001002258 | ATP5MC3 | 0,63 | 8,04 | 2,56E-03 | * |
| NM_001076552 | ACSS2 | 3,93 | 3,75 | 2,66E-05 | # | NM_001689 | ATP5MC3 | 0,63 | 8,04 | 2,56E-03 | * |
| NM_018677 | ACSS2 | 3,94 | 3,75 | 2,66E-05 | # | NM_001350001 | JCAD | 3,31 | 2,64 | 2,56E-03 | * |
| NM_001242393 | ACSS2 | 3,95 | 3,74 | 2,83E-05 | # | NM_020848 | JCAD | 3,31 | 2,64 | 2,56E-03 | * |
| NM_001317955 | IDI1 | 1,91 | 6,68 | 3,21E-05 | # | NM_001163817 | DHCR7 | 2,43 | 5,22 | 2,57E-03 | # |
| NM_006435 | IFITM2 | 2,78 | 4,98 | 3,21E-05 | * | NM_001360 | DHCR7 | 2,43 | 5,22 | 2,57E-03 | # |
| NM_001244871 | DAB2 | 1,96 | 8,01 | 3,28E-05 | * | NM_001350021 | JCAD | 3,31 | 2,64 | 2,57E-03 | * |
| NM_001343 | DAB2 | 1,96 | 8,01 | 3,28E-05 | * | NM_001350022 | JCAD | 3,31 | 2,64 | 2,57E-03 | * |
| NM_013349 | NENF | 1,70 | 5,52 | 3,43E-05 | * | NM_001697 | ATP5PO | 0,64 | 7,78 | 2,72E-03 | * |
| NM_001099773 | CYP11A1 | 5,00 | 2,91 | 3,68E-05 | # | NM_000527 | LDLR | 3,99 | 3,01 | 2,74E-03 | * |
| NM_001317956 | IDI1 | 1,92 | 6,67 | 3,68E-05 | # | NM_001195798 | LDLR | 3,99 | 3,01 | 2,74E-03 | * |
| NM_000781 | CYP11A1 | 5,01 | 2,93 | 3,76E-05 | # | NM_001145306 | CDK6 | 1,35 | 4,26 | 2,75E-03 | * |
| NM_001317957 | IDI1 | 1,92 | 6,66 | 3,76E-05 | # | NM_001195799 | LDLR | 3,98 | 3,01 | 2,75E-03 | * |
| NM_004508 | IDI1 | 1,91 | 6,68 | 3,76E-05 | # | NM_001259 | CDK6 | 1,34 | 4,26 | 2,78E-03 | * |
| NM_006558 | KHDRBS3 | 1,37 | 6,20 | 4,09E-05 | # | NM_001195803 | LDLR | 3,97 | 3,00 | 2,84E-03 | * |
| NM_001017369 | MSMO1 | 2,27 | 6,70 | 4,22E-05 | # | NM_004104 | FASN | 1,74 | 4,49 | 2,91E-03 | * |
| NM_006745 | MSMO1 | 2,28 | 6,73 | 4,25E-05 | # | NM_001195800 | LDLR | 3,95 | 2,98 | 3,02E-03 | * |
| NM_033140 | CALD1 | 2,54 | 4,51 | 6,08E-05 | * | NM_002567 | PEBP1 | 0,96 | 6,14 | 3,09E-03 | * |
| NM_004342 | CALD1 | 2,52 | 4,49 | 6,14E-05 | * | NM_005979 | S100A13 | 1,16 | 6,86 | 3,10E-03 | * |
| NM_001291738 | CD24 | 3,77 | 4,55 | 7,33E-05 | # | NM_001328666 | ARHGAP29 | 1,58 | 4,32 | 3,10E-03 | * |
| NM_033139 | CALD1 | 2,52 | 4,52 | 7,66E-05 | * | NM_004815 | ARHGAP29 | 1,58 | 4,32 | 3,10E-03 | * |
| NM_033157 | CALD1 | 2,50 | 4,49 | 7,66E-05 | * | NM_001024211 | S100A13 | 1,16 | 6,86 | 3,10E-03 | * |
| NM_001291737 | CD24 | 3,76 | 4,55 | 7,66E-05 | # | NM_001289939 | SLC1A3 | 2,05 | 4,72 | 3,14E-03 | * |
| NM_001291739 | CD24 | 3,77 | 4,54 | 7,66E-05 | # | NM_001289940 | SLC1A3 | 2,05 | 4,70 | 3,21E-03 | * |
| NM_001359084 | CD24 | 3,77 | 4,55 | 7,66E-05 | # | NM_001024212 | S100A13 | 1,15 | 6,86 | 3,23E-03 | * |
| NM_013230 | CD24 | 3,76 | 4,56 | 7,66E-05 | # | NM_001320930 | GTF2A2 | 0,62 | 6,89 | 3,23E-03 | * |
| NM_001136479 | ANP32E | 2,55 | 3,67 | 9,10E-05 | * | NM_004492 | GTF2A2 | 0,62 | 6,87 | 3,28E-03 | * |
| NM_001257386 | ABCG2 | 2,41 | 5,56 | 9,21E-05 | # | NM_001008392 | CTDSPL | 2,02 | 3,35 | 3,31E-03 | * |
| NM_001348985 | ABCG2 | 2,41 | 5,56 | 9,21E-05 | # | NM_005808 | CTDSPL | 2,02 | 3,35 | 3,31E-03 | * |
| NM_001348989 | ABCG2 | 2,41 | 5,56 | 9,21E-05 | # | NM_001320929 | GTF2A2 | 0,62 | 6,88 | 3,35E-03 | * |
| NM_033138 | CALD1 | 2,44 | 4,54 | 9,21E-05 | * | NM_001024213 | S100A13 | 1,14 | 6,87 | 3,39E-03 | * |
| NM_001348987 | ABCG2 | 2,41 | 5,60 | 9,24E-05 | # | NM_001166695 | SLC1A3 | 2,04 | 4,74 | 3,42E-03 | * |
| NM_001348988 | ABCG2 | 2,41 | 5,60 | 9,24E-05 | # | NM_018235 | CNDP2 | 1,08 | 5,74 | 3,46E-03 | * |
| NM_004827 | ABCG2 | 2,41 | 5,60 | 9,24E-05 | # | NM_004172 | SLC1A3 | 2,04 | 4,74 | 3,46E-03 | * |
| NM_052845 | MMAB | 2,60 | 4,00 | 9,24E-05 | * | NM_002966 | S100A10 | 0,69 | 8,21 | 3,58E-03 | * |
| NM_006868 | RAB31 | 3,67 | 3,93 | 9,24E-05 | * | NM_001328664 | ARHGAP29 | 1,58 | 4,31 | 3,70E-03 | * |
| NM_001348986 | ABCG2 | 2,41 | 5,60 | 9,25E-05 | # | NM_001168499 | CNDP2 | 1,07 | 5,72 | 3,70E-03 | * |
| NM_002867 | RAB3B | 2,61 | 3,08 | 9,96E-05 | * | NM_001328667 | ARHGAP29 | 1,58 | 4,29 | 3,73E-03 | * |
| NM_080491 | GAB2 | 1,74 | 3,74 | 1,12E-04 | * | NM_001077490 | GNAS | 0,90 | 6,24 | 3,74E-03 | * |
| NM_001242825 | FDPS | 1,74 | 6,99 | 1,13E-04 | # | NM_016592 | GNAS | 0,90 | 6,24 | 3,74E-03 | * |
| NM_012296 | GAB2 | 1,74 | 3,74 | 1,15E-04 | * | NM_080425 | GNAS | 0,90 | 6,24 | 3,74E-03 | * |
| NM_001135822 | FDPS | 1,74 | 7,01 | 1,16E-04 | # | NM_001309861 | GNAS | 0,90 | 6,24 | 3,75E-03 | * |
| NM_001242824 | FDPS | 1,74 | 7,01 | 1,16E-04 | # | NM_001024210 | S100A13 | 1,11 | 6,90 | 3,76E-03 | * |
| NM_001135821 | FDPS | 1,73 | 7,01 | 1,20E-04 | # | NM_001309840 | GNAS | 0,90 | 6,25 | 3,85E-03 | * |
| NM_002004 | FDPS | 1,73 | 7,01 | 1,20E-04 | # | NM_080426 | GNAS | 0,90 | 6,25 | 3,85E-03 | * |
| NM_001256534 | SLC25A1 | 2,11 | 6,20 | 1,30E-04 | * | NM_001077489 | GNAS | 0,90 | 6,25 | 3,87E-03 | * |
| NM_001287387 | SLC25A1 | 2,11 | 6,20 | 1,30E-04 | * | NM_001137553 | LRRFIP1 | 1,02 | 5,59 | 3,88E-03 | * |
| NM 005984 | SLC25A1 | 2,11 | 6,20 | 1,30E-04 | * | NM_001321386 | PTN | 2,34 | 3,36 | 3,88E-03 | * |
| NM_001280559 | ANP32E | 2,52 | 3,83 | 1,37E-04 | * | NM_000516 | GNAS | 0,90 | 6,25 | 3,90E-03 | * |
| NM_001280560 | ANP32E | 2,52 | 3,83 | 1,37E-04 | * | NM_001077488 | GNAS | 0,90 | 6,25 | 3,90E-03 | * |
| NM_030920 | ANP32E | 2,52 | 3,83 | 1,37E-04 | * | NM_006788 | RALBP1 | 0,66 | 6,20 | 3,94E-03 | * |
| NM_001136478 | ANP32E | 2,52 | 3,83 | 1,58E-04 | * | NM_003849 | SUCLG1 | 0,79 | 6,46 | 3,94E-03 | * |
| NM_024605 | ARHGAP10 | 2,52 | 3,30 | 1,83E-04 | # | NM_001199462 | PDCD2 | 0,88 | 4,99 | 3,97E-03 | * |
| NM_001030019 | SUN3 | 1,43 | 5,07 | 2,25E-04 | * | NM_002598 | PDCD2 | 0,88 | 4,99 | 3,97E-03 | * |
| NM_001284350 | SUN3 | 1,43 | 5,07 | 2,25E-04 | * | NM_001161560 | TNIK | 4,16 | 1,44 | 3,97E-03 | * |
| NM_152782 | SUN3 | 1,43 | 5,07 | 2,25E-04 | * | NM_001161561 | TNIK | 4,16 | 1,44 | 3,97E-03 | * |
| NM_178556 | TRIML1 | 3,90 | 2,79 | 2,26E-04 | * | NM_001161562 | TNIK | 4,16 | 1,44 | 3,97E-03 | * |
| NM_001029989 | PCLAF | 2,78 | 3,20 | 2,57E-04 | * | NM_001161563 | TNIK | 4,16 | 1,44 | 3,97E-03 | * |
| NM_014736 | PCLAF | 2,78 | 3,21 | 2,64E-04 | * | NM_001161564 | TNIK | 4,16 | 1,44 | 3,97E-03 | * |
| NM_001130455 | DYSF | 2,51 | 3,44 | 2,67E-04 | * | NM_001161565 | TNIK | 4,16 | 1,44 | 3,97E-03 | * |
| NM_001130976 | DYSF | 2,47 | 3,53 | 2,67E-04 | * | NM_001161566 | TNIK | 4,16 | 1,44 | 3,97E-03 | * |
| NM_001130977 | DYSF | 2,47 | 3,53 | 2,67E-04 | * | NM_015028 | TNIK | 4,16 | 1,44 | 3,97E-03 | * |
| NM_001130978 | DYSF | 2,47 | 3,53 | 2,67E-04 | * | NM_004735 | LRRFIP1 | 1,02 | 5,60 | 3,97E-03 | * |
| NM_001130979 | DYSF | 2,47 | 3,53 | 2,67E-04 | * | NM_001137552 | LRRFIP1 | 1,02 | 5,60 | 3,98E-03 | * |
| NM_001130980 | DYSF | 2,47 | 3,53 | 2,67E-04 | * | NM_001321387 | PTN | 2,34 | 3,31 | 4,02E-03 | * |
| NM_001130981 | DYSF | 2,47 | 3,53 | 2,67E-04 | * | NM_002825 | PTN | 2,34 | 3,31 | 4,02E-03 | * |
| NM_001130982 | DYSF | 2,50 | 3,44 | 2,67E-04 | * | NM_016406 | UFC1 | 0,85 | 5,77 | 4,03E-03 | * |
| NM_001130983 | DYSF | 2,51 | 3,44 | 2,67E-04 | * | NM_005340 | HINT1 | 1,41 | 6,05 | 4,24E-03 | * |
| NM_0011309 84 | DYSF | 2,51 | 3,44 | 2,67E-04 | * | NM_003681 | PDXK | 0,91 | 6,68 | 4,27E-03 | * |
| NM_001130985 | DYSF | 2,50 | 3,44 | 2,67E-04 | * | NM_014899 | RHOBTB3 | 4,12 | 1,65 | 4,41E-03 | * |
| NM_001130986 | DYSF | 2,51 | 3,44 | 2,67E-04 | * | NM_003641 | IFITM1 | 1,41 | 3,31 | 4,41E-03 | * |
| NM_001130987 | DYSF | 2,50 | 3,44 | 2,67E-04 | * | NM_001916 | CYC1 | 0,95 | 6,99 | 4,43E-03 | * |
| NM_003494 | DYSF | 2,47 | 3,53 | 2,67E-04 | * | NM_001331030 | PDXK | 0,91 | 6,62 | 4,44E-03 | * |
| NM_198321 | GALNT10 | 2,12 | 4,32 | 2,67E-04 | * | NM_001001503 | NDUFV3 | 1,01 | 4,86 | 4,49E-03 | * |
| NM_001282211 | NDRG2 | 3,99 | 2,66 | 2,67E-04 | * | NM_001328665 | ARHGAP29 | 1,58 | 4,25 | 4,56E-03 | * |
| NM_001282212 | NDRG2 | 3,99 | 2,66 | 2,67E-04 | * | NM_001303029 | PKP3 | 2,24 | 4,10 | 4,67E-03 | * |
| NM_001282213 | NDRG2 | 3,99 | 2,66 | 2,67E-04 | * | NM_007183 | PKP3 | 2,24 | 4,10 | 4,67E-03 | * |
| NM_001282214 | NDRG2 | 3,99 | 2,66 | 2,67E-04 | * | NM_018946 | NANS | 1,02 | 4,96 | 5,00E-03 | * |
| NM_001282215 | NDRG2 | 3,99 | 2,66 | 2,67E-04 | * | NM_016021 | UBE2J1 | 3,75 | 2,77 | 5,39E-03 | * |
| NM_001282216 | NDRG2 | 3,99 | 2,66 | 2,67E-04 | * | NM_001688 | ATP5PB | 0,59 | 7,63 | 5,63E-03 | * |
| NM_001354558 | NDRG2 | 3,99 | 2,66 | 2,67E-04 | * | NM_001277224 | TAGLN2 | 0,67 | 8,29 | 5,67E-03 | * |
| NM_001354559 | NDRG2 | 4,00 | 2,67 | 2,67E-04 | * | NM_003564 | TAGLN2 | 0,67 | 8,29 | 5,67E-03 | * |
| NM_001354560 | NDRG2 | 3,99 | 2,66 | 2,67E-04 | * | NM_001277223 | TAGLN2 | 0,67 | 8,29 | 5,68E-03 | * |
| NM_001354561 | NDRG2 | 3,99 | 2,66 | 2,67E-04 | * | NM_001017367 | C4BPB | 2,71 | 3,09 | 5,82E-03 | * |
| NM_001354562 | NDRG2 | 3,99 | 2,66 | 2,67E-04 | * | NM_007176 | ERG28 | 1,43 | 5,14 | 5,84E-03 | * |
| NM_001354564 | NDRG2 | 3,99 | 2,66 | 2,67E-04 | * | NM_022071 | SH2D4A | 1,84 | 4,36 | 5,91E-03 | * |
| NM_001354565 | NDRG2 | 4,00 | 2,67 | 2,67E-04 | * | NM_001174159 | SH2D4A | 1,84 | 4,36 | 5,94E-03 | * |
| NM_001354566 | NDRG2 | 3,99 | 2,66 | 2,67E-04 | * | NM_000716 | C4BPB | 2,71 | 3,10 | 6,00E-03 | * |
| NM_001354567 | NDRG2 | 4,00 | 2,67 | 2,67E-04 | * | NM_001017364 | C4BPB | 2,71 | 3,10 | 6,00E-03 | * |
| NM_001354568 | NDRG2 | 3,99 | 2,66 | 2,67E-04 | * | NM_007278 | GABARAP | 0,91 | 5,76 | 6,00E-03 | * |
| NM_001354569 | NDRG2 | 3,99 | 2,66 | 2,67E-04 | * | NM_014737 | RASSF2 | 3,69 | 2,18 | 6,23E-03 | * |
| NM_001354570 | NDRG2 | 4,00 | 2,67 | 2,67E-04 | * | NM_170774 | RASSF2 | 3,68 | 2,17 | 6,30E-03 | * |
| NM_016250 | NDRG2 | 3,99 | 2,66 | 2,67E-04 | * | NM_152227 | SNX5 | 0,83 | 6,65 | 6,45E-03 | * |
| NM 201535 | NDRG2 | 4,00 | 2,67 | 2,67E-04 | * | NM_014426 | SNX5 | 0,82 | 6,65 | 6,73E-03 | * |
| NM_201536 | NDRG2 | 3,99 | 2,66 | 2,67E-04 | * | NM_001308059 | STARD4 | 3,46 | 2,31 | 6,73E-03 | * |
| NM_201537 | NDRG2 | 3,99 | 2,66 | 2,67E-04 | * | NM_001017365 | C4BPB | 2,68 | 3,03 | 6,86E-03 | * |
| NM_201538 | NDRG2 | 3,99 | 2,66 | 2,67E-04 | * | NM_001017366 | C4BPB | 2,68 | 3,03 | 6,86E-03 | * |
| NM_201539 | NDRG2 | 3,99 | 2,66 | 2,67E-04 | * | NM_152357 | ZNF440 | 2,85 | 2,22 | 6,89E-03 | * |
| NM_201540 | NDRG2 | 4,00 | 2,67 | 2,67E-04 | * | NM_021005 | NR2F2 | 3,55 | 1,20 | 6,94E-03 | # |
| NM_201541 | NDRG2 | 3,99 | 2,66 | 2,67E-04 | * | NM_001282454 | SNX5 | 0,86 | 6,61 | 6,99E-03 | * |
| NM_001329678 | PAXX | 1,37 | 4,84 | 2,67E-04 | * | NM_001265 | CDX2 | 2,11 | 3,70 | 7,07E-03 | * |
| NM_183241 | PAXX | 1,37 | 4,84 | 2,67E-04 | * | NM_001354700 | CDX2 | 2,11 | 3,70 | 7,07E-03 | * |
| NM_002461 | MVD | 2,16 | 5,39 | 2,82E-04 | # | NM_012339 | TSPAN15 | 2,81 | 3,47 | 7,09E-03 | * |
| NM_001320329 | NDRG2 | 4,01 | 2,73 | 2,84E-04 | * | NM_001281717 | UBB | 0,78 | 7,99 | 7,28E-03 | * |
| NM_001318337 | FRMD4A | 2,30 | 2,98 | 3,12E-04 | * | NM_001281716 | UBB | 0,78 | 7,99 | 7,34E-03 | * |
| NM_001318336 | FRMD4A | 2,30 | 2,98 | 3,12E-04 | * | NM_001281718 | UBB | 0,78 | 7,99 | 7,34E-03 | * |
| NM_018027 | FRMD4A | 2,30 | 2,98 | 3,12E-04 | * | NM_018955 | UBB | 0,78 | 7,99 | 7,34E-03 | * |
| NM_001318338 | FRMD4A | 2,30 | 3,07 | 3,35E-04 | * | NM_001281719 | UBB | 0,78 | 7,99 | 7,35E-03 | * |
| NM_001318398 | RASGRP2 | 1,10 | 5,28 | 3,56E-04 | * | NM_001281720 | UBB | 0,78 | 7,99 | 7,37E-03 | * |
| NM_001098671 | RASGRP2 | 1,10 | 5,28 | 3,61E-04 | * | NM_001253913 | TBC1D1 | 0,98 | 4,30 | 7,38E-03 | * |
| NM_001098670 | RASGRP2 | 1,10 | 5,28 | 3,68E-04 | * | NM_001195218 | DCXR | 1,08 | 5,02 | 7,41E-03 | * |
| NM_153819 | RASGRP2 | 1,10 | 5,28 | 3,68E-04 | * | NM_016286 | DCXR | 1,08 | 5,02 | 7,41E-03 | * |
| NM_001804 | CDX1 | 7,38 | 1,36 | 3,80E-04 | * | NM_014059 | RGCC | 0,98 | 6,30 | 7,44E-03 | * |
| NM_004092 | ECHS1 | 1,00 | 7,54 | 4,17E-04 | * | NM_001253914 | TBC1D1 | 0,97 | 4,30 | 7,67E-03 | * |
| NM_001001973 | ATP5F1C | 0,70 | 8,07 | 4,79E-04 | * | NM_001351263 | TSPAN15 | 2,79 | 3,44 | 7,73E-03 | * |
| NM_001320886 | ATPSF1C | 0,70 | 8,07 | 4,79E-04 | * | NM_001358511 | PRDX5 | 0,81 | 6,12 | 7,81E-03 | * |
| NM_005174 | ATP5F1C | 0,70 | 8,07 | 4,80E-04 | * | NM_001358516 | PRDX5 | 0,81 | 6,12 | 7,81E-03 | * |
| NM_001287751 | FDFT1 | 2,39 | 6,65 | 5,45E-04 | * | NM_012094 | PRDX5 | 0,81 | 6,12 | 7,81E-03 | * |
| NM_001287756 | FDFT1 | 2,39 | 6,46 | 5,45E-04 | * | NM_181651 | PRDX5 | 0,81 | 6,12 | 7,81E-03 | * |
| NM_005348 | HSP90AA1 | 0,90 | 9,58 | 5,48E-04 | * | NM_001289414 | ANAPC11 | 0,78 | 6,63 | 7,82E-03 | * |
| NM_001444 | FABPS | 1,22 | 6,43 | 5,56E-04 | * | NM_001687 | ATP5F1D | 0,88 | 6,81 | 7,97E-03 | * |
| NM_001017963 | HSP90AA1 | 0,89 | 9,58 | 5,56E-04 | * | NM_004537 | NAP1L1 | 0,76 | 6,66 | 8,04E-03 | * |
| NM_001346590 | INSIG1 | 1,95 | 4,90 | 5,56E-04 | # | NM_006755 | TALDO1 | 0,72 | 8,30 | 8,07E-03 | * |
| NM_001346591 | INSIG1 | 1,95 | 4,90 | 5,56E-04 | # | NM_001289986 | TIPIN | 0,87 | 5,84 | 8,09E-03 | * |
| NM_001346592 | INSIG1 | 1,95 | 4,90 | 5,56E-04 | # | NM_017858 | TIPIN | 0,87 | 5,84 | 8,09E-03 | * |
| NM_001346594 | INSIG1 | 1,95 | 4,90 | 5,56E-04 | # | NM_001293320 | C8orf59 | 0,80 | 5,24 | 8,16E-03 | * |
| NM_005542 | INSIG1 | 1,95 | 4,90 | 5,56E-04 | # | NM_015987 | HEBP1 | 1,95 | 2,58 | 8,16E-03 | * |
| NM_001346593 | INSIG1 | 1,95 | 4,82 | 5,63E-04 | # | NM_032601 | MCEE | 1,83 | 3,23 | 8,18E-03 | * |
| NM_198337 | INSIG1 | 1,95 | 4,82 | 5,63E-04 | # | NM_001260512 | MGST1 | 2,40 | 3,91 | 8,18E-03 | * |
| NM_001287749 | FDFT1 | 2,38 | 6,69 | 5,65E-04 | * | NM_145791 | MGST1 | 2,40 | 3,90 | 8,24E-03 | * |
| NM_001287750 | FDFT1 | 2,38 | 6,69 | 5,65E-04 | * | NM_145792 | MGST1 | 2,40 | 3,90 | 8,24E-03 | * |
| NM_001287748 | FDFT1 | 2,38 | 6,69 | 5,76E-04 | * | NM_003129 | SQLE | 1,95 | 5,82 | 8,24E-03 | # |
| NM_198336 | INSIG1 | 1,95 | 4,77 | 5,77E-04 | # | NM_030579 | CYB5B | 0,93 | 6,19 | 8,26E-03 | * |
| NM_001287747 | FDFT1 | 2,37 | 6,70 | 5,81E-04 | * | NM_001260511 | MGST1 | 2,40 | 3,90 | 8,27E-03 | * |
| NM_001145661 | GATA2 | 1,98 | 5,94 | 5,81E-04 | * | NM_020300 | MGST1 | 2,40 | 3,90 | 8,27E-03 | * |
| NM_001145662 | GATA2 | 1,98 | 5,93 | 5,82E-04 | * | NM_145764 | MGST1 | 2,40 | 3,90 | 8,27E-03 | * |
| NM_032638 | GATA2 | 1,98 | 5,93 | 5,86E-04 | * | NM_005659 | UFD1 | 0,78 | 5,84 | 8,27E-03 | * |
| NM_004462 | FDFT1 | 2,37 | 6,70 | 6,06E-04 | * | NM_001035247 | UFD1 | 0,78 | 5,84 | 8,27E-03 | * |
| NM_001287745 | FDFT1 | 2,37 | 6,70 | 6,12E-04 | * | NM_001099673 | C8orf59 | 0,80 | 5,24 | 8,72E-03 | * |
| NM_001287744 | FDFT1 | 2,36 | 6,69 | 6,25E-04 | * | NM_001099672 | C8orf59 | 0,80 | 5,25 | 8,82E-03 | * |
| NM_001206836 | RAB11A | 0,72 | 7,21 | 6,32E-04 | * | NM_152550 | SH3RF2 | 2,93 | 1,95 | 8,87E-03 | * |
| NM_004663 | RAB11A | 0,72 | 7,21 | 6,32E-04 | * | NM_005990 | STK10 | 1,26 | 4,78 | 8,90E-03 | * |
| NM_001287742 | FDFT1 | 2,36 | 6,70 | 6,40E-04 | * | NM_002475 | MYL6B | 1,61 | 3,94 | 9,00E-03 | * |
| NM_001287743 | FDFT1 | 2,36 | 6,70 | 6,40E-04 | * | NM_001099670 | C8orf59 | 0,80 | 5,25 | 9,03E-03 | * |
| NM_004547 | NDUFB4 | 0,82 | 6,04 | 6,84E-04 | * | NM_001099671 | C8orf59 | 0,80 | 5,25 | 9,03E-03 | * |
| NM_002013 | FKBP3 | 0,85 | 6,12 | 6,90E-04 | * | NM_012203 | GRHPR | 1,33 | 4,37 | 9,03E-03 | * |
| NM_016619 | PLAC8 | 1,96 | 4,51 | 6,90E-04 | * | NM_001304512 | HSD17B7 | 3,44 | 2,49 | 9,10E-03 | * |
| NM_014573 | TMEM97 | 1,60 | 4,87 | 7,06E-04 | * | NM_001304513 | HSD17B7 | 3,44 | 2,49 | 9,10E-03 | * |
| NM_001168331 | NDUFB4 | 0,81 | 6,05 | 7,12E-04 | * | NM_001253915 | TBC1D1 | 1,02 | 3,95 | 9,15E-03 | * |
| NM_001130716 | PLAC8 | 1,95 | 4,51 | 7,12E-04 | * | NM_001199629 | MYL6B | 1,60 | 3,94 | 9,23E-03 | * |
| NM_004525 | LRP2 | 2,56 | 5,64 | 7,43E-04 | * | NM_001278546 | NR6A1 | 1,41 | 3,54 | 9,25E-03 | * |
| NM_001002249 | ANAPC11 | 1,03 | 6,33 | 7,94E-04 | * | NM_001489 | NR6A1 | 1,41 | 3,54 | 9,25E-03 | * |
| NM_001289420 | ANAPC11 | 1,03 | 6,33 | 8,11E-04 | * | NM_033334 | NR6A1 | 1,41 | 3,54 | 9,25E-03 | * |
| NM_001002245 | ANAPC11 | 1,03 | 6,33 | 8,18E-04 | * | NM 033427 | CTTNBP2 | 2,59 | 2,43 | 9,44E-03 | * |
| NM_001002246 | ANAPC11 | 1,03 | 6,33 | 8,18E-04 | * | NM_001001975 | ATP5F1D | 0,86 | 6,77 | 9,48E-03 | * |
| NM_001002247 | ANAPC11 | 1,03 | 6,33 | 8,18E-04 | * | NM_005809 | PRDX2 | 0,95 | 7,02 | 9,75E-03 | * |
| NM_001002248 | ANAPC11 | 1,03 | 6,33 | 8,18E-04 | * | NM_138499 | PWWP2B | 2,66 | 2,99 | 9,86E-03 | * |
| NM_001289415 | ANAPC11 | 1,03 | 6,33 | 8,18E-04 | * | NM_181652 | PRDX5 | 0,79 | 6,04 | 9,89E-03 | * |
| NM_001289416 | ANAPC11 | 1,03 | 6,32 | 8,18E-04 | * | NM_000146 | FTL | 0,86 | 8,10 | 1,00E-02 | * |
| NM_001289417 | ANAPC11 | 1,03 | 6,33 | 8,18E-04 | * | NM_017819 | TRMT10C | 0,75 | 6,40 | 1,00E-02 | * |
| NM_001289418 | ANAPC11 | 1,03 | 6,33 | 8,18E-04 | * | NM_018465 | PLGRKT | 1,09 | 3,84 | 1,01E-02 | * |
| NM_001289419 | ANAPC11 | 1,03 | 6,33 | 8,18E-04 | * | NM_001731 | BTG1 | 1,11 | 4,44 | 1,01E-02 | * |
| NM_016476 | ANAPC11 | 1,03 | 6,33 | 8,18E-04 | * | NM_001308056 | STARD4 | 3,48 | 2,30 | 1,02E-02 | * |
| NM_001002244 | ANAPC11 | 1,02 | 6,33 | 8,40E-04 | * | NM_001308061 | STARD4 | 3,48 | 2,30 | 1,02E-02 | * |
| NM_001320454 | C1orf61 | 2,36 | 2,47 | 9,95E-04 | * | NM_139164 | STARD4 | 3,48 | 2,30 | 1,02E-02 | * |
| NM_001320455 | C1orf61 | 2,39 | 2,39 | 1,04E-03 | * | NM_004280 | EEF1E1 | 0,82 | 4,85 | 1,03E-02 | * |
| NM_001914 | CYBSA | 1,37 | 4,19 | 1,04E-03 | * | NM_000903 | NQO1 | 1,69 | 4,44 | 1,03E-02 | * |
| NM_148923 | CYBSA | 1,37 | 4,19 | 1,05E-03 | * | NM_001025433 | NQO1 | 1,69 | 4,44 | 1,03E-02 | * |
| NM_001190807 | CYBSA | 1,37 | 4,19 | 1,05E-03 | * | NM_001025434 | NQO1 | 1,69 | 4,44 | 1,03E-02 | * |
| NM_016371 | HSD17B7 | 3,47 | 3,70 | 1,10E-03 | * | NM_001308060 | STARD4 | 3,51 | 2,30 | 1,03E-02 | * |
| NM_001064 | TKT | 1,18 | 5,93 | 1,12E-03 | * | NM_001127218 | POLD2 | 0,97 | 5,66 | 1,04E-02 | * |
| NM_001258028 | TKT | 1,18 | 5,93 | 1,12E-03 | * | NM_001256879 | POLD2 | 0,97 | 5,66 | 1,04E-02 | * |
| NM_001130715 | PLAC8 | 1,94 | 4,46 | 1,13E-03 | * | NM_006230 | POLD2 | 0,97 | 5,66 | 1,04E-02 | * |
| NM_005192 | CDKN3 | 1,18 | 4,37 | 1,15E-03 | * | NM_001286137 | NQO1 | 1,69 | 4,43 | 1,04E-02 | * |
| | | | **(P = Preferred):** * = **at least one of these genes** | | | | | | | | |

**Table A2**

| **YMA/rba-YMA non-coding NR_ transcripts Qvalue < 0.01** | | | | | | | | | | | |
|---|---|---|---|---|---|---|---|---|---|---|---|
| **refSeq** | **gene name** | **Log FC** | **Log CPM** | **FDR** | **P** | **refSeq** | **gene name** | **Log FC** | **Log CPM** | **FDR** | **P** |
| NR_144397 | HSD17B 1 | 3,44 | 5,02 | 2,30E-07 | * | NR_003086 | HSD17B7P2 | 3,72 | 1,38 | 7,07E-04 | * |
| NR_144402 | LOC 108783 654 | 3,22 | 4,77 | 6,90E-07 | * | NR_027709 | IDI2-AS1 | 1,99 | 3,98 | 8,92E-04 | # |
| NR_049759 | IFITM3 | 3,00 | 5,72 | 4,51E-06 | * | NR_109925 | MIR548XHG | 2,77 | 2,41 | 9,49E-04 | # |
| NR_134300 | IDI1 | 1,85 | 6,63 | 4,73E-05 | * | NR_135261 | C1orf61 | 2,38 | 2,29 | 1,16E-03 | * |
| NR 026598 | NENF | 1,64 | 5,51 | 6,12E-05 | * | NR 047580 | TKT | 1,10 | 5,91 | 1,54E-03 | * |
| NR_145426 | RUSC1-AS1 | 1,66 | 5,22 | 7,11E-05 | * | NR_037669 | GGCT | 1,23 | 4,45 | 2,41E-03 | * |
| NR_117089 | CD24 | 3,64 | 4,50 | 1,06E-04 | * | NR_037166 | SOD2-OT1 | 1,68 | 3,17 | 2,79E-03 | * |
| NR_038118 | MMAB | 2,51 | 3,98 | 1,39E-04 | * | NR_024611 | HINT1 | 1,34 | 6,04 | 5,99E-03 | * |
| NR_046298 | SLC25A1 | 2,04 | 6,18 | 1,62E-04 | * | NR_147091 | TSPAN15 | 2,72 | 3,44 | 8,11E-03 | * |
| NR_109934 | PCLAF | 2,71 | 3,18 | 2,12E-04 | * | NR_048547 | MGST1 | 2,35 | 3,89 | 8,83E-03 | * |
| NR_138074 | PAXX | 1,31 | 4,82 | 3,47E-04 | * | **(P = Preferred): * = at least one of these genes** | | | | | |

The genes and gene transcripts in Table B have been found to have an elevated level of expression that is statistically significantly associated with trophoblasts having an advanced chronological/biological reproductive age and are listed in Table B in order of statistical significance expressed by false discovery rate (FDR) adjusted p-value (FDR<0.05). The expression level of any one or more of the genes listed in Table B can, therefore, be used as a biomarker to identify blastocysts having a reduced likelihood of implantation success.

Table B is made up of Table B1, referred to as "Table B protein coding transcripts", and Table B2, referred to as "Table B non-coding transcripts". Table B1 and Table B2 report the more highly significant (FDR<0.01) AMA/rba-AMA transcripts, including 230 (94 genes) coding and 19 non-coding transcripts, respectively.

**Table B1**

| **AMA/rba-AMA present in both analyses (i.e. with and without non-coding NR_) and Qvalue<0.01** | | | | | | | | | | | |
|---|---|---|---|---|---|---|---|---|---|---|---|
| **refSeq** | **gene name** | **log FC** | **log CPM** | **FDR** | **P** | **refSeq** | **gene name** | **log FC** | **log CPM** | **FDR** | **P** |
| NM_001188 | BAK1 | -2,48 | 5,15 | 3,17E-12 | # | NM_001142573 | IMPDH1 | -1,03 | 6,77 | 2,50E-03 | * |
| NM_001102420 | ZFAND5 | -1,63 | 6,50 | 2,95E-11 | * | NM_001142574 | IMPDH1 | -1,03 | 6,77 | 2,50E-03 | * |
| NM_001102421 | ZFAND5 | -1,63 | 6,50 | 2,95E-11 | * | NM_001142575 | IMPDH1 | -1,03 | 6,77 | 2,50E-03 | * |
| NM_006007 | ZFAND5 | -1,63 | 6,50 | 2,95E-11 | * | NM_183243 | IMPDH1 | -1,03 | 6,77 | 2,50E-03 | * |
| NM_001278243 | ZFAND5 | -1,62 | 6,44 | 7,60E-11 | * | NM_001145443 | PFKFB3 | -3,01 | 3,23 | 2,50E-03 | * |
| NM_001278245 | ZFAND5 | -1,62 | 6,44 | 7,60E-11 | * | NM_001282630 | PFKFB3 | -3,01 | 3,23 | 2,50E-03 | * |
| NM_001278244 | ZFAND5 | -1,60 | 6,39 | 2,14E-10 | * | NM_001323016 | PFKFB3 | -3,01 | 3,23 | 2,50E-03 | * |
| NM_001197 | BIK | -3,41 | 4,51 | 1,02E-07 | * | NM_001136218 | TMEM51 | -1,79 | 3,38 | 2,52E-03 | * |
| NM_001198773 | PI4KB | -1,67 | 4,63 | 5,94E-07 | * | NM_001319665 | TMEM51 | -1,79 | 3,38 | 2,52E-03 | * |
| NM_001198774 | PI4KB | -1,67 | 4,62 | 5,94E-07 | * | NM_001136217 | TMEM51 | -1,79 | 3,38 | 2,56E-03 | * |
| NM_001330721 | PI4KB | -1,67 | 4,63 | 5,94E-07 | * | NM 018022 | TMEM51 | -1,79 | 3,38 | 2,56E-03 | * |
| NM_002651 | PI4KB | -1,67 | 4,63 | 5,94E-07 | * | NM_000602 | SERPINE1 | -2,99 | 3,35 | 2,57E-03 | * |
| NM_024597 | MAP7D3 | -2,12 | 3,80 | 1,29E-06 | * | NM_001136216 | TMEM51 | -1,79 | 3,39 | 2,61E-03 | * |
| NM_001173516 | MAP7D3 | -2,12 | 3,80 | 1,29E-06 | * | NM 021137 | TNFAIP1 | -1,00 | 4,08 | 2,63E-03 | * |
| NM_001173517 | MAP7D3 | -2,09 | 3,73 | 2,36E-06 | * | NM_058219 | EXOSC6 | -1,34 | 4,64 | 2,75E-03 | * |
| NM_001198775 | PI4KB | -1,62 | 4,32 | 5,28E-06 | * | NM_147161 | ACOT11 | -1,75 | 7,13 | 3,01E-03 | * |
| NM_001031717 | CRELD1 | -2,83 | 3,15 | 1,03E-05 | * | NM_001308195 | SIMC1 | -1,66 | 3,74 | 3,28E-03 | * |
| NM_001077415 | CRELD1 | -2,85 | 3,14 | 1,36E-05 | * | NM_001308196 | SIMC1 | -1,66 | 3,74 | 3,28E-03 | * |
| NM_015513 | CRELD1 | -2,84 | 3,14 | 1,36E-05 | * | NM_001172699 | PPARGC1B | -1,18 | 4,16 | 3,39E-03 | * |
| NM_022783 | DEPTOR | -2,44 | 3,82 | 1,36E-05 | * | NM_133263 | PPARGC1B | -1,18 | 4,16 | 3,42E-03 | * |
| NM_152405 | JMY | -2,60 | 3,95 | 1,55E-05 | * | NM_001172698 | PPARGC1B | -1,18 | 4,16 | 3,43E-03 | * |
| NM_001243794 | CHST12 | -1,88 | 4,39 | 1,60E-05 | * | NM 001171192 | GDPD2 | -1,39 | 4,72 | 3,46E-03 | * |
| NM_001243795 | CHST12 | -1,88 | 4,39 | 1,60E-05 | * | NM_017711 | GDPD2 | -1,39 | 4,72 | 3,46E-03 | * |
| NM_018641 | CHST12 | -1,88 | 4,39 | 1,60E-05 | * | NM_001171193 | GDPD2 | -1,39 | 4,72 | 3,58E-03 | * |
| NM_003811 | TNFSF9 | -3,32 | 2,68 | 1,64E-05 | * | NM_001171191 | GDPD2 | -1,39 | 4,71 | 3,60E-03 | * |
| NM_001204450 | CCPG1 | -2,24 | 5,69 | 2,56E-05 | * | NM_001204887 | RAB43 | -1,95 | 3,39 | 3,60E-03 | * |
| NM_020739 | CCPG1 | -2,25 | 5,81 | 3,06E-05 | * | NM_001204888 | RAB43 | -1,95 | 3,39 | 3,60E-03 | * |
| NM_004748 | CCPG1 | -2,24 | 5,81 | 3,26E-05 | * | NM_198490 | RAB43 | -1,95 | 3,39 | 3,60E-03 | * |
| NM_001204451 | CCPG1 | -2,22 | 5,26 | 3,76E-05 | * | NM_001204883 | RAB43 | -1,95 | 3,39 | 3,63E-03 | * |
| NM_006158 | NEFL | -4,47 | 2,51 | 7,33E-05 | * | NM_001204884 | RAB43 | -1,95 | 3,39 | 3,63E-03 | * |
| NM_001142805 | SLC6A8 | -1,62 | 7,03 | 8,11E-05 | * | NM_001204885 | RAB43 | -1,95 | 3,39 | 3,63E-03 | * |
| NM_005629 | SLC6A8 | -1,62 | 7,03 | 8,11E-05 | * | NM_001204886 | RAB43 | -1,95 | 3,39 | 3,63E-03 | * |
| NM_001142806 | SLC6A8 | -1,62 | 7,02 | 8,49E-05 | * | NM_015033 | FNBP1 | -1,18 | 3,88 | 3,77E-03 | * |
| NM_001199198 | TBC1D23 | -1,36 | 5,52 | 9,24E-05 | * | NM_018003 | UACA | -1,34 | 6,17 | 3,97E-03 | * |
| NM_018309 | TBC1D23 | -1,36 | 5,52 | 9,24E-05 | * | NM_001008224 | UACA | -1,33 | 6,17 | 3,99E-03 | * |
| NM_001284497 | FAM234A | -2,23 | 3,98 | 1,09E-04 | * | NM_001242312 | FAM124A | -3,31 | 1,98 | 4,14E-03 | * |
| NM_032039 | FAM234A | -2,23 | 3,98 | 1,09E-04 | * | NM_145019 | FAM124A | -3,31 | 1,98 | 4,14E-03 | * |
| NM_178043 | LARP1B | -1,43 | 4,49 | 1,15E-04 | * | NM_001001479 | SLC35E4 | -1,58 | 5,58 | 4,21E-03 | * |
| NM_001283012 | DEPTOR | -2,31 | 3,52 | 1,16E-04 | * | NM_001294344 | MTMR12 | -1,55 | 3,88 | 4,40E-03 | * |
| NM_001350531 | LARP1B | -1,39 | 4,53 | 1,55E-04 | * | NM_001040446 | MTMR12 | -1,56 | 3,91 | 4,43E-03 | * |
| NM_001256856 | BTRC | -1,36 | 4, 24 | 1,83E-04 | * | NM_001294343 | MTMR12 | -1,56 | 3,91 | 4,43E-03 | * |
| NM_033637 | BTRC | -1,36 | 4,24 | 1,83E-04 | * | NM_001105214 | ASH2L | -1,26 | 4,50 | 4,50E-03 | * |
| NM_018078 | LARP1B | -1,39 | 4,54 | 1,83E-04 | * | NM_001261832 | ASH2L | -1,26 | 4,50 | 4,51E-03 | * |
| NM_003939 | BTRC | -1,36 | 4,24 | 1,84E-04 | * | NM_004674 | ASH2L | -1,26 | 4,50 | 4,56E-03 | * |
| NM_024954 | UBTD1 | -2,43 | 3,17 | 2,22E-04 | * | NM_006675 | TSPAN9 | -2,30 | 1,86 | 4,60E-03 | * |
| NM_018184 | ARL8B | -0,96 | 5,45 | 3,15E-04 | * | NM_002444 | MSN | -1,65 | 4,26 | 4,67E-03 | * |
| NM_000676 | ADORA2B | -2,12 | 2,94 | 3,22E-04 | * | NM_198567 | SIMC1 | -1,60 | 3,11 | 4,67E-03 | * |
| NM_005257 | GATA6 | -1,06 | 6,41 | 3,35E-04 | * | NM_001282272 | ASH2L | -1,25 | 4,50 | 4,77E-03 | * |
| NM_032312 | YIPF4 | -1,35 | 5,22 | 3,68E-04 | * | NM_139015 | SPPL3 | -1,14 | 4,25 | 5,32E-03 | * |
| NM_012083 | FRAT2 | -1,14 | 6,97 | 3,76E-04 | * | NM_014637 | MTFR1 | -0,95 | 3,78 | 5,42E-03 | * |
| NM_176895 | PLPP1 | -1,24 | 5,09 | 4,01E-04 | * | NM_001145838 | MTFR1 | -0,95 | 3,77 | 5,57E-03 | * |
| NM_001206916 | CACNB3 | -3,45 | 1,89 | 4,17E-04 | * | NM_001105562 | UBE4B | -1,19 | 4,20 | 6,08E-03 | * |
| NM_003711 | PLPP1 | -1,24 | 5,09 | 4,38E-04 | * | NM_005376 | MYCL | -2,40 | 1,82 | 6,11E-03 | * |
| NM_014330 | PPP1R15A | -1,85 | 3,92 | 4,43E-04 | * | NM_006048 | UBE4B | -1,19 | 4,20 | 6,16E-03 | * |
| NM_000725 | CACNB3 | -3,42 | 1,86 | 5,03E-04 | * | NM_002087 | GRN | -1,62 | 5,28 | 6,35E-03 | * |
| NM_001206917 | CACNB3 | -3,42 | 1,86 | 5,03E-04 | * | NM_001348091 | F11R | -0,90 | 6,87 | 6,40E-03 | * |
| NM_001206915 | CACNB3 | -3,42 | 1,86 | 5,05E-04 | * | NM_016946 | F11R | -0,90 | 6,87 | 6,40E-03 | * |
| NM_001193532 | RAB42 | -2,24 | 2,91 | 5,05E-04 | * | NM_001111045 | CCNA1 | -2,53 | 3,76 | 6,49E-03 | * |
| NM_001352501 | RCBTB1 | -1,47 | 4,91 | 5,44E-04 | * | NM_003914 | CCNA1 | -2,53 | 3,76 | 6,49E-03 | * |
| NM_001352502 | RCBTB1 | -1,47 | 4,91 | 5,44E-04 | * | NM_001111046 | CCNA1 | -2,53 | 3,74 | 6,61E-03 | * |
| NM_021220 | OVOL2 | -1,46 | 4,28 | 5,45E-04 | * | NM_001111047 | CCNA1 | -2,53 | 3,74 | 6,62E-03 | * |
| NM_001352500 | RCBTB1 | -1,47 | 4,91 | 5,45E-04 | * | NM_001319054 | GEMIN7 | -0,98 | 4,81 | 7,48E-03 | * |
| NM_001352503 | RCBTB1 | -1,47 | 4,91 | 5,45E-04 | * | NM_004839 | HOMER2 | -1,42 | 4,17 | 7,51E-03 | * |
| NM_018191 | RCBTB1 | -1,47 | 4,91 | 5,45E-04 | * | NM_199330 | HOMER2 | -1,42 | 4,17 | 7,51E-03 | * |
| NM_001174156 | SAMD8 | -1,53 | 3,77 | 5,45E-04 | * | NM_001286679 | LARP6 | -2,05 | 2,78 | 7,70E-03 | * |
| NM_144660 | SAMD8 | -1,53 | 3,77 | 5,45E-04 | * | NM_001127895 | CHST8 | -1,62 | 3,23 | 7,81E-03 | * |
| NM_001352506 | RCBTB1 | -1,47 | 4,91 | 5,48E-04 | * | NM_001127896 | CHST8 | -1,63 | 3,23 | 7,81E-03 | * |
| NM_001303461 | OVOL2 | -1,46 | 4,27 | 5,56E-04 | * | NM_001135649 | FOXI3 | -1,09 | 4,97 | 8,07E-03 | * |
| NM_001303462 | OVOL2 | -1,45 | 4,23 | 5,86E-04 | * | NM_052853 | ADCK2 | -1,20 | 4,99 | 8,10E-03 | * |
| NM_001278604 | LARP1B | -1,41 | 4,91 | 6,15E-04 | * | NM_173565 | RSPH10B | -2,36 | 3,04 | 8,18E-03 | * |
| NM_001291663 | MKRN1 | -1,09 | 5,04 | 6,16E-04 | * | NM 001099697 | RSPH10B2 | -2,36 | 3,04 | 8,18E-03 | * |
| NM_001145125 | MKRN1 | -1,09 | 5,04 | 6,23E-04 | * | NM_001321867 | ZNF600 | -0,86 | 5,21 | 8,18E-03 | * |
| NM_001352504 | RCBTB1 | -1,91 | 3,71 | 6,34E-04 | * | NM_001288771 | C17orf80 | -0,81 | 4,22 | 8,22E-03 | * |
| NM_032239 | LARP1B | -1,41 | 4,92 | 6,42E-04 | * | NM_198457 | ZNF600 | -0,86 | 5,21 | 8,22E-03 | * |
| NM_001243186 | PIM1 | -2,15 | 4,41 | 6,42E-04 | * | NM_001017916 | CYB561 | -1,70 | 3,07 | 8,26E-03 | * |
| NM_002648 | PIM1 | -2,15 | 4,41 | 6,42E-04 | * | NM_001915 | CYB561 | -1,70 | 3,07 | 8,26E-03 | * |
| NM_152304 | RAB42 | -2,24 | 2,85 | 7,00E-04 | * | NM_001017917 | CYB561 | -1,70 | 3,07 | 8,26E-03 | * |
| NM_013446 | MKRN1 | -1,00 | 6,03 | 9,39E-04 | * | NM_001330421 | CYB561 | -1,70 | 3,07 | 8,26E-03 | * |
| NM_001077526 | MTMR14 | -1,61 | 5,36 | 9,79E-04 | * | NM_153252 | BRWD3 | -1,72 | 4,11 | 8,27E-03 | * |
| NM_001077525 | MTMR14 | -1,61 | 5,36 | 1,01E-03 | * | NM_001012659 | ARGFX | -2,23 | 3,72 | 8,34E-03 | * |
| NM_022485 | MTMR14 | -1,61 | 5,34 | 1,01E-03 | * | NM_001007269 | GEMIN7 | -0,97 | 4,83 | 8,38E-03 | * |
| NM_006768 | BRAP | -1,00 | 5,24 | 1,12E-03 | * | NM_012454 | TIAM2 | -1,62 | 2,31 | 8,50E-03 | * |
| NM_080878 | ITLN2 | -3,15 | 2,11 | 1,20E-03 | * | NM_021960 | MCL1 | -0,78 | 5,86 | 8,55E-03 | * |
| NM_001286457 | USP7 | -1,01 | 6,97 | 1,29E-03 | * | NM_001319055 | GEMIN7 | -0,97 | 4,83 | 8,82E-03 | * |
| NM_001286458 | USP7 | -1,01 | 6,97 | 1,29E-03 | * | NM_001321866 | ZNF600 | -0,84 | 5,26 | 8,87E-03 | * |
| NM_003470 | USP7 | -1,01 | 6,97 | 1,29E-03 | * | NM_001168320 | TSPAN9 | -2,30 | 1,84 | 8,98E-03 | * |
| NM_001321858 | USP7 | -1,01 | 6,97 | 1,29E-03 | * | NM_001197320 | MCL1 | -0,78 | 5,82 | 9,03E-03 | * |
| NM_003019 | SFTPD | -3,41 | 1,94 | 1,32E-03 | * | NM_024707 | GEMIN7 | -0,96 | 4,84 | 9,09E-03 | * |
| NM_001184906 | FBXL20 | -1,40 | 5,37 | 1,32E-03 | * | NM_001007270 | GEMIN7 | -0,96 | 4,83 | 9,23E-03 | * |
| NM_032875 | FBXL20 | -1,40 | 5,37 | 1,33E-03 | * | NM_182763 | MCL1 | -0,77 | 5,84 | 9,23E-03 | * |
| NM_031459 | SESN2 | -1,29 | 4,33 | 1,46E-03 | * | NM_015993 | PLLP | -2,43 | 3,72 | 9,44E-03 | # |
| NM_001007561 | IRGQ | -1,80 | 3,60 | 1,48E-03 | * | NM_031308 | EPPK1 | -1,71 | 5,09 | 9,62E-03 | * |
| NM_176782 | FAM151A | -1,76 | 7,98 | 1,63E-03 | * | NM_001308200 | SIMC1 | -1,59 | 2,80 | 9,62E-03 | * |
| NM_152914 | NATD1 | -2,33 | 2,87 | 1,65E-03 | * | NM_022488 | ATG3 | -0,84 | 7,25 | 9,89E-03 | * |
| NM_001012414 | TRIM61 | -1,03 | 5,56 | 1,66E-03 | * | NM_001322383 | ENTPD6 | -0,84 | 4,62 | 9,95E-03 | * |
| NM_001319670 | SPSB2 | -1,90 | 2,92 | 1,70E-03 | * | NM_001278712 | ATG3 | -0,84 | 7,26 | 1,00E-02 | * |
| NM_032641 | SPSB2 | -1,90 | 2,92 | 1,74E-03 | * | NM_001322389 | ENTPD6 | -0,84 | 4,62 | 1,01E-02 | * |
| NM_001146316 | SPSB2 | -1,90 | 2,92 | 1,79E-03 | * | NM_001322398 | ENTPD6 | -0,84 | 4,62 | 1,01E-02 | * |
| NM_002065 | GLUL | -0,98 | 7,09 | 2,21E-03 | * | NM_001322378 | ENTPD6 | -0,84 | 4,62 | 1,01E-02 | * |
| NM_001033044 | GLUL | -0,98 | 7,09 | 2,21E-03 | * | NM_001322395 | ENTPD6 | -0,84 | 4,62 | 1,01E-02 | * |
| NM_001201477 | NETO2 | -1,20 | 3,76 | 2,21E-03 | * | NM_001114089 | ENTPD6 | -0,84 | 4,62 | 1,02E-02 | * |
| NM_018092 | NETO2 | -1,20 | 3,76 | 2,21E-03 | * | NM_001247 | ENTPD6 | -0,84 | 4,62 | 1,02E-02 | * |
| NM_015627 | LDLRAP1 | -1,49 | 3,40 | 2,22E-03 | * | NM_001317941 | ENTPD6 | -0,84 | 4,62 | 1,02E-02 | * |
| NM_001033056 | GLUL | -0,98 | 7,09 | 2,22E-03 | * | NM_001322386 | ENTPD6 | -0,84 | 4,62 | 1,02E-02 | * |
| NM_001007024 | GOSR1 | -0,85 | 5,13 | 2,27E-03 | * | NM_001322391 | ENTPD6 | -0,84 | 4,62 | 1,02E-02 | * |
| NM_001007025 | GOSR1 | -0,85 | 5,13 | 2,27E-03 | * | NM_001322392 | ENTPD6 | -0,84 | 4,62 | 1,02E-02 | * |
| NM_004871 | GOSR1 | -0,85 | 5,13 | 2,27E-03 | * | NM_001322394 | ENTPD6 | -0,84 | 4,62 | 1,02E-02 | * |
| NM_001314063 | PFKFB3 | -3,04 | 3,30 | 2,31E-03 | * | NM_001010883 | FAM102B | -1,72 | 3,45 | 1,02E-02 | * |
| NM_001323017 | PFKFB3 | -3,04 | 3,30 | 2,31E-03 | * | NM_001322393 | ENTPD6 | -0,84 | 4,62 | 1,02E-02 | * |
| NM_004566 | PFKFB3 | -3,04 | 3,30 | 2,31E-03 | * | NM_001322388 | ENTPD6 | -0,84 | 4,62 | 1,03E-02 | * |
| NM_001352505 | RCBTB1 | -2,01 | 3,22 | 2,34E-03 | * | NM_001322396 | ENTPD6 | -0,84 | 4,62 | 1,03E-02 | * |
| NM_001142576 | IMPDH1 | -1,04 | 6,77 | 2,47E-03 | * | NM_001322397 | ENTPD6 | -0,84 | 4,62 | 1,03E-02 | * |
| NM_001304521 | IMPDH1 | -1,04 | 6,77 | 2,47E-03 | * | NM_001322385 | ENTPD6 | -0,84 | 4,62 | 1,03E-02 | * |
| NM_000883 | IMPDH1 | -1,03 | 6,77 | 2,50E-03 | * | NM_001322387 | ENTPD6 | -0,84 | 4,62 | 1,03E-02 | * |
| NM_001102605 | IMPDH1 | -1,03 | 6,77 | 2,50E-03 | * | NM_001322390 | ENTPD6 | -0,84 | 4,62 | 1,04E-02 | * |
| | | | | | | (P = Preferred): * = at least one of these genes | | | | | |

**Table B2.**

| **AMA/rba-AMA non-coding NR_ transcripts Qvalue < 0.01** | | | | | | | | | | | |
|---|---|---|---|---|---|---|---|---|---|---|---|
| **refSeq** | **gene name** | **log FC** | **log CPM** | **FDR** | **P** | **refSeq** | **gene name** | **log FC** | **log CPM** | **FDR** | **P** |
| NR_126449 | LINC01224 | -3,37 | 3,23 | 3,96E-08 | * | NR_135082 | TMEM51 | -1,83 | 3,39 | 1,66E-03 | * |
| NR_028346 | TRIM53AP | -6,06 | 1,74 | 1,54E-06 | * | NR_136554 | PFKFB3 | -3,05 | 3,23 | 2,04E-03 | * |
| NR_037923 | DNAAF4-CCPG1 | -2,22 | 5,76 | 1,88E-05 | * | NR_131772 | SIMC1 | -1,65 | 3,12 | 2,74E-03 | * |
| NR_110695 | LOC 101928372 | -3,35 | 1,97 | 3,29E-05 | * | NR_134887 | GEMIN7-AS1 | -1,09 | 4,10 | 3,76E-03 | * |
| NR_146772 | LARP1B | -1,43 | 4,54 | 8,94E-05 | * | NR_024451 | KDM7A-DT | -2,96 | 1,45 | 3,80E-03 | * |
| NR_103485 | PLPP1 | -1,28 | 5,11 | 2,97E-04 | * | NR_136655 | SNAP47 | -0,85 | 5,14 | 4,47E-03 | * |
| NR_148016 | RCBTB1 | -1,52 | 4,93 | 2,97E-04 | * | NR_033959 | SMG1P7 | -2,96 | 2,63 | 5,87E-03 | * |
| NR_117084 | MKRN1 | -1,06 | 5,89 | 3,47E-04 | * | NR_024071 | PLCD1 | -1,72 | 3,54 | 6,38E-03 | * |
| NR_135826 | USP7 | -1,05 | 6,97 | 5,70E-04 | * | NR_038433 | LINC00330 | -2,23 | 2,26 | 6,76E-03 | * |
| NR_104317 | FAM234A | -2,09 | 3,04 | 9,35E-04 | * | **(P = Preferred): * = at least one of these genes** | | | | | |

The biomarkers in Table C have a statistically significantly increased level of expression in cells that have a high likelihood of implantation success compared with those that have a lower or reduced likelihood of implantation success. Table C includes genes and gene transcripts with overall higher FDRs (approximately between 0.01 and 0.05, with some exemptions) and overall lower logFC in relation to Table A.

**Table C**

| **rba-YMA biomarkers with q-value above 0.01 or CPM<2 or present in one of the analyses (with & without non-coding transcripts)** | | | | | | | | | | | |
|---|---|---|---|---|---|---|---|---|---|---|---|
| **refSeq** | **gene name** | **log FC** | **log CPM** | **FDR** | **P** | **refSeq** | **gene name** | **log FC** | **log CPM** | **FDR** | **P** |
| NM_000126 | ETFA | 0,77 | 6,46 | 1,91E-02 | * | NM_001326381 | HEBP2 | 0,64 | 5,07 | 4,38E-02 | * |
| NM_000224 | KRT18 | 0,78 | 10,33 | 2,65E-02 | * | NM_001329722 | TMEM54 | 1,00 | 5,99 | 2,85E-02 | * |
| NM_000405 | GM2A | 1,56 | 4,15 | 1,92E-02 | * | NM_001329723 | TMEM54 | 1,00 | 5,98 | 2,85E-02 | * |
| NM_000560 | CD53 | 1,02 | 6,33 | 2,85E-02 | * | NM_001329724 | TMEM54 | 1,00 | 5,98 | 2,85E-02 | * |
| NM_000847 | GSTA3 | 2,17 | 2,01 | 4,49E-02 | * | NM_001329725 | TMEM54 | 1,00 | 5,98 | 2,89E-02 | * |
| NM_001002027 | ATP5MC1 | 0,75 | 5,85 | 2,04E-02 | * | NM_001330156 | PID1 | 4,26 | 0,78 | 2,38E-03 | * |
| NM_001002292 | WLS | 3,26 | 1,91 | 2,78E-02 | * | NM_001330157 | PID1 | 4,40 | 0,79 | 2,52E-03 | * |
| NM_001003395 | TPD52L1 | 3,22 | 2,26 | 2,89E-02 | * | NM_001330158 | PID1 | 4,31 | 0,82 | 2,22E-03 | * |
| NM_001003396 | TPD52L1 | 3,21 | 2,27 | 2,97E-02 | * | NM_001330231 | NAP1L1 | 0,74 | 6,60 | 1,10E-02 | * |
| NM_001003397 | TPD52L1 | 3,21 | 2,27 | 2,97E-02 | * | NM_001330232 | NAP1L1 | 0,75 | 6,59 | 1,08E-02 | * |
| NM_001003794 | MGLL | 2,14 | 2,87 | 1,92E-02 | * | NM_001330626 | CAST | 1,30 | 4,82 | 2,80E-02 | * |
| NM_001011546 | DSTN | 0,72 | 5,96 | 1,71E-02 | * | NM_001330627 | CAST | 1,30 | 4,82 | 2,80E-02 | * |
| NM_001018009 | SH3BPS | 4,12 | 0,73 | 1,45E-02 | * | NM_001330628 | CAST | 1,30 | 4,82 | 2,81E-02 | * |
| NM_001024948 | FNBP1L | 0,76 | 5,63 | 2,89E-02 | * | NM_001330629 | CAST | 1,30 | 4,82 | 2,81E-02 | * |
| NM_001024956 | SCSD | 1,90 | 2,68 | 3,64E-02 | # | NM_001330630 | CAST | 1,31 | 4,81 | 2,65E-02 | * |
| NM_001025197 | CHI3L2 | 1,18 | 4,70 | 4,01E-02 | * | NM_001330631 | CAST | 1,31 | 4,81 | 2,65E-02 | * |
| NM_001025199 | CHI3L2 | 1,18 | 4,70 | 4,03E-02 | * | NM_001330632 | CAST | 1,31 | 4,81 | 2,65E-02 | * |
| NM_001033549 | BABAM1 | 0,86 | 5,37 | 2,78E-02 | * | NM_001330633 | CAST | 1,31 | 4,81 | 2,65E-02 | * |
| NM_001035505 | BOLA3 | 0,80 | 5,72 | 1,46E-02 | * | NM_001330634 | CAST | 1,31 | 4,81 | 2,65E-02 | * |
| NM_001037637 | BTF3 | 0,45 | 7,86 | 1,90E-02 | * | NM_001346292 | MICAL2 | 1,76 | 2,23 | 2,02E-02 | * |
| NM_001040033 | CD53 | 1,02 | 6,33 | 2,85E-02 | * | NM_001346293 | MICAL2 | 1,76 | 2,23 | 2,00E-02 | * |
| NM_001042440 | CAST | 1,30 | 4,82 | 2,80E-02 | * | NM_001346294 | MICAL2 | 1,76 | 2,23 | 2,02E-02 | * |
| NM_001042441 | CAST | 1,30 | 4,82 | 2,80E-02 | * | NM_001346295 | MICAL2 | 1,75 | 2,24 | 2,14E-02 | * |
| NM_001042442 | CAST | 1,30 | 4,82 | 2,80E-02 | * | NM_001346296 | MICAL2 | 1,75 | 2,24 | 2,14E-02 | * |
| NM_001042443 | CAST | 1,31 | 4,81 | 2,65E-02 | * | NM_001346297 | MICAL2 | 1,76 | 2,23 | 2,00E-02 | * |
| NM_001042444 | CAST | 1,31 | 4,81 | 2,65E-02 | * | NM_001349655 | PIBF1 | 1,23 | 4,66 | 2,58E-02 | * |
| NM_001042445 | CAST | 1,31 | 4,81 | 2,65E-02 | * | NM_001349877 | RAB36 | 4,19 | -0,11 | 1,43E-02 | * |
| NM_001042446 | CAST | 1,31 | 4,81 | 2,65E-02 | * | NM_001349878 | RAB36 | 4,19 | -0,11 | 1,43E-02 | * |
| NM_001048201 | UHRF1 | 1,55 | 2,96 | 3,70E-02 | * | NM_001350488 | MTHFD1L | 0,94 | 4,70 | 2,28E-02 | * |
| NM_001077263 | TMPRSS13 | 3,30 | 1,99 | 4,75E-02 | * | NM_001350489 | MTHFD1L | 0,97 | 4,65 | 1,82E-02 | * |
| NM_001077484 | SLC38A1 | 2,63 | 3,60 | 4,99E-02 | * | NM_001350491 | MTHFD1L | 0,95 | 4,69 | 2,15E-02 | * |
| NM_001080404 | ZNF878 | 6,10 | -0,06 | 1,31E-03 | * | NM_001512 | GSTA4 | 2,40 | 2,64 | 3,26E-02 | * |
| NM_001080477 | TENM3 | 2,15 | 1,73 | 3,68E-02 | * | NM 001692 | ATP6V1B1 | 1,10 | 6,14 | 1,84E-02 | * |
| NM_001082959 | SCARB1 | 7,88 | 0,03 | 3,85E-03 | * | NM 001743 | CALM2 | 0,51 | 8,93 | 1,27E-02 | * |
| NM_001085486 | SELENOP | 3,79 | 2,50 | 1,83E-02 | * | NM_001750 | CAST | 1,30 | 4,82 | 2,80E-02 | * |
| NM_001093726 | SELENOP | 3,79 | 2,50 | 1,82E-02 | * | NM 001776 | ENTPD1 | 0,96 | 4,87 | 4,05E-02 | * |
| NM_001098175 | ENTPD1 | 0,96 | 4,87 | 4,05E-02 | * | NM_001800 | CDKN2D | 1,10 | 3,64 | 2,32E-02 | * |
| NM_001098637 | PWWP2B | 2,64 | 2,95 | 1,07E-02 | * | NM_001863 | COX6B1 | 0,60 | 7,33 | 2,85E-02 | * |
| NM_001098845 | ANXA8L1 | 1,36 | 4,77 | 2,97E-02 | * | NM_002273 | KRT8 | 0,83 | 10,01 | 1,88E-02 | * |
| NM_001100388 | C11orf88 | 7,54 | -1,11 | 2,23E-03 | * | NM_002276 | KRT19 | 1,91 | 6,00 | 2,44E-02 | * |
| NM 001100818 | PID1 | 4,31 | 0,82 | 2,22E-03 | * | NM_002298 | LCP1 | 3,45 | 3,62 | 1,83E-02 | * |
| NM_001127505 | ASAH1 | 1,60 | 4,88 | 1,56E-02 | # | NM_002492 | NDUFB5 | 0,64 | 5,90 | 1,46E-02 | * |
| NM_001127716 | ETFA | 0,77 | 6,46 | 1,91E-02 | * | NM_002787 | PSMA2 | 0,47 | 7,50 | 2,56E-02 | * |
| NM_001130701 | STYX | 0,90 | 3,61 | 3,77E-02 | * | NM_002792 | PSMA7 | 0,52 | 7,86 | 1,56E-02 | * |
| NM_001130721 | ELOVL6 | 1,40 | 4,26 | 1,77E-02 | * | NM_003287 | TPD52L1 | 3,21 | 2,27 | 2,97E-02 | * |
| NM_001135650 | EEF1E1 | 0,79 | 4,55 | 2,65E-02 | * | NM_003847 | PEX11A | 1,46 | 2,58 | 3,62E-02 | * |
| NM_001142502 | PPP1R13L | 2,07 | 2,50 | 1,09E-02 | * | NM_004000 | CHI3L2 | 1,18 | 4,70 | 4,00E-02 | * |
| NM_001145155 | NR2F2 | 3,49 | 1,12 | 2,36E-02 | * | NM_004035 | ACOX1 | 1,38 | 4,84 | 1,16E-02 | * |
| NM_001145156 | NR2F2 | 3,59 | 1,11 | 1,85E-02 | * | NM_004152 | OAZ1 | 0,52 | 8,45 | 2,65E-02 | * |
| NM_001145157 | NR2F2 | 3,58 | 1,10 | 1,95E-02 | * | NM_004173 | SLC7A4 | 1,72 | 4,14 | 3,49E-02 | * |
| NM_001145998 | SLC15A2 | 3,59 | 1,27 | 2,25E-02 | * | NM_004315 | ASAH1 | 1,60 | 4,88 | 1,55E-02 | # |
| NM_001146108 | PTGR1 | 1,36 | 6,10 | 1,70E-02 | * | NM_004458 | ACSL4 | 0,99 | 4,30 | 4,98E-02 | * |
| NM_001146109 | PTGR1 | 1,33 | 5,99 | 2,52E-02 | * | NM_004551 | NDUFS3 | 0,58 | 6,43 | 1,49E-02 | * |
| NM_001155 | ANXA6 | 1,85 | 5,44 | 2,86E-02 | * | NM_004625 | WNT7A | 2,59 | 2,37 | 2,11E-02 | * |
| NM_001160243 | RPAIN | 0,97 | 4,58 | 1,54E-02 | * | NM_004763 | ITGB1BP1 | 0,68 | 5,47 | 2,06E-02 | * |
| NM_001164178 | ENTPD1 | 0,96 | 4,87 | 4,05E-02 | * | NM_004821 | HAND1 | 4,90 | 2,75 | 2,00E-02 | * |
| NM_001164179 | ENTPD1 | 0,96 | 4,87 | 4,05E-02 | * | NM_004844 | SH3BP5 | 4,14 | 0,74 | 1,41E-02 | * |
| NM_001164181 | ENTPD1 | 0,96 | 4,87 | 4,05E-02 | * | NM_004905 | PRDX6 | 0,61 | 6,36 | 4,29E-02 | * |
| NM_001164182 | ENTPD1 | 0,96 | 4,87 | 4,05E-02 | * | NM 004914 | RAB36 | 4,19 | -0,11 | 1,43E-02 | * |
| NM_001164183 | ENTPD1 | 0,96 | 4,87 | 4,08E-02 | * | NM_005175 | ATP5MC1 | 0,75 | 5,85 | 2,03E-02 | * |
| NM_001164473 | FNBP1L | 0,69 | 5,57 | 4,05E-02 | * | NM_005410 | SELENOP | 3,79 | 2,50 | 1,83E-02 | * |
| NM_001166110 | PALLD | 1,08 | 4,11 | 2,84E-02 | * | NM_005505 | SCARB1 | 7,88 | 0,03 | 3,85E-03 | * |
| NM_001167607 | GM2A | 1,56 | 4,14 | 1,95E-02 | * | NM_005715 | UST | 1,98 | 2,72 | 3,13E-02 | * |
| NM_001174160 | SH2D4A | 1,85 | 4,19 | 1,06E-02 | * | NM_005918 | MDH2 | 0,84 | 7,55 | 1,76E-02 | * |
| NM_001185039 | ACOX1 | 1,38 | 4,84 | 1,16E-02 | * | NM_006002 | UCHL3 | 0,65 | 5,60 | 2,92E-02 | * |
| NM_001190442 | CAST | 1,30 | 4,82 | 2,80E-02 | * | NM_006082 | TUBA1B | 0,54 | 9,60 | 1,50E-02 | * |
| NM_001193334 | WLS | 3,33 | 2,13 | 1,84E-02 | * | NM_006346 | PIBF1 | 1,23 | 4,66 | 2,65E-02 | * |
| NM_001193339 | SLC13A3 | 6,29 | -1,44 | 1,80E-02 | * | NM_006547 | IGF2BP3 | 1,30 | 3,03 | 4,79E-02 | * |
| NM_001193342 | SLC13A3 | 7,12 | -1,47 | 6,40E-03 | * | NM_006579 | EBP | 0,84 | 7,03 | 1,80E-02 | # |
| NM_001193544 | ANXA6 | 1,85 | 5,43 | 2,88E-02 | * | NM_006601 | PTGES3 | 0,60 | 7,76 | 2,13E-02 | * |
| NM_001199957 | NDUFB5 | 0,64 | 5,89 | 1,47E-02 | * | NM_006663 | PPP1R13L | 2,05 | 2,50 | 1,25E-02 | * |
| NM_001199958 | NDUFB5 | 0,65 | 5,89 | 1,36E-02 | * | NM_006830 | UQCR11 | 0,65 | 6,23 | 2,79E-02 | * |
| NM_001204221 | ZNF695 | 1,30 | 3,30 | 1,56E-02 | * | NM_006870 | DSTN | 0,72 | 5,96 | 1,70E-02 | * |
| NM_001206789 | TMPRSS13 | 3,29 | 1,99 | 4,83E-02 | * | NM_006918 | SC5D | 1,90 | 2,68 | 3,52E-02 | * |
| NM_001207 | BTF3 | 0,46 | 7,86 | 1,89E-02 | * | NM_007062 | PWP1 | 0,71 | 5,82 | 2,81E-02 | * |
| NM_001218 | CA12 | 8,03 | 1,00 | 1,62E-03 | * | NM_007283 | MGLL | 2,14 | 2,88 | 1,95E-02 | * |
| NM_001242769 | MTHFD1L | 0,94 | 4,70 | 2,28E-02 | * | NM_007292 | ACOX1 | 1,38 | 4,84 | 1,16E-02 | * |
| NM_001242911 | ZFAND6 | 0,77 | 5,47 | 1,43E-02 | * | NM_007310 | COMT | 1,49 | 3,36 | 4,99E-02 | * |
| NM_001242912 | ZFAND6 | 0,77 | 5,47 | 1,43E-02 | * | NM_012212 | PTGR1 | 1,36 | 6,10 | 1,70E-02 | * |
| NM_001242913 | ZFAND6 | 0,77 | 5,47 | 1,43E-02 | * | NM_013282 | UHRF1 | 1,55 | 2,96 | 3,73E-02 | * |
| NM_001242914 | ZFAND6 | 0,77 | 5,47 | 1,46E-02 | * | NM_014143 | CD274 | 7,27 | -0,20 | 2,21E-02 | * |
| NM_001242915 | ZFAND6 | 0,77 | 5,46 | 1,40E-02 | * | NM_014173 | BABAM1 | 0,86 | 5,37 | 2,78E-02 | * |
| NM_001242916 | ZFAND6 | 0,77 | 5,46 | 1,44E-02 | * | NM_014298 | QPRT | 2,44 | 3,21 | 1,52E-02 | * |
| NM_001242917 | ZFAND6 | 0,77 | 5,47 | 1,46E-02 | * | NM_014320 | HEBP2 | 0,65 | 5,07 | 4,33E-02 | * |
| NM_001242918 | ZFAND6 | 0,77 | 5,47 | 1,43E-02 | * | NM_014632 | MICAL2 | 1,75 | 2,24 | 2,14E-02 | * |
| NM_001242919 | ZFAND6 | 0,77 | 5,46 | 1,43E-02 | * | NM_014900 | COBLL1 | 1,37 | 4,12 | 1,52E-02 | * |
| NM_001251989 | GMNN | 0,64 | 6,34 | 1,56E-02 | * | NM_015027 | PDXDC1 | 0,72 | 4,93 | 4,92E-02 | * |
| NM_001251990 | GMNN | 0,63 | 6,32 | 1,61E-02 | * | NM_015173 | TBC1D1 | 0,88 | 5,09 | 2,11E-02 | * |
| NM_001251991 | GMNN | 0,64 | 6,28 | 1,73E-02 | * | NM_015315 | LARP1 | 0,66 | 6,24 | 2,72E-02 | * |
| NM_001253912 | TBC1D1 | 0,88 | 5,06 | 2,47E-02 | * | NM_015525 | IBTK | 0,76 | 4,89 | 2,18E-02 | * |
| NM_001256282 | KRT8 | 0,83 | 10,01 | 1,88E-02 | * | NM_015895 | GMNN | 0,64 | 6,34 | 1,56E-02 | * |
| NM_001256293 | KRT8 | 0,83 | 10,01 | 1,88E-02 | * | NM_016126 | HSPB11 | 1,21 | 5,02 | 1,91E-02 | * |
| NM_001256585 | MGLL | 2,14 | 2,88 | 1,95E-02 | * | NM_016147 | PPME1 | 0,90 | 4,91 | 2,47E-02 | * |
| NM_001258248 | SP6 | 2,43 | 2,08 | 2,31E-02 | * | NM_017572 | MKNK2 | 1,71 | 2,42 | 3,64E-02 | * |
| NM_001267560 | TJP3 | 1,75 | 4,34 | 4,36E-02 | * | NM_017737 | FNBP1L | 0,69 | 5,57 | 4,12E-02 | * |
| NM_001267561 | TJP3 | 1,75 | 4,34 | 4,38E-02 | * | NM_017855 | ODAM | 7,11 | 1,05 | 2,38E-04 | * |
| NM_001267598 | MGST1 | 2,32 | 2,84 | 1,42E-02 | * | NM_017933 | PID1 | 4,36 | 0,85 | 2,04E-03 | * |
| NM_001267706 | CD274 | 7,12 | -0,32 | 2,65E-02 | # | NM_018339 | RFK | 0,74 | 6,50 | 2,43E-02 | * |
| NM_001270952 | UCHL3 | 0,65 | 5,61 | 2,89E-02 | * | NM_018464 | CISD1 | 0,63 | 5,96 | 1,98E-02 | * |
| NM_001271572 | PEX11A | 1,46 | 2,58 | 3,62E-02 | * | NM_019006 | ZFAND6 | 0,77 | 5,47 | 1,43E-02 | * |
| NM_001271573 | PEX11A | 1,55 | 2,60 | 2,94E-02 | * | NM_019112 | ABCA7 | 5,08 | -0,61 | 2,67E-02 | * |
| NM_001271593 | PPME1 | 0,90 | 4,91 | 2,49E-02 | * | NM_020145 | SH3GLB2 | 1,20 | 5,77 | 1,52E-02 | * |
| NM_001278387 | SLC38A1 | 2,63 | 3,61 | 4,98E-02 | * | NM_020387 | RAB25 | 1,26 | 6,03 | 2,67E-02 | * |
| NM_001278458 | COBLL1 | 1,38 | 4,12 | 1,45E-02 | * | NM_020394 | ZNF695 | 1,24 | 3,50 | 2,31E-02 | * |
| NM_001278460 | COBLL1 | 1,37 | 4,12 | 1,46E-02 | * | NM_021021 | SNTB1 | 3,69 | 1,24 | 1,69E-02 | * |
| NM_001278461 | COBLL1 | 1,37 | 4,12 | 1,52E-02 | * | NM_021074 | NDUFV2 | 0,64 | 6,56 | 1,45E-02 | * |
| NM_001278923 | ANXA8L1 | 1,29 | 4,76 | 3,67E-02 | * | NM_021075 | NDUFV3 | 0,78 | 5,22 | 3,72E-02 | * |
| NM_001278924 | ANXA8L1 | 1,29 | 4,76 | 3,68E-02 | * | NM_021082 | SLC15A2 | 3,59 | 1,27 | 2,25E-02 | * |
| NM_001282167 | PORCN | 1,90 | 2,66 | 3,90E-02 | * | NM_022334 | ITGB1BP1 | 0,69 | 5,46 | 1,99E-02 | * |
| NM_001282403 | MDH2 | 0,84 | 7,55 | 1,76E-02 | * | NM_022772 | EPS8L2 | 1,46 | 4,31 | 4,66E-02 | * |
| NM_001282404 | MDH2 | 0,84 | 7,55 | 1,75E-02 | * | NM_022825 | PORCN | 1,89 | 2,64 | 4,87E-02 | * |
| NM_001282601 | PTGES3 | 0,60 | 7,76 | 2,17E-02 | * | NM_022829 | SLC13A3 | 6,29 | -1,44 | 1,80E-02 | * |
| NM_001282602 | PTGES3 | 0,60 | 7,76 | 2,13E-02 | * | NM_024090 | ELOVL6 | 1,40 | 4,26 | 1,77E-02 | * |
| NM_001282603 | PTGES3 | 0,60 | 7,76 | 2,13E-02 | * | NM_024307 | GDPD3 | 3,06 | 1,78 | 1,94E-02 | * |
| NM_001282604 | PTGES3 | 0,60 | 7,76 | 2,21E-02 | * | NM_024310 | PLEKHF1 | 7,84 | 0,52 | 9,25E-05 | * |
| NM_001282605 | PTGES3 | 0,60 | 7,75 | 2,15E-02 | * | NM_024911 | WLS | 3,36 | 2,20 | 1,70E-02 | * |
| NM_001282663 | MICAL2 | 1,75 | 2,24 | 2,14E-02 | * | NM_031469 | SH3BGRL2 | 1,86 | 2,82 | 4,05E-02 | * |
| NM_001282664 | MICAL2 | 1,75 | 2,23 | 2,34E-02 | * | NM_032233 | SETD3 | 1,02 | 6,32 | 2,95E-02 | * |
| NM_001284212 | CAST | 1,31 | 4,81 | 2,65E-02 | * | NM_032731 | TXNDC17 | 0,63 | 6,16 | 4,12E-02 | * |
| NM_001284213 | CAST | 1,31 | 4,81 | 2,72E-02 | * | NM_033128 | SCIN | 6,45 | 0,63 | 3,42E-03 | * |
| NM_001285444 | PDXDC1 | 0,72 | 4,93 | 4,92E-02 | * | NM_033504 | TMEM54 | 1,00 | 5,99 | 2,85E-02 | * |
| NM_001285445 | PDXDC1 | 0,72 | 4,93 | 4,92E-02 | * | NM_033515 | ARHGAP18 | 0,87 | 5,73 | 2,51E-02 | * |
| NM_001285447 | PDXDC1 | 0,73 | 4,93 | 4,79E-02 | * | NM_079421 | CDKN2D | 1,10 | 3,64 | 2,32E-02 | * |
| NM_001285448 | PDXDC1 | 0,73 | 4,92 | 4,72E-02 | * | NM_080659 | C11orf52 | 2,91 | 1,81 | 1,47E-02 | * |
| NM_001287045 | SH3GLB2 | 1,20 | 5,77 | 1,52E-02 | * | NM_080833 | RBBP8NL | 7,29 | -1,32 | 1,45E-02 | * |
| NM_001287046 | SH3GLB2 | 1,19 | 5,67 | 1,90E-02 | * | NM_130777 | XAGE2 | 1,62 | 4,31 | 2,53E-02 | * |
| NM_001288622 | ICA1L | 1,94 | 1,99 | 4,27E-02 | * | NM_130902 | COX7B2 | 0,92 | 4,72 | 2,56E-02 | * |
| NM_001288623 | ICA1L | 1,95 | 1,99 | 4,08E-02 | * | NM_138468 | ICA1L | 1,95 | 1,99 | 4,05E-02 | * |
| NM_001288756 | BABAM1 | 0,86 | 5,37 | 2,78E-02 | * | NM_139207 | NAP1L1 | 0,74 | 6,60 | 1,05E-02 | * |
| NM_001288757 | BABAM1 | 0,87 | 5,35 | 2,78E-02 | * | NM_145244 | DDIT4L | 1,03 | 4,48 | 1,46E-02 | * |
| NM_001290050 | UHRF1 | 1,55 | 2,96 | 3,80E-02 | * | NM_145251 | STYX | 0,96 | 3,64 | 2,80E-02 | * |
| NM_001290051 | UHRF1 | 1,55 | 2,96 | 3,73E-02 | * | NM_152342 | CDYL2 | 5,33 | 0,41 | 1,18E-02 | * |
| NM_001290052 | UHRF1 | 1,55 | 2,96 | 3,73E-02 | * | NM_152766 | TMEM256 | 1,19 | 2,88 | 2,58E-02 | * |
| NM_001292026 | TPD52L1 | 3,22 | 2,26 | 2,89E-02 | * | NM_173060 | CAST | 1,31 | 4,81 | 2,65E-02 | * |
| NM_001293642 | CA12 | 8,03 | 1,00 | 1,62E-03 | * | NM_177924 | ASAH1 | 1,60 | 4,88 | 1,56E-02 | * |
| NM_001296 | ACKR2 | 6,42 | 0,51 | 2,87E-06 | * | NM_198182 | GRHL1 | 1,67 | 3,83 | 2,33E-02 | * |
| NM_001300906 | IBTK | 0,76 | 4,89 | 2,18E-02 | * | NM_199054 | MKNK2 | 1,78 | 2,66 | 2,61E-02 | * |
| NM_001300994 | TPD52L1 | 3,21 | 2,27 | 2,97E-02 | * | NM_199187 | KRT18 | 0,78 | 10,33 | 2,65E-02 | * |
| NM_001301020 | OAZ1 | 0,52 | 8,45 | 2,65E-02 | * | NM_199262 | SP6 | 2,42 | 2,07 | 2,36E-02 | * |
| NM_001305624 | CALM2 | 0,51 | 8,93 | 1,27E-02 | * | NM_203473 | PORCN | 1,89 | 2,64 | 4,87E-02 | * |
| NM_001305625 | CALM2 | 0,51 | 8,93 | 1,27E-02 | * | NM_203475 | PORCN | 1,89 | 2,65 | 4,83E-02 | * |
| NM_001305626 | CALM2 | 0,51 | 8,93 | 1,25E-02 | * | NM 206925 | CA12 | 8,03 | 1,00 | 1,62E-03 | * |
| NM_001312654 | ENTPD1 | 0,96 | 4,87 | 4,05E-02 | * | NM_207430 | C11orf88 | 7,55 | -1,11 | 2,22E-03 | * |
| NM_001316935 | HSPB11 | 1,21 | 5,02 | 1,91E-02 | * | NM 212552 | BOLA3 | 0,80 | 5,72 | 1,46E-02 | * |
| NM_001317962 | PWP1 | 0,71 | 5,81 | 2,84E-02 | * | NR_027679 | RPAIN | 0,91 | 4,56 | 2,23E-02 | * |
| NM_001317963 | PWP1 | 0,71 | 5,82 | 2,81E-02 | * | NR_037651 | HSPB2-C11orf52 | 2,80 | 1,80 | 1,83E-02 | * |
| NM_001318249 | QPRT | 2,43 | 2,92 | 2,13E-02 | * | NR_037892 | ZNF695 | 1,24 | 3,29 | 2,25E-02 | * |
| NM_001318250 | QPRT | 2,41 | 3,02 | 1,50E-02 | * | NR_037894 | ZNF670-ZNF695 | 1,17 | 3,39 | 2,97E-02 | * |
| NM_001318509 | ACSL4 | 0,99 | 4,31 | 4,92E-02 | * | NR_045962 | KRT8 | 0,74 | 10,45 | 2,88E-02 | * |
| NM_001318510 | ACSL4 | 0,99 | 4,30 | 4,99E-02 | * | NR_104219 | PTGES3 | 0,54 | 7,73 | 3,62E-02 | * |
| NM_001318903 | TPD52L1 | 3,11 | 2,26 | 3,31E-02 | * | NR_104285 | CAST | 1,23 | 3,94 | 4,99E-02 | * |
| NM_001318907 | TPD52L1 | 3,13 | 2,24 | 3,17E-02 | * | NR_120681 | C8orf59 | 0,75 | 4,95 | 1,55E-02 | * |
| NM_001319066 | ITGB1BP1 | 0,68 | 5,50 | 2,36E-02 | * | NR_131754 | STARD4 | 3,40 | 2,26 | 1,10E-02 | * |
| NM_001319067 | ITGB1BP1 | 0,68 | 5,49 | 2,29E-02 | * | NR_133632 | HSPB11 | 1,15 | 5,00 | 2,29E-02 | * |
| NM_001319068 | ITGB1BP1 | 0,69 | 5,49 | 2,11E-02 | * | NR_133653 | RAB25 | 1,18 | 6,01 | 3,66E-02 | * |
| NM_001319069 | ITGB1BP1 | 0,68 | 5,43 | 2,13E-02 | * | NR_134536 | QPRT | 2,33 | 2,99 | 1,93E-02 | * |
| NM_001319070 | ITGB1BP1 | 0,68 | 5,44 | 1,98E-02 | * | NR_134952 | ITGB1BP1 | 0,63 | 5,46 | 3,54E-02 | * |
| NM_001320638 | CD53 | 1,02 | 6,32 | 2,84E-02 | * | NR_144418 | MICAL2 | 1,62 | 2,27 | 3,05E-02 | * |
| NM_001324019 | PDXDC1 | 0,72 | 4,93 | 4,92E-02 | * | NR 146205 | PIBF1 | 1,19 | 4,65 | 2,85E-02 | * |
| NM_001326380 | HEBP2 | 0,65 | 5,06 | 4,35E-02 | * | **(P** = **Preferred):** * = **at least one of these genes** | | | | | |

The biomarkers in Table D have a statistically significantly increased level of expression in cells that have a lower likelihood of implantation success compared with those that have a higher or increased likelihood of implantation success. Table D includes genes and gene transcripts with overall higher FDRs (between 0.01 and 0.05) and lower logFC in relation to Table B.

**Table D**

| **rba-AMA biomarkers with q-value above 0.01 or CPM<2 or present in one of the analyses (with and without non-coding transcripts)** | | | | | | | | | | | |
|---|---|---|---|---|---|---|---|---|---|---|---|
| **refSeq** | **gene name** | **log FC** | **log CPM** | **FDR** | **P** | **refSeq** | **gene name** | **log FC** | **log CPM** | **FDR** | **P** |
| NM_000104 | CYP1B1 | -2,94 | 3,51 | 1,05E-02 | * | NM_001324277 | CXorf40A | -1,13 | 3,15 | 4,05E-02 | * |
| NM_000116 | TAZ | -1,30 | 3,34 | 4,79E-02 | * | NM_001324278 | CXorf40A | -1,13 | 3,15 | 4,05E-02 | * |
| NM_000120 | EPHX1 | -1,75 | 1,05 | 2,72E-02 | * | NM_001324279 | CXorf40A | -1,02 | 3,36 | 3,81E-02 | * |
| NM_000167 | GK | -2,42 | 2,37 | 2,30E-02 | * | NM_001324280 | CXorf40A | -1,13 | 3,15 | 4,05E-02 | * |
| NM_000179 | MSH6 | -0,99 | 5,80 | 2,96E-02 | * | NM_001329861 | TRUB2 | -0,65 | 5,48 | 3,31E-02 | * |
| NM_000232 | SGCB | -1,31 | 3,68 | 2,65E-02 | * | NM_001329863 | TRUB2 | -0,65 | 5,48 | 3,39E-02 | * |
| NM_000259 | MYO5A | -1,07 | 4,46 | 2,55E-02 | * | NM_001330782 | PBX3 | -1,59 | 2,38 | 3,86E-02 | * |
| NM_000565 | IL6R | -5,26 | 0,58 | 3,73E-03 | * | NM_001346043 | FNIP2 | -1,09 | 4,57 | 1,89E-02 | * |
| NM_000745 | CHRNA5 | -1,65 | 2,48 | 2,50E-02 | * | NM_001347947 | IFT81 | -3,59 | 0,37 | 2,84E-02 | * |
| NM_000789 | ACE | -1,92 | 2,79 | 3,16E-02 | * | NM_001347948 | IFT81 | -3,58 | 0,41 | 2,11E-02 | * |
| NM_001002838 | WNK3 | -3,00 | 1,04 | 8,62E-03 | # | NM_001348520 | KIAA1257 | -1,39 | 3,33 | 1,95E-02 | * |
| NM_001005404 | YPEL2 | -0,97 | 5,90 | 4,02E-02 | * | NM_001348521 | KIAA1257 | -1,40 | 3,33 | 1,88E-02 | * |
| NM_001008211 | OPTN | -1,67 | 3,18 | 1,53E-02 | * | NM_001349821 | MFSD2A | -6,97 | 2,20 | 2,12E-04 | * |
| NM_001008212 | OPTN | -1,67 | 3,18 | 1,53E-02 | * | NM_001349822 | MFSD2A | -6,97 | 2,20 | 2,12E-04 | * |
| NM_001008213 | OPTN | -1,67 | 3,18 | 1,53E-02 | * | NM_001349823 | MFSD2A | -6,97 | 2,20 | 2,12E-04 | * |
| NM_001008222 | ZDHHC9 | -1,15 | 4,61 | 2,81E-02 | * | NM_001351264 | C17orf80 | -0,72 | 4,66 | 2,25E-02 | * |
| NM_001010889 | PRAMEF6 | -4,10 | 1,84 | 1,73E-03 | * | NM_001351265 | C17orf80 | -0,92 | 4,07 | 3,13E-02 | * |
| NM_001010917 | GOLGA7B | -4,55 | 0,80 | 3,65E-03 | * | NM_001351307 | DUXB | -2,50 | 1,15 | 2,77E-02 | * |
| NM_001010924 | FAM171A1 | -2,05 | 2,39 | 3,63E-02 | # | NM_001351308 | DUXB | -2,56 | 1,09 | 2,65E-02 | * |
| NM_001011649 | CDK5RAP2 | -0,87 | 5,20 | 3,12E-02 | * | NM_001351309 | DUXB | -2,52 | 1,14 | 2,84E-02 | * |
| NM_001012975 | RNASE10 | -6,84 | -1,17 | 5,57E-03 | * | NM_001351404 | LPAR1 | -4,66 | 0,38 | 2,78E-02 | * |
| NM_001013407 | PRAMEF5 | -4,03 | 1,21 | 2,65E-02 | * | NM_001351405 | LPAR1 | -4,63 | 0,38 | 2,88E-02 | * |
| NM_001013627 | NHSL2 | -8,92 | -0,71 | 1,40E-05 | * | NM_001351411 | LPAR1 | -4,70 | 0,38 | 2,61E-02 | * |
| NM_001013845 | CXorf40B | -0,95 | 4,48 | 3,07E-02 | * | NM_001351414 | LPAR1 | -4,67 | 0,38 | 2,72E-02 | * |
| NM_001015038 | PAGE2B | -1,60 | 4,29 | 4,38E-02 | * | NM_001351417 | LPAR1 | -4,71 | 0,38 | 2,56E-02 | * |
| NM_001015882 | DNAJC25 | -1,16 | 3,51 | 3,13E-02 | * | NM_001351419 | LPAR1 | -4,70 | 0,38 | 2,61E-02 | * |
| NM_001024844 | CD82 | -3,86 | 1,47 | 2,79E-02 | * | NM_001351420 | LPAR1 | -4,70 | 0,38 | 2,61E-02 | * |
| NM_001029896 | WDR45 | -1,02 | 5,88 | 2,93E-02 | * | NM_001353368 | CLEC2L | -2,55 | 0,84 | 2,67E-02 | * |
| NM_001031696 | PLD3 | -1,43 | 3,73 | 2,01E-02 | * | NM_001354334 | KANK1 | -0,98 | 3,84 | 2,78E-02 | * |
| NM_001032381 | PQBP1 | -0,77 | 5,71 | 4,54E-02 | * | NM_001520 | GTF3C1 | -1,09 | 3,64 | 2,56E-02 | * |
| NM_001032382 | PQBP1 | -0,77 | 5,71 | 4,54E-02 | * | NM_001540 | HSPB1 | -1,80 | 1,89 | 1,61E-02 | * |
| NM_001032383 | PQBP1 | -0,77 | 5,71 | 4,54E-02 | * | NM_001675 | ATF4 | -0,81 | 7,16 | 1,25E-02 | * |
| NM_001032384 | PQBP1 | -0,77 | 5,70 | 4,49E-02 | * | NM_001707 | BCL7B | -0,91 | 4,53 | 2,68E-02 | * |
| NM_001037501 | NBPF8 | -1,25 | 4,06 | 4,93E-02 | * | NM_001726 | BRDT | -0,88 | 5,49 | 1,81E-02 | * |
| NM_001037633 | SIL1 | -1,04 | 4,52 | 2,78E-02 | * | NM_001975 | ENO2 | -2,30 | 3,32 | 2,37E-02 | * |
| NM_001039361 | PRAMEF10 | -2,55 | 1,90 | 3,65E-02 | * | NM_002231 | CD82 | -3,86 | 1,47 | 2,79E-02 | * |
| NM_001039697 | SNAPC3 | -0,86 | 4,10 | 2,96E-02 | * | NM_002359 | MAFG | -1,19 | 3,95 | 1,99E-02 | * |
| NM_001040451 | RUFY1 | -0,93 | 4,83 | 4,50E-02 | * | NM_002361 | MAG | -1,53 | 4,37 | 3,49E-02 | * |
| NM_001040452 | RUFY1 | -0,93 | 4,83 | 4,54E-02 | * | NM_002653 | PITX1 | -1,00 | 4,68 | 4,22E-02 | * |
| NM_001080511 | CLEC2L | -2,55 | 0,84 | 2,67E-02 | * | NM_002862 | PYGB | -0,96 | 6,98 | 2,72E-02 | * |
| NM_001083617 | RB1CC1 | -0,85 | 5,59 | 2,85E-02 | * | NM_002970 | SAT1 | -1,58 | 2,97 | 4,04E-02 | * |
| NM_001085480 | FAM162B | -2,28 | 2,24 | 1,40E-02 | * | NM_003033 | ST3GAL1 | -1,30 | 3,87 | 3,13E-02 | * |
| NM_001098479 | HLA-F | -3,75 | 0,46 | 3,29E-03 | * | NM_003150 | STAT3 | -1,69 | 3,60 | 2,25E-02 | * |
| NM_001098786 | HILPDA | -0,92 | 4,42 | 2,25E-02 | * | NM_003213 | TEAD4 | -0,66 | 5,74 | 4,05E-02 | * |
| NM_001099685 | RHOXF2B | -1,92 | 5,81 | 2,51E-02 | * | NM_003244 | TGIF1 | -0,74 | 5,01 | 3,43E-02 | * |
| NM_001099851 | PRAMEF17 | -3,58 | 1,58 | 1,35E-02 | * | NM_003395 | WNT9A | -2,91 | 1,41 | 1,67E-02 | * |
| NM_001099852 | PRAMEF20 | -2,27 | 3,13 | 1,36E-02 | * | NM_003692 | TMEFF1 | -2,07 | 1,63 | 4,38E-02 | * |
| NM_001100621 | C17orf80 | -0,92 | 4,07 | 3,11E-02 | * | NM_003705 | SLC25A12 | -2,36 | 1,53 | 2,72E-02 | * |
| NM_001100622 | C17orf80 | -1,23 | 3,44 | 1,47E-02 | * | NM_003712 | PLPP2 | -2,52 | 4,08 | 1,53E-02 | * |
| NM_001110354 | ZP3 | -1,91 | 2,99 | 2,89E-02 | * | NM_003864 | SAP30 | -1,20 | 3,23 | 2,08E-02 | * |
| NM_001114090 | NGEF | -2,25 | 1,77 | 2,58E-02 | * | NM_003963 | TM4SF5 | -1,55 | 2,73 | 3,19E-02 | * |
| NM_001122779 | FAM124B | -8,08 | -1,02 | 1,72E-04 | * | NM_003977 | AIP | -1,28 | 2,85 | 4,11E-02 | * |
| NM_001123395 | CLDN19 | -1,61 | 4,00 | 2,50E-02 | * | NM_004083 | DDIT3 | -1,02 | 5,05 | 2,78E-02 | * |
| NM_001126129 | GINS3 | -0,86 | 4,78 | 2,23E-02 | * | NM_004267 | CHST2 | -1,26 | 4,64 | 2,72E-02 | # |
| NM_001126130 | GINS3 | -0,86 | 4,77 | 2,29E-02 | * | NM_004720 | LPAR2 | -1,44 | 3,65 | 4,52E-02 | * |
| NM_001127211 | SHTN1 | -1,14 | 3,37 | 2,50E-02 | * | NM_004789 | LHX2 | -6,72 | 0,01 | 1,31E-04 | * |
| NM_001127361 | RNF19B | -1,24 | 3,64 | 2,22E-02 | * | NM_004799 | ZFYVE9 | -1,26 | 3,34 | 3,06E-02 | * |
| NM_001127698 | SPINKS | -5,22 | 1,62 | 2,22E-03 | * | NM_004834 | MAP4K4 | -0,92 | 4,65 | 1,23E-02 | * |
| NM_001127599 | SPINKS | -5,11 | 1,16 | 1,52E-03 | * | NM_004918 | TCL1B | -1,69 | 2,51 | 4,05E-02 | * |
| NM_001128127 | GK | -2,41 | 2,37 | 2,29E-02 | * | NM**_**004925 | AQP3 | -1,11 | 6,33 | 3,70E-02 | * |
| NM_001130 | AES | -2,20 | 1,69 | 1,79E-02 | * | NM_004990 | MARS | -0,76 | 7,27 | 2,24E-02 | * |
| NM_001130820 | IFT22 | -1,13 | 4,00 | 3,25E-02 | * | NM_005094 | SLC27A4 | -0,99 | 5,16 | 4,89E-02 | * |
| NM_001130821 | IFT22 | -1,13 | 4,00 | 3,25E-02 | * | NM_005242 | F2RL1 | -1,17 | 5,23 | 4,42E-02 | * |
| NM_001130822 | IFT22 | -1,13 | 4,00 | 3,25E-02 | * | NM_005271 | GLUD1 | -0,78 | 6,63 | 2,84E-02 | * |
| NM_001130964 | PLCD1 | -1,65 | 3,51 | 1,05E-02 | * | NM_005318 | H1FO | -1,34 | 5,03 | 1,50E-02 | * |
| NM_001130991 | HYOU1 | -1,29 | 4,56 | 2,65E-02 | * | NM_005566 | LDHA | -0,43 | 9,05 | 2,56E-02 | * |
| NM_001134778 | PBX3 | -1,57 | 2,36 | 4,59E-02 | * | NM_005612 | REST | -0,61 | 6,47 | 4,22E-02 | * |
| NM_001134999 | FERMT2 | -0,71 | 5,43 | 3,13E-02 | * | NM_005710 | PQBP1 | -0,77 | 5,70 | 4,49E-02 | * |
| NM_001135000 | FERMT2 | -0,73 | 5,06 | 2,82E-02 | * | NM_005802 | TOPORS | -0,97 | 4,00 | 2,33E-02 | * |
| NM_001135239 | LDHA | -0,43 | 9,04 | 2,55E-02 | * | NM_005866 | SIGMAR1 | -1,38 | 3,14 | 2,40E-02 | * |
| NM_001135768 | PVR | -1,40 | 3,46 | 2,50E-02 | * | NM_005897 | IPP | -1,31 | 2,87 | 2,60E-02 | * |
| NM_001135769 | PVR | -1,40 | 3,45 | 2,51E-02 | * | NM_006020 | ALKBH1 | -0,68 | 5,05 | 3,65E-02 | * |
| NM_001135773 | SPATA2 | -1,01 | 3,30 | 1,80E-02 | * | NM_006038 | SPATA2 | -1,01 | 3,30 | 1,82E-02 | * |
| NM_001135993 | TTC39C | -4,09 | 0,52 | 1,98E-02 | * | NM_006184 | NUCB1 | -1,10 | 5,21 | 2,51E-02 | * |
| NM_001135018 | EPHX1 | -1,75 | 1,05 | 2,74E-02 | * | NM_006195 | PBX3 | -1,59 | 2,38 | 3,86E-02 | * |
| NM_001136123 | SLF2 | -1,16 | 3,25 | 1,93E-02 | * | NM_006225 | PLCD1 | -1,65 | 3,52 | 1,05E-02 | * |
| NM_001136493 | MFSD2A | -6,97 | 2,20 | 2,12E-04 | * | NM_006322 | TUBGCP3 | -1,14 | 3,54 | 2,40E-02 | * |
| NM_001142495 | MYO5A | -1,07 | 4,46 | 2,55E-02 | * | NM_006389 | HYOU1 | -1,29 | 4,56 | 2,65E-02 | * |
| NM_001142549 | DDX4 | -5,76 | -0,60 | 3,97E-03 | * | NM_006505 | PVR | -1,40 | 3,46 | 2,48E-02 | * |
| NM_001142776 | CHAC1 | -2,57 | 4,29 | 3,27E-02 | * | NM_006526 | ZNF217 | -1,21 | 3,63 | 3,23E-02 | * |
| NM_001143688 | DIS3L | -1,06 | 4,27 | 3,39E-02 | * | NM_006572 | GNA13 | -0,77 | 4,94 | 4,75E-02 | * |
| NM_001143779 | IFT81 | -3,59 | 0,37 | 2,84E-02 | * | NM_006813 | PNRC1 | -0,96 | 3,99 | 4,15E-02 | * |
| NM_001143781 | FKBP11 | -1,26 | 3,35 | 4,40E-02 | * | NM_006832 | FERMT2 | -0,71 | 5,42 | 3,11E-02 | * |
| NM_001143782 | FKBP11 | -1,30 | 3,47 | 2,67E-02 | * | NM_006846 | SPINKS | -5,22 | 1,62 | 2,22E-03 | * |
| NM_001143821 | PLEKHA5 | -1,42 | 3,22 | 4,48E-02 | * | NM**_**006927 | ST3GAL2 | -1,28 | 3,40 | 1,60E-02 | * |
| NM_001143826 | MAPRE2 | -1,81 | *2,72* | 4,58E-02 | * | NM_006942 | SOX15 | -1,51 | 4,51 | 2,19E-02 | * |
| NM_001143827 | MAPRE2 | -1,81 | 2,72 | 4,58E-02 | * | NM_007075 | WDR45 | -1,06 | 5,89 | 1,90E-02 | * |
| NM_001143852 | TCHP | -1,38 | 2,92 | 2,38E-02 | * | NM_007121 | NR1H2 | -0,83 | 4,46 | 2,37E-02 | * |
| NM_001144382 | PLCL2 | -6,68 | -0,33 | 2,68E-03 | * | NM_007122 | USF1 | -1,81 | 2,25 | 3,49E-02 | * |
| NM_001145139 | CXorf49B | -1,91 | 5,05 | 3,85E-02 | * | NM_007155 | ZP3 | -1,92 | 2,99 | 2,89E-02 | * |
| NM_001145140 | CXorf49 | -1,91 | 5,05 | 3,85E-02 | * | NM_007223 | GPR176 | -2,27 | 2,46 | 2,10E-02 | * |
| NM_001145349 | IPP | -1,02 | 3,19 | 2,57E-02 | * | NM_007240 | DUSP12 | -0,78 | 4,32 | 2,51E-02 | * |
| NM_001145839 | MTFR1 | -1,04 | 2,94 | 3,49E-02 | * | NM_007324 | ZFYVE9 | -1,26 | 3,34 | 3,07E-02 | * |
| NM 001146343 | PQLC1 | -1,18 | 3,92 | 1,05E-02 | * | NM_007371 | BRD3 | -1,01 | 4,37 | 2,14E-02 | * |
| NM_001146345 | PQLC1 | -1,18 | 3,94 | 1,09E-02 | * | NM_012189 | CABYR | -1,66 | 3,22 | 4,83E-02 | * |
| NM_001161465 | JMJD4 | -0,83 | 4,97 | 3,34E-02 | * | NM_012268 | PLD3 | -1,43 | 3,73 | 2,06E-02 | * |
| NM_001163280 | HTATSF1 | -0,79 | 5,56 | 3,30E-02 | * | NM_013332 | HILPDA | -0,92 | 4,44 | 2,25E-02 | * |
| NM_001165414 | LDHA | -0,43 | 9,05 | 2,56E-02 | * | NM_014055 | IFT81 | -3,58 | 0,41 | 2,11E-02 | * |
| NM_001165415 | LDHA | -0,44 | 9,00 | 2,51E-02 | * | NM_014268 | MAPRE2 | -1,82 | 2,74 | 4,53E-02 | * |
| NM_001165416 | LDHA | -0,43 | 9,05 | 2,56E-02 | * | NM_014373 | GPR160 | -0,96 | 3,87 | 2,93E-02 | * |
| NM_001166283 | RGMA | -1,20 | 2,35 | 2,94E-02 | * | NM_014500 | HTATSF1 | -0,79 | 5,56 | 3,27E-02 | * |
| NM_001166286 | RGMA | -1,20 | 2,35 | 2,99E-02 | * | NM_014630 | ZNF592 | -1,11 | 3,72 | 3,49E-02 | * |
| NM_001166287 | RGMA | -1,20 | 2,35 | 3,00E-02 | * | NM_014683 | ULK2 | -2,10 | 2,14 | 3,73E-02 | * |
| NM_001155288 | RGMA | -1,20 | 2,35 | 2,94E-02 | * | NM_014730 | MLEC | -0,95 | 5,59 | 1,49E-02 | * |
| NM_001166289 | RGMA | -1,20 | 2,35 | 2,94E-02 | * | NM_014753 | BMS1 | -0,66 | 5,63 | 4,74E-02 | * |
| NM_001166533 | DDX4 | -5,92 | -0,62 | 2,51E-03 | * | NM_014754 | PTDSS1 | -0,89 | 4,98 | 4,77E-02 | * |
| NM_001166534 | DDX4 | -6,41 | -0,69 | 1,23E-03 | * | NM_014781 | RB1CC1 | -0,85 | 5,59 | 2,85E-02 | * |
| NM_001167989 | PQBP1 | -0,77 | 5,71 | 4,54E-02 | * | NM_014811 | PPP1R26 | -3,61 | 1,34 | 2,36E-02 | * |
| NM_001167990 | PQBP1 | -0,77 | 5,71 | 4,54E-02 | * | NM_015055 | SWAP70 | -0,76 | 4,95 | 2,40E-02 | * |
| NM_001167992 | PQBP1 | -0,77 | 5,57 | 4,68E-02 | * | NM_015104 | ATG2A | -1,61 | 4,51 | 2,65E-02 | * |
| NM_001168215 | TMEM92 | -4,31 | 1,02 | 3,02E-03 | * | NM_015180 | SYNE2 | -0,93 | 5,64 | 2,58E-02 | * |
| NM_001168364 | KRTCAP3 | -1,15 | 3,96 | 2,77E-02 | * | NM_015184 | PLCL2 | -6,66 | -0,35 | 2,80E-03 | * |
| NM_001170779 | FAM122C | -2,08 | 1,81 | 2,57E-02 | * | NM_015259 | ICOSLG | -1,71 | 2,90 | 4,73E-02 | * |
| NM_001170780 | FAM122C | -2,17 | 1,73 | 2,53E-02 | * | NM_015322 | FEM1B | -1,23 | 2,99 | 2,78E-02 | * |
| NM_001170781 | FAM122C | -2,06 | 2,84 | 1,43E-02 | * | NM_015456 | NELFB | -0,97 | 3,85 | 3,68E-02 | * |
| NM_001170782 | FAM122C | -2,19 | 1,74 | 2,42E-02 | * | NM_015547 | ACOT11 | -5,82 | -0,48 | 5,45E-04 | * |
| NM_001170783 | FAM122C | -2,19 | 1,74 | 2,46E-02 | * | NM_015566 | FAM169A | -1,15 | 3,88 | 2,92E-02 | * |
| NM_001170784 | FAM122C | -2,19 | 1,74 | 2,42E-02 | * | NM_015679 | TRUB2 | -0,65 | 5,48 | 3,32E-02 | * |
| NM_001171907 | CXorf40A | -1,13 | 3,15 | 4,05E-02 | * | NM_015698 | GPKOW | -0,87 | 4,70 | 3,97E-02 | * |
| NM_001171908 | CXorf40A | -1,13 | 3,15 | 4,05E-02 | * | NM_015945 | SLC35C2 | -1,42 | 3,42 | 3,39E-02 | * |
| NM_001171909 | CXorf40A | -1,02 | 3,32 | 3,76E-02 | * | NM_016032 | ZDHHC9 | -1,15 | 4,61 | 2,81E-02 | * |
| NM_001174092 | TMEM185A | -1,63 | 2,42 | 3,05E-02 | * | NM_016035 | COQ4 | -0,73 | 5,29 | 2,95E-02 | * |
| NM_001184792 | PARD3 | -0,67 | 5,24 | 4,56E-02 | * | NM_016120 | RLIM | -1,16 | 3,84 | 4,10E-02 | * |
| NM_001184793 | PARD3 | -0,67 | 5,24 | 4,56E-02 | * | NM_016176 | SDF4 | -0,97 | 4,10 | 3,39E-02 | * |
| NM_001184794 | PARD3 | -0,67 | 5,24 | 4,56E-02 | * | NM_016297 | PCYOX1 | -1,07 | 4,86 | 4,53E-02 | * |
| NM_001185117 | CLDN19 | -1,61 | 4,00 | 2,50E-02 | * | NM_016547 | SDF4 | -0,97 | 4,10 | 3,39E-02 | * |
| NM_001190860 | PLEKHA5 | -1,60 | 1,69 | 4,86E-02 | * | NM_016594 | FKBP11 | -1,26 | 3,49 | 3,95E-02 | * |
| NM_001193508 | REST | -0,61 | 6,46 | 4,29E-02 | * | NM_017436 | A4GALT | -0,85 | 5,60 | 2,33E-02 | * |
| NM_001195053 | DDIT3 | -1,01 | 5,05 | 2,84E-02 | * | NM_017515 | SLC35F2 | -1,22 | 4,12 | 4,97E-02 | * |
| NM_001195054 | DDIT3 | -1,01 | 5,05 | 2,84E-02 | * | NM_017793 | RPP25 | -0,89 | 5,79 | 2,51E-02 | * |
| NM_001195055 | DDIT3 | -1,01 | 5,05 | 2,84E-02 | * | NM_017877 | SLC35F6 | -1,10 | 3,81 | 3,68E-02 | * |
| NM_001195056 | DDIT3 | -1,01 | 5,05 | 2,84E-02 | * | NM_017941 | C17orf80 | -0,90 | 4,09 | 2,81E-02 | * |
| NM_001195057 | DDIT3 | -1,02 | 5,05 | 2,78E-02 | * | NM_018040 | GPATCH2 | -0,84 | 4,06 | 4,52E-02 | * |
| NM_001195622 | TOPORS | -0,97 | 3,99 | 2,38E-02 | * | NM_018064 | AKIRIN2 | -0,88 | 4,89 | 1,33E-02 | * |
| NM_001197244 | BCL7B | -0,91 | 4,53 | 2,68E-02 | * | NM_018121 | SLF2 | -0,95 | 3,76 | 3,41E-02 | * |
| NM_001199216 | MAG | -1,53 | 4,37 | 3,49E-02 | * | NM_018143 | KLHL11 | -1,10 | 3,26 | 4,35E-02 | * |
| NM_001199743 | DCTN5 | -0,63 | 5,87 | 4,02E-02 | * | NM_018169 | KIAA1551 | -0,85 | 6,53 | 3,64E-02 | * |
| NM_001205019 | GK | -2,43 | 2,39 | 2,14E-02 | * | NM_018243 | SEPT11 | -0,94 | 6,43 | 1,05E-02 | * |
| NM 001206866 | IL6R | -8,66 | -0,52 | 8,72E-09 | * | NM_018249 | CDK5RAP2 | -0,87 | 5,21 | 3,17E-02 | * |
| NM_001231 | CASQ1 | -4,89 | -0,63 | 2,57E-02 | * | NM_018330 | SHTN1 | -1,14 | 3,34 | 2,51E-02 | * |
| NM_001236 | CBR3 | -3,17 | 1,45 | 2,07E-03 | * | NM_018407 | LAPTM4B | -1,02 | 5,78 | 2,68E-02 | * |
| NM_001242559 | MAP4K4 | -0,92 | 4,62 | 1,36E-02 | * | NM_018950 | HLA-F | -4,23 | 0,29 | 1,12E-03 | * |
| NM_001242560 | MAP4K4 | -0,92 | 4,65 | 1,20E-02 | * | NM_019012 | PLEKHA5 | -1,38 | 3,30 | 3,07E-02 | * |
| NM_001242805 | BRDT | -0,90 | 5,64 | 1,70E-02 | * | NM_019593 | GPCPD1 | -0,90 | 6,56 | 1,64E-02 | * |
| NM_001242806 | BRDT | -0,89 | 5,59 | 1,67E-02 | * | NM_019850 | NGEF | -2,25 | 1,77 | 2,47E-02 | * |
| NM_001242807 | BRDT | -0,88 | 5,54 | 1,82E-02 | * | NM_020210 | SEMA4B | -1,88 | 2,67 | 2,68E-02 | * |
| NM_001242808 | BRDT | -0,89 | 5,59 | 1,68E-02 | * | NM_020211 | RGMA | -1,19 | 2,38 | 3,11E-02 | * |
| NM_001242810 | BRDT | -0,92 | 5,72 | 1,43E-02 | * | NM_020447 | FAM219B | -1,28 | 3,28 | 3,35E-02 | * |
| NM_001253891 | DGAT2 | -4,93 | 1,05 | 3,97E-03 | * | NM_020645 | NRIP3 | -1,15 | 4,42 | 2,46E-02 | * |
| NM_001256420 | MAPRE2 | -1,82 | 2,74 | 4,53E-02 | * | NM_020693 | DSCAML1 | -3,01 | 1,15 | 2,78E-02 | * |
| NM_001256470 | PLEKHA5 | -1,40 | 3,31 | 2,73E-02 | * | NM_020745 | AARS2 | -1,70 | 2,89 | 4,29E-02 | * |
| NM_001256647 | NR1H2 | -0,83 | 4,41 | 2,33E-02 | * | NM_020840 | FNIP2 | -1,09 | 4,57 | 1,90E-02 | * |
| NM_001256876 | KANK1 | -1,00 | 3,84 | 2,72E-02 | * | NM_020922 | WNK3 | -3,00 | 1,04 | 8,82E-03 | # |
| NM_001256877 | KANK1 | -1,00 | 3,91 | 2,14E-02 | * | NM_020925 | CACHD1 | -2,18 | 2,31 | 2,78E-02 | * |
| NM_001258025 | TRIM60 | -0,89 | 5,48 | 2,75E-02 | * | NM_021127 | PMAIP1 | -2,08 | 1,85 | 2,30E-02 | * |
| NM_001258298 | SHTN1 | -1,13 | 3,36 | 2,74E-02 | * | NM_021148 | ZNF273 | -1,14 | 2,90 | 4,28E-02 | * |
| NM_001258446 | TOP1MT | -0,89 | 4,59 | 2,38E-02 | * | NM_021260 | ZFYVE1 | -1,42 | 3,83 | 1,34E-02 | * |
| NM_001258447 | TOP1MT | -0,89 | 4,59 | 2,40E-02 | * | NM_021632 | ZNF350 | -1,26 | 3,86 | 1,38E-02 | * |
| NM_001271854 | GPR176 | -2,27 | 2,46 | 2,11E-02 | * | NM_021922 | FANCE | -1,17 | 3,78 | 2,26E-02 | * |
| NM_001271855 | GPR176 | -2,27 | 2,34 | 3,65E-02 | * | NM_021980 | OPTN | -1,67 | 3,18 | 1,53E-02 | * |
| NM_001272039 | CDK5RAP2 | -0,88 | 5,18 | 3,25E-02 | * | NM_022059 | CXCL16 | -1,47 | 1,27 | 2,96E-02 | * |
| NM_001276373 | USF1 | -1,79 | 2,27 | 3,68E-02 | * | NM_022152 | TMBIM1 | -1,33 | 5,16 | 2,65E-02 | * |
| NM_001278682 | TGIF1 | -0,73 | 4,97 | 3,64E-02 | * | NM_022464 | SIL1 | -1,04 | 4,51 | 2,86E-02 | * |
| NM_001278684 | TGIF1 | -0,74 | 5,02 | 3,62E-02 | * | NM_022467 | CHST8 | -1,61 | 3,09 | 1,20E-02 | * |
| NM_001278686 | TGIF1 | -0,73 | 4,97 | 3,75E-02 | * | NM_022770 | GINS3 | -0,86 | 4,78 | 2,23E-02 | * |
| NM_001281457 | SLC35C2 | -1,42 | 3,42 | 3,39E-02 | * | NM_022777 | IFT22 | -1,13 | 4,00 | 3,25E-02 | * |
| NM_001281458 | SLC35C2 | -1,42 | 3,42 | 3,39E-02 | * | NM_023007 | JMJD4 | -0,83 | 4,97 | 3,35E-02 | * |
| NM_001281459 | SLC35C2 | -1,42 | 3,42 | 3,39E-02 | * | NM_023080 | C8orf33 | -0,73 | 5,88 | 2,62E-02 | * |
| NM_001281460 | SLC35C2 | -1,42 | 3,43 | 3,39E-02 | * | NM_024111 | CHAC1 | -2,57 | 4,29 | 3,27E-02 | * |
| NM_001281492 | MSH6 | -1,05 | 5,74 | 1,43E-02 | * | NM_024415 | DDX4 | -5,76 | -0,60 | 3,97E-03 | * |
| NM_001281493 | MSH6 | -0,99 | 5,80 | 2,97E-02 | * | NM_024544 | MUL1 | -0,84 | 4,73 | 4,99E-02 | * |
| NM_001281494 | MSH6 | -1,05 | 5,77 | 1,42E-02 | * | NM_024669 | ANKRD55 | -3,31 | 0,39 | 1,03E-02 | * |
| NM_001281734 | ZFYVE1 | -1,42 | 3,83 | 1,34E-02 | * | NM_024772 | ZMYM1 | -0,96 | 4,35 | 2,67E-02 | * |
| NM_001281735 | ZFYVE1 | -1,46 | 3,14 | 1,33E-02 | * | NM_024785 | FAM124B | -8,08 | -1,02 | 1,56E-04 | * |
| NM_001282205 | SIGMAR1 | -1,39 | 3,12 | 2,26E-02 | * | NM_025009 | CEP135 | -1,40 | 2,56 | 3,39E-02 | * |
| NM_001282205 | SIGMAR1 | -1,38 | 3,13 | 2,40E-02 | * | NM_025078 | PQLC1 | -1,18 | 3,94 | 1,08E-02 | * |
| NM_001282207 | SIGMAR1 | -1,38 | 3,14 | 2,40E-02 | * | NM_025158 | RUFY1 | -0,92 | 4,83 | 4,84E-02 | * |
| NM_001282208 | SIGMAR1 | -1,38 | 3,14 | 2,40E-02 | * | NM_025232 | REEP4 | -1,11 | 4,31 | 3,49E-02 | * |
| NM_001282209 | SIGMAR1 | -1,36 | 3,11 | 2,51E-02 | * | NM_025245 | PBX4 | -1,73 | 1,83 | 3,61E-02 | * |
| NM_001282862 | RASGEF1A | -1,04 | 4,41 | 4,05E-02 | * | NM_030647 | KDM7A | -1,32 | 3,13 | 2,80E-02 | * |
| NM_001283052 | ICOSLG | -1,71 | 2,90 | 4,75E-02 | * | NM_030782 | CLPTM1L | -0,79 | 6,23 | 3,22E-02 | * |
| NM_001286233 | SLC2A14 | -1,61 | 3,61 | 3,27E-02 | * | NM_031412 | GABARAPL1 | -1,37 | 5,40 | 1,75E-02 | * |
| NM_001286242 | GTF3C1 | -1,09 | 3,64 | 2,56E-02 | * | NM_032300 | TCHP | -1,38 | 2,92 | 2,40E-02 | * |
| NM_001286277 | TUBGCP3 | -1,14 | 3,54 | 2,40E-02 | * | NM_032376 | TMEM101 | -1,47 | 2,76 | 2,97E-02 | * |
| NM_001286377 | MAEL | -1,39 | 4,78 | 4,79E-02 | * | NM_032382 | COG8 | -0, 68 | 5,48 | 2,51E-02 | * |
| NM_001286378 | MAEL | -1,39 | 4,78 | 4,79E-02 | * | NM_032486 | DCTN5 | -0,63 | 5,87 | 4,02E-02 | * |
| NM_001286509 | SLC35B2 | -0,98 | 3,66 | 3,69E-02 | * | NM_032498 | RHOXF2 | -1,92 | 5,81 | 2,51E-02 | * |
| NM_001286510 | SLC35B2 | -0,98 | 3,66 | 3,70E-02 | * | NM_032508 | TMEM185A | -1,63 | 2,43 | 3,05E-02 | * |
| NM_001286511 | SLC35B2 | -0,98 | 3,66 | 3,69E-02 | * | NM_032564 | DGAT2 | -4,93 | 1,05 | 3,97E-03 | * |
| NM_001286512 | SLC35B2 | -0,98 | 3,66 | 3,70E-02 | * | NM_032711 | MAFG | -1,18 | 3,95 | 2,11E-02 | * |
| NM_001286513 | SLC35B2 | -0,98 | 3,66 | 3,68E-02 | * | NM_032793 | MFSD2A | -6,97 | 2,20 | 2,12E-04 | * |
| NM_001286517 | SLC35B2 | -0,98 | 3,65 | 3,69E-02 | * | NM_032858 | MAEL | -1,39 | 4,78 | 4,79E-02 | * |
| NM_001286519 | SLC35B2 | -0,97 | 3,58 | 3,96E-02 | * | NM_032873 | UBASH3B | -1,17 | 4,61 | 1,50E-02 | * |
| NM_001285520 | SLC35B2 | -0,98 | 3,66 | 3,69E-02 | * | NM_033067 | DMRTB1 | -2,18 | 1,23 | 2,13E-02 | * |
| NM_001287525 | IFT22 | -1,13 | 4,00 | 3, 25 E-02 | * | NM_033412 | SLC25A51 | -1,22 | 3,00 | 4,29E-02 | * |
| NM_001287526 | IFT22 | -1,13 | 4,00 | 3,25E-02 | * | NM_033540 | MFN1 | -0,88 | 7,09 | 2,65E-02 | * |
| NM_001287808 | MFSD2A | -6,98 | 2,19 | 2,06E-04 | * | NM_052857 | ZNF830 | -0,86 | 3,84 | 2,92E-02 | * |
| NM_001287809 | MFSD2A | -6,97 | 2,20 | 2,12E-04 | * | NM_052943 | FAM46B | -1,04 | 5,73 | 2,72E-02 | * |
| NM_001288770 | C17orf80 | -0,75 | 4,64 | 1,86E-02 | * | NM_052963 | TOP1MT | -0,89 | 4,59 | 2,41E-02 | * |
| NM_001289088 | ZMYM1 | -0,96 | 4,35 | 2,58E-02 | * | NM_053052 | SNAP47 | -0,79 | 5,14 | 1,41E-02 | * |
| NM_001289089 | ZMYM1 | -0,96 | 4,35 | 2,67E-02 | * | NM_080600 | MAG | -1,53 | 4,37 | 3,47E-02 | * |
| NM_001289090 | ZMYM1 | -0,96 | 4,35 | 2,67E-02 | * | NM_133368 | RSPRY1 | -1,13 | 4,29 | 3,56E-02 | * |
| NM_001289091 | ZMYM1 | -0,96 | 4,35 | 2,65E-02 | * | NM_133375 | DIS3L | -1,06 | 4,27 | 3,31E-02 | * |
| NM_001290225 | PTDSS1 | -0,89 | 4,98 | 4,77E-02 | * | NM_138287 | DTX3L | -7,26 | -1,15 | 6,45E-03 | * |
| NM_001290693 | BECN2 | -5,13 | 0,72 | 3,79E-04 | * | NM_138330 | ZNF675 | -0,97 | 4,24 | 3,65E-02 | * |
| NM_001291163 | EPHX1 | -1,75 | 1,06 | 2,81E-02 | * | NM_138387 | G6PC3 | -1,10 | 3,18 | 3,81E-02 | * |
| NM_001291311 | PLD3 | -1,44 | 3,73 | 2,03E-02 | * | NM_138574 | HDGFL1 | -1,49 | 2,92 | 2,00E-02 | * |
| NM_001293274 | CACHD1 | -2,18 | 2,22 | 2,57E-02 | * | NM_138819 | FAM122C | -2,08 | 1,81 | 2,60E-02 | * |
| NM_001297714 | SWAP70 | -0,76 | 4,95 | 2,38E-02 | * | NM_139276 | STAT3 | -1,69 | 3,60 | 2,25E-02 | * |
| NM_001300826 | RNF19B | -1,24 | 3,64 | 2,22E-02 | * | NM_144495 | PQBP1 | -0,77 | 5,64 | 4,82E-02 | * |
| NM_001301061 | BCL7B | -0,91 | 4,53 | 2,68E-02 | * | NM_144653 | NACC2 | -1,53 | 3,01 | 1,52E-02 | * |
| NM_001302959 | AIP | -1,27 | 2,84 | 4,44E-02 | * | NM_145040 | CAVIN3 | -3,92 | 1,34 | 2,43E-03 | * |
| NM_001302960 | AIP | -1,28 | 2,85 | 4,11E-02 | * | NM_145313 | RASGEF1A | -1,04 | 4,41 | 4,05E-02 | * |
| NM_001303465 | TAZ | -1,30 | 3,34 | 4,77E-02 | * | NM_145686 | MAP4K4 | -0,92 | 4,66 | 1,28E-02 | * |
| NM_001303627 | MLEC | -0,94 | 5,47 | 1,95E-02 | * | NM_145687 | MAP4K4 | -0,92 | 4,62 | 1,36E-02 | * |
| NM_001303628 | MLEC | -0,95 | 5,55 | 1,46E-02 | * | NM_147157 | SIGMAR1 | -1,37 | 3,12 | 2,47E-02 | * |
| NM_001304813 | TMEM101 | -1,47 | 2,76 | 2,93E-02 | * | NM_148960 | CLDN19 | -1,56 | 3,59 | 3,21E-02 | * |
| NM_001304814 | TMEM101 | -1,47 | 2,78 | 2,51E-02 | * | NM_152373 | ZNF684 | -1,18 | 2,54 | 4,22E-02 | * |
| NM_001305163 | RSPRY1 | -1,13 | 4,29 | 3,46E-02 | * | NM_152620 | TRIM60 | -0,89 | 5,47 | 2,80E-02 | * |
| NM_001305164 | RSPRY1 | -1,13 | 4,29 | 3,56E-02 | * | NM_153229 | TMEM92 | -4,31 | 1,02 | 3,02E-03 | * |
| NM_001305182 | RSPRY1 | -1,65 | 1,98 | 2,29E-02 | * | NM_153256 | PROSER2 | -1,39 | 3,55 | 1,14E-02 | * |
| NM_001305942 | COQ4 | -0,72 | 5,27 | 3,05E-02 | * | NM_153341 | RNF19B | -1,24 | 3,64 | 2,22E-02 | * |
| NM_001306147 | SEPT11 | -0,94 | 6,43 | 1,06E-02 | * | NM_153449 | SLC2A14 | -1,62 | 3,60 | 3,27E-02 | * |
| NM_001307945 | CHRNA5 | -1,77 | 2,12 | 1,86E-02 | * | NM_153768 | CABYR | -1,66 | 3,22 | 4,83E-02 | * |
| NM_001316964 | REEP4 | -1,11 | 4,31 | 3,54E-02 | * | NM_170695 | TGIF1 | -0,74 | 4,98 | 3,06E-02 | * |
| NM_001316965 | REEP4 | -1,11 | 4,31 | 3,49E-02 | * | NM_173073 | SLC35C2 | -1,42 | 3,42 | 3,39E-02 | * |
| NM_001317056 | ATG9B | -2,04 | 3,14 | 4,22E-02 | * | NM_173179 | SLC35C2 | -1,42 | 3,43 | 3,39E-02 | * |
| NM_001318038 | A4GALT | -0,86 | 5,57 | 2,33E-02 | * | NM_173207 | TGIF1 | -0,74 | 4,97 | 3,49E-02 | * |
| NM 001318144 | AQP3 | -1,11 | 6,31 | 3,69E-02 | * | NM_173208 | TGIF1 | -0,74 | 5,02 | 3,61E-02 | * |
| NM_001318370 | SLC35E4 | -1,56 | 4,44 | 1,45E-02 | * | NM_173209 | TGIF1 | -0,72 | 4,97 | 3,90E-02 | * |
| NM_001318371 | SLC35E4 | -1,57 | 4,39 | 1,42E-02 | * | NM_173210 | TGIF1 | -0,74 | 4,98 | 3,29E-02 | * |
| NM_001318900 | GLUD1 | -0,84 | 6,57 | 1,70E-02 | * | NM_173211 | TGIF1 | -0,73 | 4,97 | 3,65E-02 | * |
| NM_001318901 | GLUD1 | -0,84 | 6,58 | 1,56E-02 | * | NM_173344 | ST3GAL1 | -1,30 | 3,87 | 3,13E-02 | * |
| NM_001318902 | GLUD1 | -0,84 | 6,57 | 1,71E-02 | * | NM_173853 | KRTCAP3 | -1,15 | 3,92 | 3,68E-02 | * |
| NM_001318904 | GLUD1 | -0,78 | 6,63 | 2,85E-02 | * | NM_174886 | TGIF1 | -0,73 | 4,97 | 3,69E-02 | * |
| NM_001318905 | GLUD1 | -0,78 | 6,64 | 2,67E-02 | * | NM_177526 | PLPP2 | -2,52 | 4,08 | 1,53E-02 | * |
| NM_001318906 | GLUD1 | -0,78 | 6,63 | 2,84E-02 | * | NM_177543 | PLPP2 | -2,52 | 4,08 | 1,53E-02 | * |
| NM_001319945 | G6PC3 | -1,09 | 3,18 | 3,83E-02 | * | NM_178124 | CXorf40A | -1,13 | 3,15 | 4,05E-02 | * |
| NM_001319955 | ZMYM1 | -0,97 | 4,33 | 2,53E-02 | * | NM_178148 | SLC35B2 | -0,98 | 3,66 | 3,69E-02 | * |
| NM_001321325 | KRTCAP3 | -1,15 | 3,92 | 3,68E-02 | * | NM_178441 | ZFYVE1 | -1,46 | 3,14 | 1,33E-02 | * |
| NM_001321427 | TMBIM1 | -1,33 | 5,16 | 2,65E-02 | * | NM_181311 | TAZ | -1,30 | 3,34 | 4,79E-02 | * |
| NM_001321428 | TMBIM1 | -1,33 | 5,16 | 2,65E-02 | * | NM_181312 | TAZ | -1,30 | 3,34 | 4,77E-02 | * |
| NM_001321429 | TMBIM1 | -1,33 | 5,16 | 2,65E-02 | * | NM_181313 | TAZ | -1,30 | 3,34 | 4,78E-02 | * |
| NM_001321430 | TMBIM1 | -1,33 | 5,16 | 2,65E-02 | * | NM_181359 | IL6R | -5,25 | 0,58 | 3,74E-03 | * |
| NM_001321432 | TMBIM1 | -1,33 | 5,16 | 2,65E-02 | * | NM_182810 | ATF4 | -0,81 | 7,16 | 1,26E-02 | * |
| NM_001321433 | TMBIM1 | -1,33 | 5,16 | 2,60E-02 | * | NM_182914 | SYNE2 | -0,93 | 5,64 | 2,58E-02 | * |
| NM_001321435 | TMBIM1 | -1,33 | 5,16 | 2,65E-02 | * | NM_183353 | RLIM | -1,16 | 3,84 | 4,11E-02 | * |
| NM_001321436 | TMBIM1 | -1,33 | 5,16 | 2,65E-02 | * | NM_198925 | SEMA4B | -1,88 | 2,67 | 2,67E-02 | * |
| NM_001321438 | TMBIM1 | -1,33 | 5,16 | 2,65E-02 | * | NM_198926 | FAM212B | -3,11 | 0,33 | 2,87E-02 | * |
| NM_001321920 | FAM219B | -1,26 | 3,14 | 3,49E-02 | * | NM_198969 | AES | -2,21 | 1,70 | 1,71E-02 | * |
| NM_001321922 | FAM219B | -1,28 | 3,28 | 3,35E-02 | * | NM_198970 | AES | -2,20 | 1,69 | 1,79E-02 | * |
| NM_001321923 | FAM219B | -1,26 | 3,14 | 3,49E-02 | * | NM_201441 | TEAD4 | -0,66 | 5,74 | 4,05E-02 | * |
| NM_001322379 | ENTPD6 | -0,83 | 4,59 | 1,33E-02 | * | NM_201443 | TEAD4 | -0,67 | 5,71 | 3,62E-02 | * |
| NM_001322380 | ENTPD6 | -0,83 | 4,59 | 1,33E-02 | * | NM_203391 | GK | -2,44 | 2,38 | 2,15E-02 | * |
| NM_001322381 | ENTPD6 | -0,82 | 4,59 | 1,38E-02 | * | NM_207005 | USF1 | -1,81 | 2,25 | 3,49E-02 | * |
| NM_001322382 | ENTPD6 | -0,83 | 4,59 | 1,38E-02 | * | NM_207111 | RNF216 | -1,05 | 4,30 | 2,26E-02 | * |
| NM_001322384 | ENTPD6 | -0,83 | 4,59 | 1,33E-02 | * | NM_207116 | RNF216 | -1,05 | 4,30 | 2,27E-02 | * |
| NM_001323916 | FNIP2 | -1,09 | 4,57 | 1,92E-02 | * | NM_207189 | BRDT | -0,89 | 5,59 | 1,67E-02 | * |
| NM_001323930 | SNAP47 | -0,80 | 4,93 | 1,88E-02 | * | NM_213662 | STAT3 | -1,69 | 3,60 | 2,25E-02 | * |
| NM_001323931 | SNAP47 | -0,79 | 5,14 | 1,40E-02 | * | NR_003099 | ZNF273 | -1,14 | 2,90 | 4,27E-02 | * |
| NM_001323932 | SNAP47 | -0,79 | 5,01 | 1,95E-02 | * | NR_024048 | TAZ | -1,30 | 3,34 | 4,79E-02 | * |
| NM_001323933 | SNAP47 | -0,79 | 5,01 | 1,95E-02 | * | NR_024123 | PBX3 | -1,58 | 2,37 | 3,96E-02 | * |
| NM_001323934 | SNAP47 | -0,80 | 4,93 | 1,88E-02 | * | NR_027783 | SAT1 | -1,57 | 2,97 | 4,05E-02 | * |
| NM_001323935 | SNAP47 | -0,82 | 4,79 | 1,80E-02 | * | NR_028500 | LDHA | -0,43 | 9,05 | 2,56E-02 | * |
| NM_001323936 | DIS3L | -1,06 | 4,27 | 3,31E-02 | * | NR 028581 | G6PC3 | -1,09 | 3,18 | 3,73E-02 | * |
| NM_001323937 | DIS3L | -1,06 | 4,27 | 3,29E-02 | * | NR_036682 | BCL7B | -0,91 | 4,52 | 2,79E-02 | * |
| NM_001323938 | DIS3L | -1,06 | 4,27 | 3,39E-02 | * | NR_037148 | DNAJC25 | -1,20 | 3,62 | 2,41E-02 | * |
| NM_001323939 | DIS3L | -1,06 | 4,27 | 3,39E-02 | * | NR_037573 | DCTN5 | -0,63 | 5,87 | 3,98E-02 | * |
| NM_001323940 | DIS3L | -1,06 | 4,27 | 3,27E-02 | * | NR_037646 | TMX2-CTNND1 | -0,63 | 5,64 | 1,70E-02 | * |
| NM_001323941 | DIS3L | -1,05 | 4,28 | 3,46E-02 | * | NR_038198 | PBX4 | -1,70 | 1,80 | 4,11E-02 | * |
| NM_001323943 | DIS3L | -1,06 | 4,27 | 3,27E-02 | * | NR_046177 | MAPRE2 | -1,82 | 2,73 | 4,54E-02 | * |
| NM_001323944 | DIS3L | -1,06 | 4,27 | 3,39E-02 | * | NR_046462 | FAM169A | -1,15 | 3,88 | 3,02E-02 | * |
| NM_001323945 | DIS3L | -1,05 | 4,28 | 3,46E-02 | * | NR_046633 | NUCB1-AS1 | -1,22 | 3,12 | 3,61E-02 | * |
| NM_001323946 | DIS3L | -1,06 | 4,27 | 3,39E-02 | * | NR_047549 | SLC25A12 | -2,36 | 1,45 | 3,46E-02 | * |
| NM_001323948 | DIS3L | -1,06 | 4,27 | 3,39E-02 | * | NR_073169 | ATG9B | -1,71 | 4,48 | 3,89E-02 | * |
| NM_001324029 | SEMA4B | -1,89 | 2,66 | 2,67E-02 | * | NR_073554 | CDK5RAP2 | -0,87 | 5,21 | 3,19E-02 | * |
| NM_001324030 | SEMA4B | -1,88 | 2,67 | 2,71E-02 | * | NR_102404 | NBPF8 | -1,24 | 4,08 | 4,83E-02 | * |
| NM_001324031 | SEMA4B | -1,88 | 2,67 | 2,67E-02 | * | NR_103546 | SPECC1L-ADORA2A | -0,97 | 4,11 | 4,37E-02 | * |
| NM_001324032 | SEMA4B | -1,88 | 2,67 | 2,67E-02 | * | NR_104108 | SIGMAR1 | -1,38 | 3,13 | 2,35E-02 | * |
| NM_001324033 | SEMA4B | -1,88 | 2,67 | 2,67E-02 | * | NR_135643 | TMBIM1 | -1,39 | 5,18 | 1,72E-02 | * |
| NM_001324034 | SEMA4B | -1,88 | 2,67 | 2,67E-02 | * | NR_135853 | FAM219B | -1,27 | 3,14 | 3,47E-02 | * |
| NM_001324274 | CXorf40A | -1,13 | 3,12 | 3,99E-02 | * | NR_152596 | SNHG15 | -1,05 | 6,30 | 2,05E-02 | * |
| NM_001324275 | CXorf40A | -1,13 | 3,12 | 3,99E-02 | * | **(P = Preferred): * = at least one of these genes** | | | | | |
| NM_001324276 | CXorf40A | -1,02 | 3,36 | 3,81E-02 | * | | | | | | |

Table F lists genes that have been found to be stably expressed at substantially the same level across all trophectoderm samples. The genes in the table are ordered on the basis of the magnitude of the coefficient of variation.

Since these genes are expressed at a substantially constant level in all trophectoderm samples, the level of expression any of these genes can be used as a baseline, against which, any differential in the level of expression of the genes of Table A and/or B and/or C and/or D can be determined.

**Table F**

| **refSeq ID** | **gene name** | **CV** | **refSeq ID** | **gene name** | **CV** | **refSeq ID** | **gene name** | **CV** |
|---|---|---|---|---|---|---|---|---|
| NM_000269 | NME1 | 0,173 | NM_001202431 | PRDX1 | 0,148 | NM_012459 | TIMM8B | 0,149 |
| NM_000969 | RPL5 | 0,165 | NM_001202548 | SYNJ2BP-COX16 | 0,177 | NM_013238 | DNAJC15 | 0,171 |
| NM_000973 | RPL8 | 0,166 | NM_001203262 | NDUFC2-KCTD14 | 0,144 | NM_013387 | UQCR10 | 0,171 |
| NM_000978 | RPL23 | 0,160 | NM_001204054 | NDUFC2 | 0,136 | NM_014047 | C19orf53 | 0,165 |
| NM_000986 | RPL24 | 0,135 | NM_001204083 | MINOS1 | 0,176 | NM_014206 | TMEM258 | 0,131 |
| NM_000989 | RPL30 | 0,166 | NM_001204858 | ELOC | 0,157 | NM_014402 | UQCRQ | 0,172 |
| NM_000990 | RPL27A | 0,166 | NM_001244938 | TXN | 0,128 | NM_015932 | POMP | 0,123 |
| NM_000995 | RPL34 | 0,163 | NM_001256802 | RPS3 | 0,141 | NM_016167 | NOL7 | 0,178 |
| NM_000996 | RPL35A | 0,136 | NM_001270481 | PSMB6 | 0,164 | NM_016200 | LSM8 | 0,166 |
| NM_000997 | RPL37 | 0,147 | NM_001271665 | NSA2 | 0,159 | NM_016497 | MRPL51 | 0,171 |
| NM_000998 | RPL37A | 0,149 | NM_001286273 | TPT1 | 0,166 | NM_017838 | NHP2 | 0,176 |
| NM_000999 | RPL38 | 0,130 | NM_001286524 | CENPW | 0,157 | NM_017971 | MRPL20 | 0,111 |
| NM_001001 | RPL36AL | 0,158 | NM_001288609 | JPT1 | 0,172 | NM_018648 | NOP10 | 0,179 |
| NM_001002 | RPLP0 | 0,175 | NM_001291916 | SNRPD1 | 0,163 | NM_018848 | MKKS | 0,157 |
| NM_001003 | RPLP1 | 0,170 | NM_001297565 | UQCRH | 0,142 | NM_019059 | TOMM7 | 0,176 |
| NM_001007073 | RPL32 | 0,137 | NM_001297769 | MRPS18C | 0,178 | NM_021128 | POLR2L | 0,150 |
| NM_001008391 | CCDC73 | 0,154 | NM_001300992 | EIF3K | 0,155 | NM_022061 | MRPL17 | 0,149 |
| NM_001009 | RPS5 | 0,170 | NM_001315530 | RPL26 | 0,142 | NM_024026 | MRPL57 | 0,168 |
| NM_001010 | RPS6 | 0,122 | NM_001318794 | COX411 | 0,127 | NM_031287 | SF3B5 | 0,135 |
| NM_001012 | RPS8 | 0,127 | NM_001321018 | UBA52 | 0,142 | NM_032747 | ATP5MD | 0,180 |
| NM_001015 | RPS11 | 0,168 | NM_001328637 | SNRPE | 0,169 | NM_033643 | RPL36 | 0,141 |
| NM_001017 | RPS13 | 0,147 | NM_001329417 | TMA7 | 0,167 | NM_079423 | MYL6 | 0,179 |
| NM_001018 | RPS15 | 0,149 | NM_001330200 | RPL19 | 0,131 | NM_080748 | ROMO1 | 0,174 |
| NM_001018136 | NME1-NME2 | 0,167 | NM_001349610 | CAMTA1 | 0,174 | NM_138820 | HIGD2A | 0,177 |
| NM_001018138 | NME2 | 0,173 | NM_001862 | COX5B | 0,132 | NM_181514 | MRPL21 | 0,174 |
| NM_001019 | RPS15A | 0,165 | NM_001959 | EEF1B2 | 0,167 | NR_015434 | LOC148413 | 0,154 |
| NM_001020 | RPS16 | 0,152 | NM_001997 | FAU | 0,127 | NR_027406 | LOC100129034 | 0,162 |
| NM_001022 | RPS19 | 0,125 | NM_002106 | H2AFZ | 0,153 | NR_029466 | COX7A2 | 0,179 |
| NM_001025 | RPS23 | 0,131 | NM_002489 | NDUFA4 | 0,178 | NR_030342 | MIR611 | 0,153 |
| NM_001032 | RPS29 | 0,162 | NM_002491 | NDUFB3 | 0,169 | NR_031695 | MIR1282 | 0,123 |
| NM_001048241 | UBL5 | 0,162 | NM_003134 | SRP14 | 0,155 | NR_033743 | ATP5ME | 0,170 |
| NM_001089591 | UQCRHL | 0,174 | NM_004374 | COX6C | 0,162 | NR_033759 | ATP5MG | 0,156 |
| NM_001099693 | RPL31 | 0,156 | NM_004552 | NDUFS5 | 0,165 | NR_037673 | SERF2-C15ORF63 | 0,119 |
| NM_001126063 | KHDC1L | 0,161 | NM_004597 | SNRPD2 | 0,121 | NR_037930 | SLMO2-ATP5E | 0,140 |
| NM_001134484 | TOMM5 | 0,161 | NM_004894 | ATP5MPL | 0,137 | NR_039624 | MIR4426 | 0,125 |
| NM_001134493 | TOMM6 | 0,153 | NM_005022 | PFN1 | 0,176 | NR 052025 | SLIRP | 0,142 |
| NM_001134693 | OST4 | 0,158 | NM_005274 | GNG5 | 0,146 | NR_102265 | LINC02067 | 0,122 |
| NM_001142282 | RPS24 | 0,152 | NM_005617 | RPS14 | 0,130 | NR_103819 | SNRPD3 | 0,167 |
| NM_001143985 | BANF1 | 0,139 | NM_005870 | SAP18 | 0,168 | NR_125371 | POLR2F | 0,168 |
| NM_001160246 | RPAIN | 0,174 | NM_006156 | NEDD8 | 0,129 | NR_132980 | SNORD141A | 0,175 |
| NM_001177413 | RPS27A | 0,123 | NM_005713 | SUB1 | 0,150 | NR_132981 | SNORD141B | 0,175 |
| NM_001193557 | ENY2 | 0,176 | NM_006808 | SEC61B | 0,171 | NR_137631 | NEDD8-MDP1 | 0,127 |
| NM_001199802 | RPL11 | 0,173 | NM_007104 | RPL10A | 0,146 | NR_138023 | AGBL5 | 0,158 |
| NM_001199877 | SERF2 | 0,126 | NM_007209 | RPL35 | 0,142 | NR_146327 | RPL27 | 0,122 |
| NM_001199987 | NDUFB6 | 0,161 | NM_012456 | TIMM10 | 0,159 | | | |

Considering that increasing maternal age could reduce implantation success rate and embryo survival (Hull et al., 1996), the biomarkers reported herein may be used in the context of maternal age, to select blastocysts with a greater potential to implant into the uterus, especially in the transition maternal age between 30 and 40 years. The current methodology employed a high throughput approach (RNA sequencing), illustrating the use of one of the methods suggested in the embodiments of the current invention, which could be used on a clinical basis for the selection of blastocysts with greater implantation potential. This approach was based on the use of at least 1 crucial YMA/rba-YMA or AMA/rba-AMA molecular biomarkers and/or one or more subsets of certain biomarkers and/or the positive correlation of the test sample with all, or part, of the YMA/rba-YMA biomarkers and/or all, or part, of the negative correlation of the test sample with the AMA/rba-AMA biomarkers and/or all, or part, of the combination of both, using references with gene expression profiles similar to YMA/rba-YMA and/or AMA/rba-AMA and/or sample(s) of average gene expression in the population and/or similar to the rba-IMA levels.

### Example 2

### Molecular biomarkers and embryo implantation success

The current invention employed blastocyst samples that derived from assisted reproductive technology for the determination of the trophectoderm transcriptome profiles in the context of maternal age and embryo implantation potential. In particular, embryo implantation success was determined by elevated levels of beta-human chorionic gonadotropin (beta-hCG) and by ultrasound scan to detect the appearance of fetal sac or fetal heartbeat. When trophectoderm samples are clustered based on the reproductive biological maternal age, all of the transferred rba-YMA blastocysts (n=13/13) and only half of the rba-AMA blastocysts (n=4/8) implanted into the uterus. The results correspond to statistically significant differences between the rba-YMA and rba-AMA groups with respect to their corresponding implantation success (two-tailed Fisher's test, p=0.012), indicating that the transcriptome profiles of the rba-YMA samples are associated with successful blastocyst implantation and/or successful embryo development, allowing the determination of the blastocysts with a greater likelihood to implant into the uterus. Trophectoderm samples clustered within rba-AMA blastocysts, on the other hand, showed a significantly reduced likelihood to implant into the uterus. Taken together, the YMA/rba-YMA and AMA/rba-AMA biomarkers reported in Tables A-D, may be used to provide an indication of the likelihood of a successful implantation process, also supporting a biological age classification efficacy based on reproductive biological maternal age.

### Example 3

### Functional annotation analysis and candidate biomarkers for maternal-fetal communication

The computational analysis that considered the coding trophectoderm transcripts, revealed biomarkers for implantation success in the transcriptome profiles of the rba-YMA and rba-AMA samples. Functional annotation analysis of these transcripts was performed in each group to reveal classes of biomarkers that could be enriched in certain biological processes and/or cellular components. In particular, functional annotation enrichment of rba-YMA and rba-AMA samples revealed biological processes, including cholesterol biosynthesis and mitochondrial ATP synthesis with FDR of 3.1E-12 and 4.8E-04, respectively, although extracellular exosome cellular component reported extremely significant enrichment (FDR 1.7E-17), and the factors involved in extracellular exosomes have a greater potential to functionally participate in embryo-endometrial communication, supporting the implantation process with greater confidence (Table 3). The rba-AMA transcripts on the other hand, did not reveal any statistically significant factors, despite the trend of apoptosis-related ontologies, however, apoptotic related biomarkers are reported (Table E4).

**Table E1. Exosome-related unique genes**

| **Gene name** | **P** | **Gene name** | **P** | **Gene name** | **P** | **Gene name** | **P** | **Gene name** | **P** | **Gene name** | | **Gene name** | **P** | **Gene name** | **P** |
|---|---|---|---|---|---|---|---|---|---|---|---|---|---|---|---|
| ACAT2 | * | CAST | * | ECHS1 | * | GSTA3 | * | MDH2 | * | PRDX2 | * | SCARB1 | * | TMEM256 | * |
| ACLY | * | CD274 | # | EEF1E1 | * | HEBP1 | * | MVK | * | PRDX5 | * | SCIN | * | TMPRSS2 | # |
| ACSL4 | * | CD53 | * | ENTPD1 | * | HEBP2 | * | MYL6B | * | PRDX6 | * | SELENOP | * | TNIK | * |
| ANXA6 | * | CISD1 | * | EPS8L2 | * | HINT1 | * | NANS | * | PSMA2 | * | SH3BGRL2 | * | TPM4 | # |
| ANXA8L1 | * | CNDP2 | * | ETFA | * | HSP90AA1 | * | NDRG2 | * | PSMA5 | * | SH3GLB2 | * | TSPAN15 | * |
| ARHGAP1 8 | * | COBLL1 | * | FABP3 | # | HSPB11 | * | NDUFB4 | * | PSMA7 | * | SLC13A3 | * | TUBA1B | * |
| ASAH1 | # | COMT | * | FABP5 | * | IFITM3 | * | NQO1 | * | PTGES3 | * | SLC15A2 | * | TXNDC17 | * |
| ATP5F1C | * | COX5A | * | FASN | * | ITGB1BP1 | * | PAXX | * | PTGR1 | * | SLC25A1 | * | UBB | * |
| ATP5PB | * | CTDSPL | * | FNBP1L | * | KRT18 | * | PDCD2 | * | QPRT | * | SLC38A1 | * | UCHL3 | * |
| ATP5PO | * | CYB5A | * | FTL | * | KRT19 | * | PDXDC1 | * | RAB11A | * | SNX5 | * | UFC1 | * |
| ATP6V1B1 | * | DAB2 | * | GDPD3 | * | KRT8 | * | PDXK | * | RAB25 | * | STK10 | * | WLS | * |
| C11orf52 | * | DBI | # | GGCT | * | LARP1 | * | PEBP1 | * | RAB3B | * | SUCLG1 | * | WNT7A | * |
| CACYBP | * | DCXR | * | GM2A | * | LCP1 | * | PKP3 | * | RHOBTB3 | * | TAGLN2 | * | | |
| CALD1 | * | DSTN | * | GNAS | * | LRP2 | * | PPME1 | * | S100A10 | * | TALDO1 | * | | |
| CALM2 | * | DYSF | * | GRHPR | * | LRRFIP1 | * | PPP1R13L | * | S100A13 | * | TKT | * | | |

**Table E2. Mouse knockout unique genes**

| **Gene name** | **P** | **Gene name** | **P** | **Gene name** | **P** | **Gene name** | **P** | **Gene name** | **P** | **Gene name** | **P** |
|---|---|---|---|---|---|---|---|---|---|---|---|
| ACAT2 | * | DAB2 | * | ECHS1 | * | GPX4 | * | LRP2 | * | PRDX2 | * |
| APOC3 | * | DBI | # | GNAS | * | HINT1 | * | PDCD2 | * | WLS | * |

**Table E3. Embryo-endometrial communication unique genes**

| **Gene name** | **P** | **Gene name** | **P** | **Gene name** | **P** | **Gene name** | **P** | **Gene name** | **P** | **Gene name** | **P** | **Gene name** | **P** |
|---|---|---|---|---|---|---|---|---|---|---|---|---|---|
| ACAT2 | * | CAST | * | EEF1E1 | * | KRT18 | * | NQO1 | * | RAB11A | * | TALDO1 | * |
| ACLY | * | CD274 | # | ETFA | * | KRT19 | * | PEBP1 | * | RAB3B | * | TKT | * |
| ANXA6 | * | CD53 | * | FABP3 | # | KRT8 | * | PKP3 | * | RHOBTB3 | * | TNIK | * |
| ARHGAP18 | * | CNDP2 | * | FABP5 | * | LCP1 | * | PPME1 | * | S100A10 | * | TPM4 | # |
| ASAH1 | # | COX5A | * | FASN | * | LRP2 | * | PPP1R13L | * | SCIN | * | TSPAN15 | * |
| ATP5F1C | * | CTDSPL | * | FTL | * | LRRFIP1 | * | PRDX5 | * | SELENOP | * | TUBA1B | * |
| ATP5PO | * | DAB2 | * | GNAS | * | MDH2 | * | PSMA2 | * | SH3GLB2 | * | UBB | * |
| ATP6V1B1 | * | DBI | # | HINT1 | * | MVK | # | PSMA5 | * | SLC25A1 | * | UCHL3 | * |
| CACYBP | * | DSTN | * | HSP90AA1 | * | MYL6B | * | PSMA7 | * | SNX5 | * | UFC1 | * |
| CALD1 | * | DYSF | * | IFITM3 | * | NANS | * | PTGES3 | * | SUCLG1 | * | WLS | * |
| CALM2 | * | ECHS1 | * | ITGB1BP1 | * | NDUFB4 | * | PTGR1 | * | TAGLN2 | * | WNT7A | * |

**Table E4. Apoptosis-related unique genes**

| **Gene name** | **P** | **Gene name** | **P** | **Gene name** | **P** | **Gene name** | **P** | **Gene name** | **P** | **Gene name** | **P** | **Gene name** | **P** |
|---|---|---|---|---|---|---|---|---|---|---|---|---|---|
| ATF4 | * | BAK1 | # | FNIP2 | * | MCL1 | * | OPTN | * | RB1CC1 | * | ZFYVE1 | * |
| ATG2A | * | BECN2 | * | GABARAPL1 | * | MFN1 | * | PI4KB | * | TOPORS | * | | |
| ATG3 | * | CHAC1 | * | GK | * | MSH6 | * | PMAIP1 | * | ULK2 | * | | |
| ATG9B | * | DDIT3 | * | MAEL | * | MTMR14 | * | PPP1R15A | * | WDR45 | * | | |

**Table E5. Important biomarkers present at the protein level in the blastocyst fluid following blastocentesis**

| **Gene name** | **P** | **Gene name** | **P** | **Gene name** | **P** | **Gene name** | **P** |
|---|---|---|---|---|---|---|---|
| FABP5 | * | PRDX2 | * | PSMA7 | * | TAGLN2 | * |
| HSP90AA1 | * | PSMA5 | * | SESN2 | * | | |

Based on the above, the factors reported in Table E1 provide an overview of the extracellular exosome factors that could be involved in the biological mechanism underpinning the implantation process. These biomarkers indicate potential functionality upon embryo-endometrial communication/interactions, through a novel route described by an age-related subset of human exosomal embryonic biomarkers, that could predict blastocyst implantation into the uterus with higher likelihood. In a different approach, extracellular exosome biomarkers were also tested against known knockout mice phenotypes and those that could compromise embryo implantation and/or development were reported (Table E2).

**Table 3**

| **rbaYMA DE gene transcripts - Reproductive biological age** | | | | | |
|---|---|---|---|---|---|
| **Biological Process** | | | **Cellular Component** | | |
| **Ontological Term** | **P-Value** | **FDR** | **Ontological Term** | **P-Value** | **FDR** |
| cholesterol biosynthetic process | 1.9E-15 | 3.1E-12 | extracellular exosome | 1.3E-20 | 1.7E-17 |
| mitochondrial ATP synthesis coupled proton transport | 3.0E-7 | 4.8E-4 | cytosol | 2.7E-6 | 3.5E-3 |
| isoprenoid biosynthetic process | 8.7E-7 | 1.4E-3 | mitochondrial inner membrane | 5.1E-6 | 6.6E-3 |
| ATP synthesis coupled proton transport | 1.0E-5 | 1.7E-2 | mitochondrial proton-transporting ATP synthase complex | 6.oE-6 | 7.8E-3 |
| ATP biosynthetic process | 4.4E-5 | 7.1E-2 | mitochondrion | 2.0E-5 | 2.6E-2 |
| lipid metabolic process | 6.6E-5 | 1.1E-1 | cell-cell adherens junction | 8.6E-5 | 1.1E-1 |
| cell-cell adhesion | 1.1E-4 | 1.7E-1 | mitochondrial matrix | 9.8E-5 | 1.3E-1 |
| | | | | | |

| **rbaAMA DE gene transcripts - Reproductive biological age** | | | | | |
|---|---|---|---|---|---|
| **Biological Process** | | | **Cellular Component** | | |
| **Ontological Term** | **P-Value** | **FDR** | **Ontological Term** | **P-Value** | **FDR** |
| autophagosome assembly | 5.0E-5 | 7.9E-2 | mitochondrial outer membrane | 3.3E-3 | 4.2E0 |
| Macroautophagy | 1.1E-4 | 1.7E-1 | pre-autophagosomal structure membrane | 1.0E-2 | 1.2E1 |
| intrinsic apoptotic signaling pathway in response to endoplasmic reticulum stress | 3.0E-4 | 4.8E-1 | pre-autophagosomal structure | 1.4E-2 | 1.7E1 |

Following on from the functional annotation analysis, a computational methodology was developed to investigate the molecular maternal-fetal interactions in the context of a receptive endometrium (Figure 1). This approach considered either the YMA/rba-YMA or the AMA/rba-AMA trophectoderm transcripts involved in cellular components associated with the extracellular exosome and/or cell adhesion, with the maternally-derived (receptive and/or non-/pre-receptive endometria) cellular components, including among others the extracellular exosome, extracellular space and cell surface, which indicate that both the maternal and fetal biomarkers derive from cellular regions with potential physical interactions and/or shared common pathways between the embryo and the endometrium, as investigated by Cytoscape GeneMANIA module. This process revealed 77 unique genes (Table E3), which suggest further increase in the likelihood that these trophectoderm factors may physically interact with the endometrial factors when they are in close proximity upon the apposition and adhesion processes. The reported biomarkers further increased the probability of maternal-fetal interactions through their functionality.

When both embryonic and endometrial factors considered together, biological process analysis revealed together with receptive endometrial products, the embryonic PRDX2, UBB, DAB2 and WNT7A participating in the negative regulation of apoptotic process. This could be a crucial process potentially depicting survival signals for the blastocysts that could lead to successful implantation. Moreover, the embryonic PEBP1, UBB and PSMA5 factors and the embryonic DAB2, HSP90AA1 and CD274 may act together with endometrial factors of the receptive endometrium, providing, respectively, significant enrichment in the MAPK cascade and signal transduction. These biomarkers could have a greater likelihood to predict implantation success, when analysed individually or in the reported small groups, due to the potential functionality upon implantation. When in close proximity, these maternal and fetal biomarkers suggest that they may finely tune cell-signaling and regulate apoptosis, through pathways that may be crucial for implantation.

### Example 4

### Detection of a subset of biomarkers (qRT-PCR) - RNA sequencing and qRT-PCR concordance

Apart from the high throughput RNA sequencing approach, qRT-PCR is an alternative assay and easier to apply methodology for determining the gene expression levels of certain biomarkers that could be used for the determination of implantation success. In particular, qRT-PCR was used to examine certain biomarkers for determining YMA/rba-YMA and AMA/rba-AMA gene expression profiles.

Trophectoderm RNA was reverse transcribed using the ultra-low SMART protocol (Takara-Clontech, USA), followed by long distance PCR (LD-PCR) amplification (as previously described) and generated sufficient cDNA quantities of full-length transcripts. Quantitative RT-PCR was performed using primers/oligo sequences for certain biomarkers that seem to be important for determining YMA/rba-YMA (IFITM3, DAB2 and FABP3) and AMA/rba-AMA (BAK1, ZFAND5 and BRAP) profiles, which are associated with certain implantation outcomes. Endogenous controls (reference genes) known in the art included GAPDH, ACTB, HNRNPC and H3F3B, hence the PCR output was normalized as described elsewhere (Livak and Schmittgen, 2001). In particular, ΔCt calculation was followed by 2^-ΔΔCt to normalise gene expression and identify fold-change (FC) levels, respectively. Primers/oligo sequences are provided in Table 2 (above).

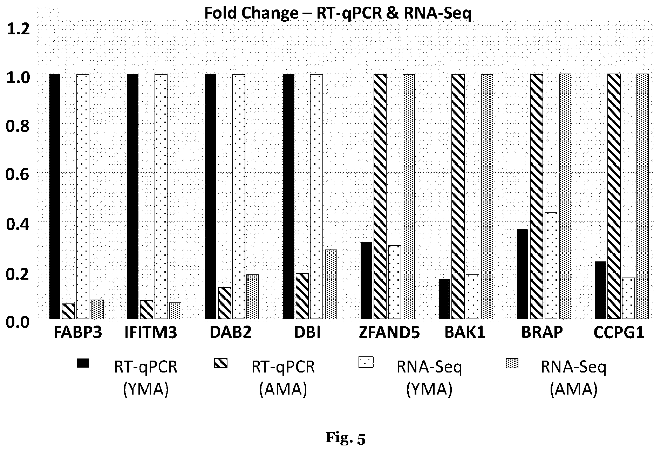

Three biological replicates were tested per group, revealing two important outcomes. First, following normalisation there is a good correlation between the RNA sequencing and qRT-PCR results (Figure 5), when independent samples classified as YMA/rba-YMA and AMA/rba-AMA were tested for the same biomarkers (r^2 = 93.9%). Similarly, analysis of covariance (ANCOVA) did not identify any statistically significant differences when the gene expression results between RNA sequencing and qRT-PCR were compared. In particular, following normalization of the qRT-PCR results against the housekeeping genes reported in Table 2 (GAPDH, ACTB, HNRNPC and H3F3B), the fold changes between YMA/rba-YMA and AMA/rba-AMA samples were compared with the fold change differences of the same biomarkers as measured by RNA sequencing (normalized by edgeR). The RNA sequencing results of the current invention were tested/confirmed using three biological replicates per YMA/rba-YMA and AMA/rba-AMA category, showing consistent gene expression patterns of the YMA/rba-YMA and AMA/rba-AMA samples in both methods.

Second, there are clear gene expression patterns for the selected biomarkers used. The outcomes of all blastocysts tested in the current invention by RNA sequencing, were used as a guide to reveal appropriate biomarkers and reported similar fold-change differences also when RT-qPCR was employed. Moreover, there is good correlation between the YMA/rba-YMA test samples with the increased expression levels of YMA/rba-YMA biomarkers and decreased levels of the AMA/rba-AMA biomarkers when tested by RT-PCR. Similarly, there is good correlation of the AMA/rba-AMA test samples with the increased expression levels of AMA/rba-AMA biomarkers and corresponding decreased expression levels with the YMA/rba-YMA biomarkers, when tested by RT-PCR (Figure 5).

Specifically, YMA/rba-YMA samples showed approximately 15, 6 and 14 times higher levels of expression for the exosome-related biomarkers FABP3, DAB2 and IFITM3, compared with the AMA/rba-AMA samples (Figure 5). On the other hand, the AMA/rba-AMA biomarkers were selected based on their mild ontological trend for ontologies/processes potentially integral to the degeneration process of the blastocyst. ZFAND5, BAK1 and BRAP and genes were selected for their association with apoptotic related processes. In this regard, ZFAND5, BAK1 and BRAP gene expression levels were approximately 3, 6 and 2.5 times higher in the AMA/rba-AMA compared with the YMA/rba-samples, respectively (Figure 5).

This analysis indicated good approximation of a test sample and selected biomarkers associated with low FDR (high confidence) and potential functionalities during implantation and possibly early embryo development. Higher levels of YMA/rba-YMA and lower levels of AMA/rba-AMA biomarkers were associated with the test sample of YMA/rba-YMA expression profile, with the test sample of AMA/rba-AMA expression profile showing, as expected, the reverse outcomes for the same biomarkers. One class of biomarkers (YMA/rba-YMA or AMA/rba-AMA) may be used to determine the YMA/rba-YMA or AMA/rba-AMA profiles, hence likelihood of implantation/developmental success of the test sample. The use of both biomarker categories/classes in combination may offer an even more robust approach, allowing the determination of the relative gene expression levels with respect to biomarkers that are both up- and down-regulated in the same sample, as an additional internal reference in agreement with the relative expression of the YMA/rba-YMA and AMA/rba-AMA profiles.

### Example 5

### Maternal age features are highlighted using alternative computational approaches

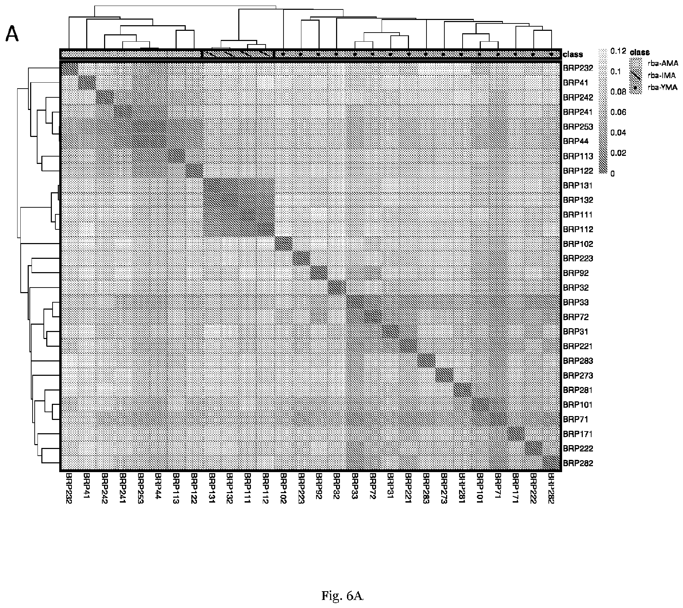

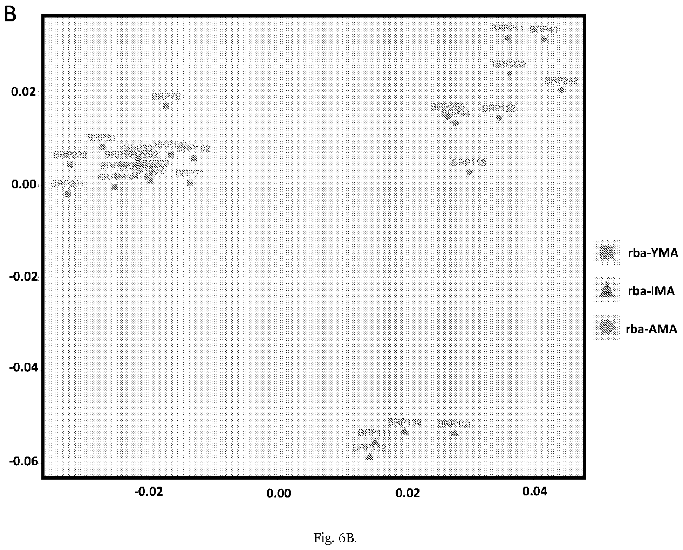

The RNA sequencing data was normalised, filtered and surrogate variables were identified minimising unwanted variation, using the DaMiRseq R package (Chiesa et al., 2018). This computational approach highlights informative biomarkers associated with the reproductive biological maternal age, hence implantation success (as reported in Example 2). The reproductive biological maternal age groups were classified based on the relationships of their gene expression profiles and illustrated in a heatmap (Spearman's correlation) and an unsupervised multidimensional scaling (MDS) plot (Figure 6). When the FSelect function was applied to rba-YMA and rba-AMA groups (more extreme groups), 290 gene transcripts were revealed (Table G). Biomarkers such as the ABCG2, ARHGAP10, SQLE and DHCR7 were among the ones that can be used to classify rba-AMA and rba-YMA groups.

**Table G. Subset of transcripts derived from DaMiRseq R package. The transcripts could be used to separate trophectoderm samples based on the maternal age. The RefSeq ID and associated gene names are displayed for each of the 290 (FSelect function) transcripts.**

| **refSeq ID** | **gene name** | **refSeq ID** | **gene name** | **refSeq ID** | **gene name** | **refSeq ID** | **gene name** |
|---|---|---|---|---|---|---|---|
| NM_000041 | APOE | NM_001244871 | DAB2 | NM_001330543 | EPSTI1 | NM_006577 | B3GNT2 |
| NM_000104 | CYP1B1 | NM_001257386 | ABCG2 | NM_001330557 | EPB41L3 | NM_006745 | MSMO1 |
| NM_000274 | OAT | NM_001260489 | LSR | NM_001330663 | HMGCS1 | NM_006868 | RAB31 |
| NM_000413 | HSD17B1 | NM_001260490 | LSR | NM_001331228 | EPSTI1 | NM_006927 | ST3GAL2 |
| NM_000499 | CYP1A1 | NM_001261403 | NFKB2 | NM_001343 | DAB2 | NM_012212 | PTGR1 |
| NM_000560 | CD53 | NM_001281533 | EPB41L3 | NM_001345944 | PFKP | NM_012307 | EPB41L3 |
| NM_000781 | CYP11A1 | NM_001281534 | EPB41L3 | NM_001346590 | INSIG1 | NM_014365 | HSPB8 |
| NM_000786 | CYP51A1 | NM_001281535 | EPB41L3 | NM_001346591 | INSIG1 | NM_014573 | TMEM97 |
| NM_000915 | OXT | NM_001282211 | NDRG2 | NM_001346592 | INSIG1 | NM_015687 | FILIP1 |
| NM_001002264 | EPSTI1 | NM_001282212 | NDRG2 | NM_001346593 | INSIG1 | NM_015908 | SRRT |
| NM_001005374 | LRSAM1 | NM_001282213 | NDRG2 | NM_001346594 | INSIG1 | NM_015925 | LSR |
| NM_001017369 | MSMO1 | NM_001282214 | NDRG2 | NM_001348985 | ABCG2 | NM_016084 | RASD1 |
| NM_001032396 | PJA1 | NM_001282215 | NDRG2 | NM_001348986 | ABCG2 | NM_016250 | NDRG2 |
| NM_001039361 | PRAMEF10 | NM_001282216 | NDRG2 | NM_001348987 | ABCG2 | NM_016619 | PLAC8 |
| NM_001040033 | CD53 | NM_001282633 | DBI | NM_001348988 | ABCG2 | NM_017826 | SOHLH2 |
| NM_001040663 | GAGE1 | NM_001282634 | DBI | NM_001348989 | ABCG2 | NM_018030 | OSBPL1A |
| NM_001042632 | SNX21 | NM_001282635 | DBI | NM_001352432 | DBI | NM_018641 | CHST12 |
| NM_001042633 | SNX21 | NM_001282636 | DBI | NM_001354558 | NDRG2 | NM_018677 | ACSS2 |
| NM_001076552 | ACSS2 | NM_001286679 | LARP6 | NM_001354559 | NDRG2 | NM_019058 | DDIT4 |
| NM_001077494 | NFKB2 | NM_001288724 | NFKB2 | NM_001354560 | NDRG2 | NM_019079 | L1TD1 |
| NM_001079815 | TMEM52B | NM_001289987 | FILIP1 | NM_001354561 | NDRG2 | NM_020166 | MCCC1 |
| NM_001079862 | DBI | NM_001293273 | MCCC1 | NM_001354562 | NDRG2 | NM_020170 | NCLN |
| NM_001079863 | DBI | NM_001300866 | FILIP1 | NM_001354564 | NDRG2 | NM_020548 | DBI |
| NM_001098272 | HMGCS1 | NM_001301188 | TRIB3 | NM_001354565 | NDRG2 | NM_020739 | CCPG1 |
| NM_001099773 | CYP11A1 | NM_001301190 | TRIB3 | NM_001354566 | NDRG2 | NM_020975 | RET |
| NM_001111045 | CCNA1 | NM_001301193 | TRIB3 | NM_001354567 | NDRG2 | NM_021034 | IFITM3 |
| NM_001111046 | CCNA1 | NM_001301196 | TRIB3 | NM_001354568 | NDRG2 | NM_021076 | NEFH |
| NM_001111047 | CCNA1 | NM_001301201 | TRIB3 | NM_001354569 | NDRG2 | NM_021158 | TRIB3 |
| NM_001127255 | NLRP7 | NM_001302623 | DHX8 | NM_001354570 | NDRG2 | NM_022071 | SH2D4A |
| NM_001128850 | RRAD | NM_001302688 | APOE | NM_001360 | DHCR7 | NM_022652 | DUSP6 |
| NM_001128852 | SRRT | NM_001302689 | APOE | NM_001424 | EMP2 | NM_024078 | NOC4L |
| NM_001128853 | SRRT | NM_001302690 | APOE | NM_001457 | FLNB | NM_024111 | CHAC1 |
| NM_001128854 | SRRT | NM_001302691 | APOE | NM_001512 | GSTA4 | NM_024605 | ARHGAP10 |
| NM_001130715 | PLAC8 | NM_001303253 | ACAT2 | NM_001675 | ATF4 | NM_024954 | UBTD1 |
| NM_001130716 | PLAC8 | NM_001316964 | REEP4 | NM_001946 | DUSP6 | NM_025107 | MYCT1 |
| NM_001130964 | PLCD1 | NM_001316965 | REEP4 | NM_001975 | ENO2 | NM_025130 | HKDC1 |
| NM_001135099 | TMPRSS2 | NM_001317955 | IDI1 | NM_002087 | GRN | NM_025232 | REEP4 |
| NM_001142776 | CHAC1 | NM_001317956 | IDI1 | NM_002130 | HMGCS1 | NM_031308 | EPPK1 |
| NM_001145139 | CXorf49B | NM_001317957 | IDI1 | NM_002337 | LRPAP1 | NM_031459 | SESN2 |
| NM_001145140 | CXorf49 | NM_001319075 | B3GNT2 | NM_002359 | MAFG | NM_032711 | MAFG |
| NM_001145160 | TPM4 | NM_001319216 | CYP1A1 | NM_002490 | NDUFA6 | NM_033138 | CALD1 |
| NM_001146108 | PTGR1 | NM_001319217 | CYP1A1 | NM_002502 | NFKB2 | NM_033139 | CALD1 |
| NM_001146109 | PTGR1 | NM_001320329 | NDRG2 | NM_002543 | OLR1 | NM_033140 | CALD1 |
| NM_001155 | ANXA6 | NM_001320638 | CD53 | NM_002627 | PFKP | NM_033157 | CALD1 |
| NM_001161342 | TMEM171 | NM_001320996 | FABP3 | NM_003129 | SQLE | NM_033255 | EPSTI1 |
| NM_001163817 | DHCR7 | NM_001321463 | NCLN | NM_003290 | TPM4 | NM_033421 | SNX21 |
| NM_001164317 | FLNB | NM_001322218 | DHX8 | NM_003641 | IFITM1 | NM_052845 | MMAB |
| NM_001164318 | FLNB | NM_001322219 | DHX8 | NM_003714 | STC2 | NM_080597 | OSBPL1A |
| NM_001164319 | FLNB | NM_001322220 | DHX8 | NM_003818 | CDS2 | NM_139176 | NLRP7 |
| NM_001164835 | L1TD1 | NM_001322934 | NFKB2 | NM_003914 | CCNA1 | NM_144497 | AKAP12 |
| NM_001171814 | OAT | NM_001322935 | NFKB2 | NM_003979 | GPRC5A | NM_145119 | PJA1 |
| NM_001172632 | OLR1 | NM_001322965 | OAT | NM_004102 | FABP3 | NM_147161 | ACOT11 |
| NM_001172633 | OLR1 | NM_001322966 | OAT | NM_004165 | RRAD | NM_152897 | SNX21 |
| NM_001174159 | SH2D4A | NM_001322967 | OAT | NM_004173 | SLC7A4 | NM_153022 | TMEM52B |
| NM_001174160 | SH2D4A | NM_001322968 | OAT | NM_004342 | CALD1 | NM_153256 | PROSER2 |
| NM_001177998 | SLC34A2 | NM_001322969 | OAT | NM_004477 | FRG1 | NM_173490 | TMEM171 |
| NM_001177999 | SLC34A2 | NM_001322970 | OAT | NM_004508 | IDI1 | NM_173648 | CCDC141 |
| NM_001178017 | DBI | NM_001322971 | OAT | NM_004525 | LRP2 | NM_176782 | FAM151A |
| NM_001178041 | DBI | NM_001322974 | OAT | NM_004748 | CCPG1 | NM_182810 | ATF4 |
| NM_001178042 | DBI | NM_001323067 | PFKP | NM_004827 | ABCG2 | NM_198336 | INSIG1 |
| NM_001178043 | DBI | NM_001323068 | PFKP | NM_005035 | POLRMT | NM_198337 | INSIG1 |
| NM_001188 | BAK1 | NM_001323069 | PFKP | NM_005100 | AKAP12 | NM_201535 | NDRG2 |
| NM_001193544 | ANXA6 | NM_001323070 | PFKP | NM_005542 | INSIG1 | NM_201536 | NDRG2 |
| NM_001197 | BIK | NM_001323071 | PFKP | NM_005656 | TMPRSS2 | NM_201537 | NDRG2 |
| NM_001198910 | CCDC169-SOHLH2 | NM_001323072 | PFKP | NM_005891 | ACAT2 | NM_201538 | NDRG2 |
| NM_001199989 | RASD1 | NM_001323073 | PFKP | NM_005983 | SKP2 | NM_201539 | NDRG2 |
| NM_001204450 | CCPG1 | NM_001323074 | PFKP | NM_006184 | NUCB1 | NM_201540 | NDRG2 |
| NM_001204451 | CCPG1 | NM_001324219 | HMGCS1 | NM_006225 | PLCD1 | NM_201541 | NDRG2 |
| NM_001242339 | PFKP | NM_001324220 | HMGCS1 | NM_006404 | PROCR | NM_205834 | LSR |
| NM_001242393 | ACSS2 | NM_001324222 | HMGCS1 | NM_006424 | SLC34A2 | NM_205835 | LSR |
| NM_001243120 | SKP2 | NM_001324223 | HMGCS1 | NM_006435 | IFITM2 | NM_206828 | NLRP7 |
| NM_001243794 | CHST12 | NM_001324224 | HMGCS1 | NM_006500 | MCAM | | |
| NM_001243795 | CHST12 | NM_001330219 | HSD17B1 | NM_006558 | KHDRBS3 | | |

### References

ALTMÄE, S., KOEL, M., VÕSA, U., ADLER, P., SUHORUTSENKO, M., LAISK-PODAR, T., KUKUSHKINA, V., SAARE, M., VELTHUT-MEIKAS, A. & KRJUTŠKOV, K. 2017. Meta-signature of human endometrial receptivity: a meta-analysis and validation study of transcriptomic biomarkers. Scientific reports, 7, 10077.
ANDREWS, S. 2010. FastQC: a quality control tool for high throughput sequence data. Available online at: http://www.bioinformatics.babraham.ac.uk/projects/fastqc.
BALABAN, B., BRISON, D., CALDERÓN, G., CATT, J., CONAGHAN, J., COWAN, L., EBNER, T., GARDNER, D., HARDARSON, T. & LUNDIN, K. 2011. The Istanbul consensus workshop on embryo assessment: proceedings of an expert meeting. Human Reproduction, 26, 1270-1283. CHIESA, M., COLOMBO, G. I. & PIACENTINI, L. 2018. DaMiRseq-an R/Bioconductor package for data mining of RNA-Seq data: normalization, feature selection and classification. Bioinformatics, 34, 1416-1418.
DAY, A. 2012. heatmap. plus: Heatmap with more sensible behavior. R package version, 1.
GARDNER, D. K. & SCHOOLCRAFT, W. B. 1999. Culture and transfer of human blastocysts. Current Opinion in Obstetrics and Gynecology, 11, 307-311.
HARTON, G. L., MUNNÉ, S., SURREY, M., GRIFO, J., KAPLAN, B., MCCULLOH, D. H., GRIFFIN, D. K., WELLS, D. & GROUP, P. P. 2013. Diminished effect of maternal age on implantation after preimplantation genetic diagnosis with array comparative genomic hybridization. Fertility and sterility, 100, 1695-1703.
HU, S., YAO, G., WANG, Y., XU, H., JI, X., HE, Y., ZHU, Q., CHEN, Z. & SUN, Y. 2014. Transcriptomic changes during the pre-receptive to receptive transition in human endometrium detected by RNA-Seq. The Journal of Clinical Endocrinology & Metabolism, 99, E2744-E2753. HUANG, D. W., SHERMAN, B. T. & LEMPICKI, R. A. 2009a. Bioinformatics enrichment tools: paths toward the comprehensive functional analysis of large gene lists. Nucleic acids research, 37, 1-13.
HUANG, D. W., SHERMAN, B. T. & LEMPICKI, R. A. 2009b. Systematic and integrative analysis of large gene lists using DAVID bioinformatics resources. Nature protocols, 4, 44-57.
HULL, M. G., FLEMING, C. F., HUGHES, A. O. & MCDERMOTT, A. 1996. The age-related decline in female fecundity: a quantitative controlled study of implanting capacity and survival of individual embryos after in vitro fertilization. Fertility and sterility, 65, 783-790.
JENSEN, L. J., KUHN, M., STARK, M., CHAFFRON, S., CREEVEY, C., MULLER, J., DOERKS, T., JULIEN, P., ROTH, A. & SIMONOVIC, M. 2009. STRING 8-a global view on proteins and their functional interactions in 630 organisms. Nucleic acids research, 37, D412-D416.
KOKKALI, G., TRAEGER-SYNODINOS, J., VRETTOU, C., STAVROU, D., JONES, G., CRAM, D., MAKRAKIS, E., TROUNSON, A.,
KANAVAKIS, E. & PANTOS, K. 2007. Blastocyst biopsy versus cleavage stage biopsy and blastocyst transfer for preimplantation genetic diagnosis of β-thalassaemia: a pilot study. Human Reproduction, 22, 1443-1449.
KOLDE, R. & KOLDE, M. R. 2015. Package 'pheatmap'. R Package, 1, 790.
KRUEGER, F. 2015. Trim Galore!: A wrapper tool around Cutadapt and FastQC to consistently apply quality and adapter trimming to FastQ files.
LI, H., HANDSAKER, B., WYSOKER, A., FENNELL, T., RUAN, J., HOMER, N., MARTH, G., ABECASIS, G. & DURBIN, R. 2009. The sequence alignment/map format and SAMtools. Bioinformatics, 25, 2078-2079.
LIAO, Y., SMYTH, G. K. & SHI, W. 2013. The Subread aligner: fast, accurate and scalable read mapping by seed-and-vote. Nucleic acids research, 41, e108-e108.
LIVAK, K. J. & SCHMITTGEN, T. D. 2001. Analysis of relative gene expression data using real-time quantitative PCR and the 2- ΔΔCT method. methods, 25, 402-408.
MEDICINE, P. C. O. T. A. S. F. R. 2006. Aging and infertility in women. Fertility and sterility, 86, S248.
METSALU, T. & VILO, J. 2015, Available online at https://biit.cs.ut.ee/clustvis/. ClustVis: a web tool for visualizing clustering of multivariate data using Principal Component Analysis and heatmap. Nucleic acids research, 43, W566-W570.
MOSTAFAVI, S., RAY, D., WARDE-FARLEY, D., GROUIOS, C. & MORRIS, Q. 2008. GeneMANIA: a real-time multiple association network integration algorithm for predicting gene function. Genome biology, 9, 1.
NELSON, S. M. & LAWLOR, D. A. 2011. Predicting live birth, preterm delivery, and low birth weight in infants born from in vitro fertilisation: a prospective study of 144,018 treatment cycles. PLoS medicine, 8.
NTOSTIS, P., KOKKALI, G., ILES, D., HUNTRISS, J., TZETIS, M., PICTON, H., PANTOS, K. & MILLER, D. 2019. Can trophectoderm RNA analysis predict human blastocyst competency? Systems Biology in Reproductive Medicine, 1-14.
PERTEA, M., KIM, D., PERTEA, G. M., LEEK, J. T. & SALZBERG, S. L. 2016. Transcript-level expression analysis of RNA-seq experiments with HISAT, StringTie and Ballgown. Nature Protocols, 11, 1650-1667.
PERTEA, M., PERTEA, G. M., ANTONESCU, C. M., CHANG, T.-C., MENDELL, J. T. & SALZBERG, S. L. 2015. StringTie enables improved reconstruction of a transcriptome from RNA-seq reads. Nature biotechnology, 33, 290.
POLl, M., ORI, A., CHILD, T., JAROUDI, S., SPATH, K., BECK, M. & WELLS, D. 2015. Characterization and quantification of proteins secreted by single human embryos prior to implantation. EMBO molecular medicine, 7, 1465-1479.
PYTHONSCRIPT Accessed on March 2018. prepDE.py.https://ccb.jhu.edu/software/stringtie/dl/prepDE.py.
ROBINSON, M. D. & OSHLACK, A. 2010. A scaling normalization method for differential expression analysis of RNA-seq data. Genome biology, 11, R25.
SARAVELOS, S. H. & LI, T.-C. 2012. Unexplained recurrent miscarriage: how can we explain it? Human reproduction, 27, 1882-1886. SCHMIDT, L., SOBOTKA, T., BENTZEN, J. G., NYBOE ANDERSEN, A., REPRODUCTION, E. & FORCE, S. T. 2012. Demographic and medical consequences of the postponement of parenthood. Human reproduction update, 18, 29-43.
SCOTT JR, R. T., FERRY, K., SU, J., TAO, X., SCOTT, K. & TREFF, N. R. 2012. Comprehensive chromosome screening is highly predictive of the reproductive potential of human embryos: a prospective, blinded, nonselection study. Fertility and sterility, 97, 870-875.
TEAM, R. C. 2019. R: A language and environment for statistical computing. R Foundation for Statistical Computing, Vienna, Austria.
WARDE-FARLEY, D., DONALDSON, S. L., COMES, O., ZUBERI, K., BADRAWI, R., CHAO, P., FRANZ, M., GROUIOS, C., KAZI, F. & LOPES, C. T. 2010. The GeneMANIA prediction server: biological network integration for gene prioritization and predicting gene function. Nucleic acids research, 38, W214-W220.
WEISSBEIN, U., SCHACHTER, M., EGLI, D. & BENVENISTY, N. 2016. Analysis of chromosomal aberrations and recombination by allelic bias in RNA-Seq. Nature communications, 7, 12144.
WICKHAM, H. 2016. ggplot2: elegant graphics for data analysis, springer.
ZHAO, S., LI, C.-I., GUO, Y., SHENG, Q. & SHYR, Y. 2018. RnaSeqSampleSize: real data based sample size estimation for RNA sequencing. BMC bioinformatics, 19, 191.

## Claims

1. Use of the expression profile of at least 35 genes selected from any of those listed in Table A and/or Table C, or gene transcripts, expression products or fragments thereof, in or as a biomarker panel for the young reproductive chronological maternal age (YMA) and/or young reproductive biological maternal age (rba-YMA) of a blastocyst in a method for assessing the increased likelihood of blastocyst implantation success.

2. The use as claimed in claim 1, comprising at least 50 genes selected from any of those listed in Table A and/or Table C, or gene transcripts, expression products or fragments thereof.

3. The use as claimed in claim 1, wherein the at least 35 genes are selected from those listed in Table A, or gene transcripts, expression products or fragments thereof.

4. The use as claimed in claim 1, wherein the at least 35 genes comprise at least 2 of the first 50 genes in Table A1, or gene transcripts, expression products or fragments thereof.

5. The use as claimed in claim 1, wherein the at least 35 genes comprise at least 7 genes selected from any of those listed in Table E1, or gene transcripts, expression products or fragments thereof.

6. An in vitro method of identifying the reproductive chronological and/or biological maternal age of a blastocyst and thereby the likelihood of implantation success of the blastocyst for potential embryo transfer, the method comprising:
determining, in a test sample obtained from the blastocyst, the level of expression of at least 35 genes selected from the genes and gene transcripts listed in Table A and/or Table C, or gene trascripts, expression products or fragments thereof;
comparing the level of expression of the at least 35 genes, gene transcripts, expression products, or fragments thereof, in the test sample, with the level of expression of the at least 35 genes, gene transcripts, expression products, or fragments thereof, in a reference sample;
wherein the level of expression of the at least 35 genes, gene transcripts, expression products, or fragments thereof, in the test sample relative to the level of expression in the reference sample identifies the reproductive chronological and/or biological maternal age of a blastocyst and thereby the likelihood of implantation success of the blastocyst.

7. A method as claimed in claim 6, further comprising:
determining, in the test sample, the level of expression of at least one stably expressed gene, gene transcript, expression product, or fragment thereof;
comparing the level of expression of the at least one stably expressed gene, gene transcript, expression product, or fragment thereof in the test sample, with the level of expression of the at least one stably expressed gene, gene transcript, expression product, or fragment thereof, in the reference sample;
wherein a difference in the relative level of expression of the corresponding at least 35 genes, gene transcripts, expression products, or fragments thereof listed in Table A and/or Table C, versus the relative level of expression of the at least one stably expressed gene, gene transcript, expression product, or fragment thereof in the reference sample, identifies the likelihood of implantation success of the blastocyst.

8. A method as claimed in claim 7, wherein the at least one stably expressed gene, gene transcript, expression product, or fragment thereof includes any one or combination of the genes listed in Table F.

9. A method as claimed in claim 6, further comprising:
determining in the test sample, a gene expression profile of the at least 35 genes or gene transcripts listed in Table A and/or Table C;
comparing the gene expression profile of the test sample, with the gene expression profile of the reference sample;
wherein the reference sample is representative of a blastocyst having a young reproductive chronological (YMA) and/or young reproductive biological maternal age (rba-YMA), or an advanced reproductive chronological (AMA) and/or advanced biological maternal age (rba-AMA), or that is representative of an intermediate profile between YMA/rba-YMA and AMA/rba-AMA samples; and
wherein a correlation in the gene expression profiles, or the absence of a correlation, identifies the likelihood of implantation success of the blastocyst.

10. The use as claimed in any of claims 1-5, or method as claimed in any of claims 6-9, wherein the sample assayed comprises culture media in which the subject blastocyst has previously been cultured.

11. The use as claimed in any of claims 1-5 or 10, or method as claimed in any of claims 6-9, wherein at least one of the genes is selected from those listed in Table A* and/or Table C*.

## Patentansprüche

1. Verwendung des Expressionsprofils von mindestens 35 Genen, die ausgewählt sind aus beliebigen derjenigen, die in Tabelle A und/oder Tabelle C aufgeführt sind, oder Gentranskripten, Expressionsprodukten oder Fragmenten davon in einer oder als eine Biomarkertafel für das junge reproduktive chronologische maternale Alter (YMA) und/oder junge reproduktive biologische maternale Alter (rba-YMA) eines Blastozysten in einem Verfahren zum Bewerten der erhöhten Wahrscheinlichkeit eines Implantationserfolgs von Blastozysten.

2. Verwendung nach Anspruch 1, umfassend mindestens 50 Gene, die ausgewählt sind aus beliebigen derjenigen, die in Tabelle A und/oder Tabelle C aufgeführt sind, oder Gentranskripte, Expressionsprodukte oder Fragmente davon.

3. Verwendung nach Anspruch 1, wobei die mindestens 35 Gene ausgewählt sind aus denjenigen, die in Tabelle A aufgeführt sind, oder Gentranskripten, Expressionsprodukten oder Fragmenten davon.

4. Verwendung nach Anspruch 1, wobei die mindestens 35 Gene mindestens 2 der ersten 50 Gene in Tabelle A1 oder Gentranskripte, Expressionsprodukte oder Fragmente davon umfassen.

5. Verwendung nach Anspruch 1, wobei die mindestens 35 Gene mindestens 7 Gene, die ausgewählt sind aus beliebigen derjenigen, die in Tabelle E1 aufgeführt sind, oder Gentranskripte, Expressionsprodukte oder Fragmente davon umfassen.

6. In-vitro-Verfahren zum Identifizieren des reproduktiven chronologischen und/oder biologischen maternalen Alters eines Blastozysten und dadurch der Wahrscheinlichkeit eines Implantationserfolgs des Blastozysten bei potenziellem Embryotransfer, wobei das Verfahren umfasst:
Bestimmen, in einer aus dem Blastozysten erhaltenen Testprobe, des Expressionsniveaus von mindestens 35 Genen, die ausgewählt sind aus den Genen und Gentranskripten, die in Tabelle A und/oder Tabelle C aufgeführt sind, oder Gentranskripten, Expressionsprodukten oder Fragmenten davon;
Vergleichen des Expressionsniveaus der mindestens 35 Gene, Gentranskripte, Expressionsprodukte oder Fragmente davon in der Testprobe mit dem Expressionsniveau der mindestens 35 Gene, Gentranskripte, Expressionsprodukte oder Fragmente davon in einer wobei das Expressionsniveau der mindestens 35 Gene, Gentranskripte, Expressionsprodukte oder Fragmente davon in der Testprobe relativ zu dem Expressionsniveau in der Referenzprobe das reproduktive chronologische und/oder biologische maternale Alter eines Blastozysten und dadurch die Wahrscheinlichkeit eines Implantationserfolgs des Blastozysten identifiziert.

7. Verfahren nach Anspruch 6, ferner umfassend:
Bestimmen, in der Testprobe, des Expressionsniveaus mindestens eines stabil exprimierten Gens, Gentranskripts, Expressionsprodukts oder Fragments davon;
Vergleichen des Expressionsniveaus des mindestens einen stabil exprimierten Gens, Gentranskripts, Expressionsprodukts oder Fragments davon in der Testprobe mit dem Expressionsniveau des mindestens einen stabil exprimierten Gens, Gentranskripts, Expressionsprodukts oder Fragments davon in der Referenzprobe;
wobei eine Differenz im relativen Expressionsniveau der entsprechenden mindestens 35 Gene, Gentranskripte, Expressionsprodukte oder Fragmente davon, die in Tabelle A und/oder Tabelle C aufgeführt sind, gegenüber dem relativen Expressionsniveau des mindestens einen stabil exprimierten Gens, Gentranskripts, Expressionsprodukts oder Fragments davon in der Referenzprobe die Wahrscheinlichkeit eines Implantationserfolgs des Blastozysten identifiziert.

8. Verfahren nach Anspruch 7, wobei das mindestens eine stabil exprimierte Gen, Gentranskript, Expressionsprodukt oder Fragment davon beliebige oder eine Kombination der in Tabelle F aufgeführten Gene beinhaltet.

9. Verfahren nach Anspruch 6, ferner umfassend:
Bestimmen, in der Testprobe, eines Genexpressionsprofils der mindestens 35 Gene oder Gentranskripte, die in Tabelle A und/oder Tabelle C aufgeführt sind;
Vergleichen des Genexpressionsprofils der Testprobe mit dem Genexpressionsprofil der Referenzprobe;
wobei die Referenzprobe repräsentativ für einen Blastozysten mit einem jungen reproduktiven chronologischen (YMA) und/oder jungen reproduktiven biologischen maternalen Alter (rba-YMA) oder einem fortgeschrittenen reproduktiven chronologischen (AMA) und/oder fortgeschrittenen biologischen maternalen Alter (rba-AMA) ist oder die repräsentativ für ein Zwischenprofil zwischen YMA/rba-YMA- und AMA/rba-AMA-Proben ist; und
wobei eine Korrelation in den Genexpressionsprofilen oder das Fehlen einer Korrelation die Wahrscheinlichkeit eines Implantationserfolgs des Blastozysten identifiziert.

10. Verwendung nach einem der Ansprüche 1-5 oder Verfahren nach einem der Ansprüche 6-9, wobei die untersuchte Probe Kulturmedien umfasst, in denen der betreffende Blastozyst zuvor kultiviert wurde.

11. Verwendung nach einem der Ansprüche 1-5 oder 10 oder Verfahren nach einem der Ansprüche 6-9, wobei mindestens eines der Gene ausgewählt ist aus denjenigen, die in Tabelle A* und/oder Tabelle C* aufgeführt sind.

## Revendications

1. Utilisation du profil d'expression d'au moins 35 gènes sélectionnés parmi l'un de ceux répertoriés dans le tableau A et/ou le tableau C, ou de transcrits de gènes, de produits d'expression ou de fragments de ceux-ci, dans ou en tant que panel de biomarqueurs pour le jeune âge maternel chronologique de reproduction (YMA) et/ou le jeune âge maternel biologique de reproduction (rba-YMA) d'un blastocyste dans un procédé d'évaluation de la probabilité accrue de succès de l'implantation du blastocyste.

2. Utilisation selon la revendication 1, comprenant au moins 50 gènes sélectionnés parmi l'un de ceux répertoriés dans le tableau A et/ou le tableau C, ou de transcrits de gènes, de produits d'expression ou de fragments de ceux-ci.

3. Utilisation selon la revendication 1, lesdits au moins 35 gènes étant sélectionnés parmi ceux répertoriés dans le tableau A, ou des transcrits de gènes, des produits d'expression ou des fragments de ceux-ci.

4. Utilisation selon la revendication 1, lesdits au moins 35 gènes comprenant au moins 2 des 50 premiers gènes dans le tableau A1, ou des transcrits de gènes, des produits d'expression ou des fragments de ceux-ci.

5. Utilisation selon la revendication 1, lesdits au moins 35 gènes comprenant au moins 7 gènes sélectionnés parmi l'un de ceux répertoriés dans le tableau E1, ou des transcrits de gènes, des produits d'expression ou des fragments de ceux-ci.

6. Procédé in vitro d'identification de l'âge maternel chronologique et/ou biologique de reproduction d'un blastocyste et, par conséquent, de la probabilité de succès de l'implantation du blastocyste pour un transfert embryonnaire potentiel, le procédé comprenant :
la détermination, dans un échantillon d'essai obtenu à partir du blastocyste, du niveau d'expression d'au moins 35 gènes sélectionnés parmi les gènes et les transcrits de gènes répertoriés dans le tableau A et/ou le tableau C, ou les transcrits de gènes, les produits d'expression ou les fragments de ceux-ci ;
la comparaison du niveau d'expression des au moins 35 gènes, des transcrits de gènes, des produits d'expression, ou des fragments de ceux-ci, dans l'échantillon d'essai, avec le niveau d'expression des au moins 35 gènes, des transcrits de gènes, des produits d'expression, ou des fragments de ceux-ci, dans un échantillon de référence ;
ledit niveau d'expression des au moins 35 gènes, des transcrits de gènes, des produits d'expression ou des fragments de ceux-ci, dans l'échantillon d'essai par rapport au niveau d'expression dans l'échantillon de référence identifiant l'âge maternel chronologique et/ou biologique de reproduction d'un blastocyste et, par conséquent, la probabilité de succès de l'implantation du blastocyste.

7. Procédé selon la revendication 6, comprenant en outre :
la détermination, dans l'échantillon d'essai, du niveau d'expression d'au moins un gène, un transcrit de gène, un produit d'expression ou un fragment de celui-ci, exprimé de manière stable ;
la comparaison du niveau d'expression du au moins un gène, un transcrit de gène, un produit d'expression ou un fragment de ceux-ci exprimé de manière stable dans l'échantillon d'essai, avec le niveau d'expression du au moins un gène, un transcrit de gène, un produit d'expression ou un fragment de ceux-ci, dans l'échantillon de référence ;
une différence dans le niveau relatif d'expression des au moins 35 gènes correspondants, des transcrits de gènes, des produits d'expression ou des fragments de ceux-ci répertoriés dans le tableau A et/ou le tableau C, contre le niveau relatif d'expression du au moins un gène, un transcrit de gènes, un produit d'expression ou un fragment de celui-ci exprimé de manière stable dans l'échantillon de référence, identifiant la probabilité de succès de l'implantation du blastocyste.

8. Procédé selon la revendication 7, ledit au moins un gène, un transcrit de gène, un produit d'expression ou un fragment de celui-ci exprimé de manière stable comprenant l'un quelconque ou une combinaison des gènes répertoriés dans le tableau F.

9. Procédé selon la revendication 6, comprenant en outre :
la détermination dans l'échantillon d'essai, d'un profil d'expression génique des au moins 35 gènes ou des transcrits de gènes répertoriés dans le tableau A et/ou le tableau C ;
la comparaison du profil d'expression génique de l'échantillon d'essai, avec le profil d'expression génique de l'échantillon de référence ;
ledit échantillon de référence représentant un blastocyste possédant un jeune âge maternel chronologique de reproduction (YMA) et/ou un jeune âge maternel biologique de reproduction (rba-YMA), ou un âge maternel chronologique de reproduction avancé (AMA) et/ou un âge maternel biologique avancé (rba-AMA), ou qui représente un profil intermédiaire entre les échantillons YMA/rba-YMA et AMA/rba-AMA ; et
une corrélation dans les profils d'expression géniques, ou l'absence d'une corrélation, identifiant la probabilité de succès de l'implantation du blastocyste.

10. Utilisation selon l'une quelconque des revendications 1 à 5, ou procédé selon l'une quelconque des revendications 6 à 9, ledit échantillon sous essai comprenant des milieux de culture dans lesquels le blastocyste sujet a été préalablement cultivé.

11. Utilisation selon l'une quelconque des revendications 1 à 5 ou 10, ou procédé selon l'une quelconque des revendications 6 à 9, au moins l'un des gènes étant sélectionné parmi ceux répertoriés dans le tableau A* et/ou le tableau C*.
